# EUROPEAN PATENT APPLICATION

(11) **EP 4 595 955 A1**
(43) Date of publication of application: **06.08.2025**
(21) Application number: 24155403.9
(22) Date of filing: 01.02.2024
(51) Int. Cl.: A61K 9/28, A61K 31/785, A61K 47/26, A61K 47/32, A61K 47/38

(54) **COMPOSITIONS AND METHODS FOR TREATING BILE ACID MALABSORPTION**

(71) Applicant: Cosmo Technologies Ltd., Dublin D02KV60 Dublin (IE)
(72) Inventor: LONGO, Luigi Maria, Milan (IT); PAGANIA, Stefania, Milan (IT)
(74) Representative: FRKelly

(57) **Abstract**

Pharmaceutical compositions comprising bile acid sequestrants, their use in treating bile acid malabsorption, and associated conditions such as bile acid diarrhea and colitis are described herein. Methods for treating bile acid malabsorption and associated conditions are also described.

## Description

### Field of the Invention

The present invention relates to pharmaceutical compositions comprising bile acid sequestrants, and their use in treating bile acid malabsorption, and associated conditions such as bile acid diarrhea and bile acid colitis, as well as methods for treating bile acid malabsorption and associated conditions.

### Background

Bile acids (also called bile salts) are the main functional components of bile, which is produced in the liver and stored in the gallbladder. After a meal, this stored bile is secreted into the duodenum, where it exerts its functional role in digestion. Bile acids are natural surfactants, which solubilize lipids and lipophilic substances through formation of micelles.

Bile acids are endogenous surfactants synthetized in the liver by the cytochrome P450-mediated oxidation of cholesterol and play a key role in the absorption of dietary fats from the small intestine. They are conjugated with the amino acids taurine or glycine, or with a sulfate or a glucuronide, and are then transported across canalicular membrane of the hepatocytes into the bile and stored in the gallbladder, from which they are secreted into the duodenum to solubilize fats contained into the diet.

After each meal, bile acids are released into duodenum, the first tract of the small intestine, where they exert their role as physiological detergents molecules, facilitating the absorption of lipids and liposoluble nutrients, including liposoluble vitamins, from the small intestine, bile acid are ionized in the lumen of the small intestine, and the ionization increases their water solubility and renders bile acids impermeable to cell membranes, allowing them to reach the critical micellar concentration, which is the concentration above which micelles are formed. The micelles are needed in the small intestine for digestion and absorption of lipids, and lipophilic substances such as some vitamins.

Once the bile acids have exerted their role in the duodenum and jejunum, about 95% of the secreted amount is reabsorbed by active transport in the ileum and delivered back to the liver via the portal vein. This process, known as enterohepatic circulation of bile acid, may involve hepatic conjugation and intestinal deconjugation. As an effect, only about 5% of the secreted amount is lost into the feces. Daily loss of Bile acids is compensated by de novo synthesis in the liver and thus, a constant bile acid pool is maintained. The intestinal bile acid uptake is mainly mediated by the apical sodium dependent bile salt transporter (ASBT). *(*Li T. et al., Regulation of Bile Acid and Cholesterol Metabolism by PPARs. Hindawi Publishing Corporation, Vol 2009, Article ID 501739*).*

In humans, the bile acid pool consists of primary bile acids and secondary bile acids. The primary bile acids are synthesized from cholesterol exclusively in the liver through two pathways, the classic pathway (accounting for about 90% of bile acid synthesis) and the alternative pathway (accounting for about the remaining 10%). The secondary bile acids are synthesized from primary bile acids by bacterial enzymes of the natural microbiota resident into the intestine. (Li T. et al., Regulation of Bile Acid and Cholesterol Metabolism by PPARs. Hindawi Publishing Corporation, Vol 2009, Article ID 501739*).*

Enterohepatic circulation of bile acids is an efficient process capable of cycling these important compounds between the liver and intestine multiple times during the day.

Abnormal bile acids homeostasis and lack of, or impaired reabsorption into the bloodstream, can exacerbate several disorders including Crohn's disease (CD), hepatic microvascular dysplasia, inflammatory bowel disease (IBD), irritable bowel syndrome (IBS), colonic cancer, cholestasis, insufficient control of blood glucose and cardiovascular disease. Subsequently, lack of, or impaired reabsorption of bile acids in the ileum and the enterohepatic bile acid circulation resulting in watery diarrhea. Bile acids in the large bowel cause abnormally high levels of water and salts to get into the large bowel from the bloodstream, causing watery diarrhea.

Bile acid malabsorption (BAM) is a defect in this enterohepatic circulation of bile acids, whereby increased proportions of the secreted bile acids are not reabsorbed in the ileum and reach the colon consequently. Bile acids in the colon activate increase fluid secretion, increase colonic motility and thereby decrease colonic transit time. Overall, this generates a chronic watery diarrhea (also known as Bile Acid Diarrhea, (BAD)), accompanied by further symptoms such as bloating, pain, faecal urgency and faecal incontinence. It is currently estimated that about 1% of the population has Bile Acid Diarrhea, and that about 30% of all irritable bowel syndrome with diarrhea (IBS-D) cases are due to Bile Acid Malabsorption. Bile Acid Malabsorption has also been associated with other diseases and conditions, such as microscopic colitis, Crohn's disease, HIV-related enteritis, persistent diarrhea following bacterial infection and exocrine pancreatic insufficiency.

Currently, Bile Acid Diarrhea is subdivided in 3 main types: type 1 results from terminal ileal resection/bypass or disease (for example: Crohn's disease or ileal resection), which results in failure of enterohepatic recycling of bile acids, and excess amounts entering the colon. Type 2 often referred to as primary (or idiopathic) bile acid diarrhea, is the most common cause of bile acid malabsorption and may account for at least 30% of individuals who would otherwise be labeled as having IBS-D or functional diarrhea. Despite much investigation, the etiology and pathogenesis of type 2 Bile Acid Diarrhea is poorly understood. Type 3 includes causes not included with types 1 and 2 that may interfere with normal bile acid cycling, small intestinal motility, or composition of ileal contents: for example, small intestine bacterial overgrowth, chronic pancreatitis, celiac disease, etc. (DiBaise, Nutrition Issues in Practical Gastroenterology, Series #198, Practical Gastroenterology 2020*).* There is also a type 4, which is the excess of bile acid production as side effect of therapy with metformin.

To treat the diarrheic effects generated by the excess of bile acids in the colon, bile acid sequestrants substances are commonly used in clinical practice, although these products have no approval for such use and are approved for lowering the cholesterol in the blood. Cholestyramine, colestipol, and colesevelam are the three main bile acid sequestrants currently available in the US and European markets.

Currently used sequestrant substances are available as granules for suspension or capsule shape tablets that require a very high amount of active ingredient with several administrations per day. This creates issues with patient compliance.

First-generation bile acid sequestrants (or resins) are cholestyramine or colestipol are available as powders or granules. However, patients are often intolerant to those sequestrants, given its side effects such as nausea, bloating, and constipation. Moreover, an efficacious treatment of BAD requires the administration of very high doses of these bile acid sequestrants: cholestyramine up to 36 g per day; colestipol up to 30 g per day (Wilcox et al., Aliment Pharmacol Ther 2014; 39: 923-939). These very high doses of cholestyramine or colestipol are not tolerated by many patients, because these compounds are in form of powders and have a very low palatability. Discontinuation rates have been reported to range from 34% to 60%. Colesevelam (and salts thereof, e.g. hydrochloride salt) is a second-generation bile acid sequestrant, which is commercially available as immediate release tablets or granules. Even though the recommended dose of colesevelam for treating BAD is reduced as compared to cholestyramine and colestipol, it remains high and corresponds to 6 or 7 tablets per day. Given the chronicity of the disease, there are patient compliance issues reported as well for colesevelam, because of the high number of tablets per day. When administered in the form of granules, colesevelam is known to generate a so called "sand effect" in the mouth that most patients find particularly unpleasant, and which discourages them from continuing their treatment. There is an immediate need for an improved formulation of bile acid sequestrants capable of increasing the patient's compliance; in particular, there is an immediate need for a formulation of bile acid sequestrants in form of tablets, with a high drug content of active substance per dosage unit while maintaining the dimensions small enough to allow for easy administration.

Currently available bile acid sequestrant is released in the upper GI tract. As such, the use of such compositions for treatment of BAM has several drawbacks, such as negative drug-drug interactions, decreased absorption of fat-soluble vitamins, and a negative impact on the absorption of other medications. Moreover, the available formulations of bile acid sequestrants have some adverse reactions (e.g., nausea, dyspepsia and bloating), which are due to their unspecific effect in the upper GI tract.

WO2021/163007 (Viscera) provides a colesevelam colon specific drug delivery system for use in the treatment of cholestasis and/or cholestatic pruritis. Described therein is an oral drug delivery system comprising: a) a core comprising a therapeutically effective amount of at least one active agent present in an amount of from about 35% to about 65% w/w of the core, and a drug release controlling component capable of providing release of the active agent primarily in a region selected from the group consisting of the lower gastrointestinal tract, the large intestine, the jejunum, the ileum, the cecum, the colon, the rectum, and combinations thereof, b) an outer coating encasing the core, and optionally a plasticizer, wherein after ingestion by a patient the active agent is released primarily in the region selected from the group consisting of the lower gastrointestinal tract, the large intestine, the jejunum, the ileum, the cecum, the colon, the rectum, and combinations thereof.

The recommended dose for colesevelam for treating bile acid malabsorption is high (3.75 g per day), which corresponds to around 6 tablets comprising 625 mg colesevelam hydrochloride per day. The tablets can be administered either in a single non-fractionated administration or in multiple fractionated administrations, e.g., six tablets once per day, or three tablets twice per day, or two tablets thrice per day. Given the chronicity of the disease, and the high number of tablets required per day, patient compliance issues often occur when treating with colesevelam.

The current commercially available formulations of bile acid sequestrants are characterized by immediate release in the stomach. The bile acid sequestrants therefore exert their pharmacological action starting in the duodenum: once the bile acids are released by the gallbladder in the duodenum, they are bound by the sequestrants and cannot exert their physiological digestive role of solubilization and absorption-enhancement of lipids and liposoluble vitamins. This leads to nutritional disorders and deficiencies of liposoluble vitamins.

Additionally, the effect on the bile acids in the upper GI is suboptimal when the disease to be treated is caused by an excess of bile acids entering the colon; in other words, the currently available formulations do not target only the excess of bile acids entering the colon in patient with bile acid malabsorption/bile acid diarrhea, but target the whole pool of bile acids released from the gallbladder and transiting the upper small intestine segments, i.e. the duodenum and jejunum.

Furthermore, bile acid sequestrants are non-specific, in that they bind a lot of different substances, preventing their absorption from the small intestine. Importantly, bile acid sequestrants are known to bind several drugs administered orally, a characteristic which has an impact on the systemic bioavailability of those drugs, because, once these are bound to the resin polymer chain, they are not absorbed and are excreted in the faeces. Most orally administered drugs are absorbed in the upper GI tract, i.e. in the stomach, or in the duodenum or in the jejunum. The currently available formulations of bile acid sequestrants, which release the resin in the stomach, have a significant interaction with drugs absorbed in the upper GI tract, which leads to the need of administering these drugs at least 4 hours prior to bile acid sequestrants, leading to difficulties for the physician to draw an effective treatment schedule for patients taking several concomitant medications.

Finally, the currently available formulations of bile acid sequestrants, although in general are regarded to as safe, have some adverse reactions (e.g., nausea, dyspepsia, bloating), which are due to their effect in, and transit throughout, the whole GI tract, from the stomach to the colon.

There is therefore the need for new treatment options for bile acid malabsorption and bile acid diarrhea which overcomes the above highlighted limitations of the currently available treatments.

### SUMMARY

The present invention provides pharmaceutical compositions which resolve the above-mentioned drawbacks. The pharmaceutical compositions described herein are characterized by a high drug load, allowing for reduced number of administrations per day, and delivery of the active substance in the low intestinal districts to avoid unwanted interference with the natural role of the bile acids.

In one aspect, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof and optionally one or more of a diluent, a disintegrant, a glidant and a lubricant; and
wherein the colesevelam and/or the pharmaceutically acceptable salts thereof is present in an amount of at least 66 wt% of the tablet core, such as at least about 70 wt% of the tablet core.

Thus, the total amount of colesevelam and/or pharmaceutically acceptable salts thereof in the unit dose pharmaceutical composition for oral administration accounts for at least 66 wt% of the tablet core, such as at least about 70 wt% of the tablet core. The total amount of colesevelam and or pharmaceutically acceptable salts thereof in the unit dose includes the amount of colesevelam and/or pharmaceutically acceptable salts thereof in the granulate component and in the non-granulate component.

The colesevelam and/or pharmaceutically acceptable salts thereof may be present in the granulate component in an amount of from 30 to 60 wt% of the tablet core, optionally 30 to 50 wt% of the tablet core, optionally 35 to 45 wt% of the tablet core.

The colesevelam and/or pharmaceutically acceptable salts thereof may be present in the non-granulate component in an amount of from 30 to 60 wt% of the tablet core, optionally 30 to 50 wt% of the tablet core, optionally 35 to 45 wt% of the tablet core.

Together the amount of colesevelam and/or pharmaceutically acceptable salts thereof in the non-granulate component and the amount in the granulate component accounts for at least 66 wt% of the tablet core, such as at least about 70 wt% of the tablet core. For example, the amount of colesevelam and/or pharmaceutically acceptable salts thereof in the non-granulate component and the amount in the granulate component accounts for at least 66 wt% of the tablet core, such as an amount of from 66 wt% to 90 wt% of the tablet core, optionally in an amount of from 70 wt% to 90 wt% of the tablet core, optionally, in an amount of from 75 wt% to 90 wt% of the tablet core, such as from 76 wt% to 90 wt% of the tablet core, suitably 80 wt% to 90 wt% of the tablet core, such as from 81 wt% to 90 wt% of the tablet core, optionally, from 82 wt% to 90 wt% of the tablet core.

Suitably, colesevelam and/or pharmaceutically acceptable salts thereof is present in the unit dose pharmaceutical composition for oral administration in an amount of at least 70 wt%, or at least 75 wt%, or at least 76 wt% of the tablet core, suitably at least 80 wt% of the tablet core, more suitably at least 81 wt% of the tablet core, optionally at least 82 wt% of the tablet core. For example, colesevelam and/or pharmaceutically acceptable salts thereof is present in the unit dose pharmaceutical composition for oral administration in an amount of 70 wt% to 90 wt% of the tablet core, or in an amount of 75 wt% to 90 wt% of the tablet core, or in an amount of 76 wt% to 90 wt% of the tablet core, suitably in an amount of 80 wt% to 90 wt% of the tablet core, more suitably 81 wt% to 90 wt% of the tablet core, optionally in an amount of from 82 wt% to 90 wt% of the tablet core.

The weight percentages of colesevelam or a pharmaceutically acceptable salt(s) thereof refers to the weight percent of anhydrous colesevelam or a pharmaceutically acceptable salt(s) thereof. For example, "at least 66 wt% colesevelam and/or pharmaceutically acceptable salt thereof' implies "at least 66 wt% anhydrous colesevelam and/or pharmaceutically acceptable salt thereof". For example, "at least 66 wt% colesevelam hydrochloride" implies "at least 66 wt% anhydrous colesevelam hydrochloride". Hydrated forms of colesevelam may also be used, however, the amounts employed are adjusted to compensate for the amount of water present i.e. when using hydrated forms of colesevelam, the amount of water/hydrate present in the hydrated colesevelam does not contribute to the weight percentage of colesevelam based on dry weight.

Suitably, hydrated forms of colesevelam or pharmaceutically acceptable salts thereof that may be used in the present invention comprise less than about 20 wt% water based on the total weight of the hydrated colesevelam or pharmaceutically acceptable salt thereof, preferably less than about 10 wt%, e.g. about 1 wt% , or about 2% wt%, or 3 wt%, or 4 wt%, or 5 wt%, or 6 wt%, or 7 wt%, or 8 wt%, or 9 wt%, water based on the total weight of the hydrated colesevelam or pharmaceutically acceptable salt thereof or any fractions of the above integers. When using hydrated forms of colesevelam, the amount of water present in the hydrated colesevelam (shall be compensated for, thus in order to achieve 0.9 X g of colesevelam (based on dry weight colesevelam), X g of a hydrated colesevelam comprising 10% water would be required.

Suitably, the colesevelam and/or pharmaceutically acceptable salts thereof is present in an amount of from 400 mg to 1250 mg in the tablet core, preferably in an amount of from 400 mg to 1200 mg in the tablet core, such as from 700 to 1200 mg in the tablet core, more preferably in an amount of from 850 mg to 1100 mg in the tablet core. Suitably, the colesevelam and/or pharmaceutically acceptable salts thereof is present in an amount of about 900 mg in the tablet core.

The unit dose may be formulated for delayed release, optionally for delayed release in the ileum. Optionally, the unit dose may be formulated for release starting and reaching 100% release in the ileum prior to entering the colon. The unit dose may be formulated for extended release, optionally for extended release starting in the ileum and continuing into the initial portion of the colon. The unit dose may be formulated for delayed/extended release, optionally for delayed release in the ileum and extended release continuing into the colon, e.g., continuing into the ascending colon.

Optionally, pharmaceutical composition is formulated for release starting and reaching 100% release in the ileum prior to entering the ascending colon.

Optionally, the pharmaceutical composition is formulated for release starting in the ileum and continuing into the colon, e.g., continuing into the ascending colon.

Optionally, the pharmaceutical composition is formulated for release starting in the ileum and continuing throughout the ascending colon.

Optionally, the pharmaceutical composition is formulated for release starting in the ileum and continuing throughout the ascending and transverse colon.

Optionally, the pharmaceutical composition is formulated for release starting in the ileum and continuing throughout the ascending and transverse colon and the descending colon.

Optionally, the pharmaceutical composition is formulated for release starting in the ileum and continuing throughout the ascending and transverse colon and the descending colon and sigmoid colon.

Optionally, the pharmaceutical composition is formulated for release starting in the ileum and continuing throughout the ascending and transverse colon and the descending colon and sigmoid colon and rectum.

Optionally, the non-granulate component of the unit dose pharmaceutical composition may also comprise a diluent.

The diluent (of the granulate component and/or non-granulate component) may be selected from one or more of polyols such as mannitol, lactose; sugars such as sucrose, dextrose, dextrates, sorbitol, fructose; inorganic salts such as dibasic and tribasic calcium phosphate, sodium chloride, starch, modified starch and derivatives thereof, cellulose, a cellulose derivative (i.e., cellulose derivatives such as, for example, methyl, ethyl, hydroxyethyl cellulose and similar), and kaolin. Preferably, the diluent is selected from one or more of mannitol, sucrose, cellulose, and a cellulose derivative.

The diluent (of the granulate component and/or non-granulate component) may be present in an amount of from 5 to 20 wt% of the tablet core, such as from 6 to 18 wt% of the tablet core, optionally from 7 to 16 wt% of the tablet core, optionally from 10 to 15 wt% of the tablet core.

The binder may be selected from one or more of polyvinyl pyrrolidone, polyvinyl alcohol and related graft copolymers, such as polyvinyl alcohol graft polyethylene glycol copolymer, copovidone, cellulose, a cellulose derivative, such as hydroxypropylcellulose, ethylcellulose, and hypromellose, a polymethacrylate, acacia gum and starch or modified starch. Preferably, the binder is polyvinyl pyrrolidone or polyvinyl alcohol.

The binder may be present in an amount of from 1 wt% to 5 wt% of the tablet core, optionally from 2 wt % to 4 wt% of the tablet core.

The composition may optionally include a disintegrant. The disintegrant may be selected from one or more of sodium carboxy methyl cellulose, such as cross-linked sodium carboxy methyl cellulose, crospovidone, bentonite, an algin (e.g., alginic acid or a salt thereof, such as sodium alginate), a gum, and modified starch, preferably, wherein the disintegrant is cross-linked sodium carboxy methyl cellulose or modified starch, more preferably wherein the disintegrant is cross-linked sodium carboxy methyl cellulose.

The disintegrant may be present in an amount of from 0.1 wt% to 7 wt% of the tablet core, optionally from 0.5 wt % to 5 wt%, such as from 1 to 2.5 wt% of the tablet core, optionally from 1 to 2 wt% of the tablet core.

Suitably, the disintegrant is present in the non-granulate component of the unit dose pharmaceutical composition.

The unit dose pharmaceutical composition may further comprise a glidant and/or lubricant.

Optionally, the tablet core comprises a glidant selected from the group comprising: talc, starch, magnesium oxide, silica (silicon dioxide), and a silicate, such as magnesium or calcium silicate. Preferably, the glidant is colloidal silicon dioxide, talc, or magnesium oxide.

Optionally, the tablet core comprises a glidant present in an amount of from 0.1 to 2 wt% of the tablet core, optionally the glidant is present in an amount of from 0.5 to 1.5 wt% of the tablet core, optionally the glidant is present in an amount of from 0.5 to 1 wt% of the tablet core, further, optionally, the glidant is present in an amount of from 0.7 to 1.2 wt% of the tablet core.

Optionally, the tablet core comprises a lubricant selected from the group comprising: stearic acid and salts thereof (e.g., magnesium, calcium or zinc stearate), polyethylene glycol, fumaric acid and salts thereof, glyceryl behenate, glyceryl palmitostearate, wax, poloxamer, sodium benzoate, and any combination thereof. Preferably, the lubricant is stearic acid or a salt thereof, fumaric acid or a salt thereof, or polyethylene glycol.

Optionally, the tablet core comprises a lubricant present in an amount of from 0.1 to 2 wt% of the tablet core, optionally the lubricant is present in an amount of from 0.5 to 1.5 wt% of the tablet core, optionally the lubricant is present in an amount of from 0.5 to 1 wt% of the tablet core, further optionally, the lubricant is present in an amount of from 0.7 to 1.2 wt% of the tablet core.

Optionally, the unit dose pharmaceutical composition further comprises a sub-coating between the tablet core and the gastro-resistant coating, wherein optionally, the sub-coating comprises hydroxypropyl cellulose.

The gastro-resistant coating (film) covering the tablet core may contain a polymethacrylate, acrylic and/or methacrylic acids polymers or copolymers, or a cellulose derivative, such as cellulose acetophthalate. Optionally, the gastro-resistant coating can comprise methacrylic acid/methyl methacrylate (1:1) copolymer (e.g., commercially known as EUDRAGIT L). Optionally, the gastro-resistant coating comprises methacrylic acid/methyl methacrylate (1:2) copolymer (e.g., commercially known as EUDRAGIT S). Optionally, the gastro-protective coating comprises methacrylic acid-ethylacrylate (1:1) copolymer (e.g., commercially known as EUDRAGIT L30 D55). Optionally, the gastro-resistant coating contains polymethacrylate copolymer including, but not limited to, compounds known commercially as EUDRAGIT S (methacrylic acid-methyl methacrylate copolymer 1:2), EUDRAGIT L (methacrylic acid- methyl methacrylate 1:1 copolymer) and/or EUDRAGIT L30 D55 (methylacrylic acid-ethylacrylate 1:1 copolymer), and/or mixtures thereof. The gastro-resistant coating may comprise a methacrylic acid -(meth)acrylate copolymer. Suitably, the gastro-resistant coating comprises methacrylic acid/methyl methacrylate copolymer.

Optionally, the gastro-resistant coating comprises methacrylic acid/methyl methacrylate (1:1) copolymer (EUDRAGIT L).

Optionally, the gastro-resistant coating comprises methacrylic acid/methyl methacrylate (1:2) copolymer (EUDRAGIT S).

Suitably, the gastro-resistant coating comprises a mixture of methacrylic acid/methyl methacrylate (1:1) copolymer and of methacrylic acid/methyl methacrylate (1:2) copolymer. Optionally, the mixture comprises a weight ratio of 0.5:1 to 3:1 of methacrylic acid/methyl methacrylate (1:1) copolymer to methacrylic acid/methyl methacrylate (1:2). Optionally, the mixture comprises a weight ratio of 1:1 to 3:1 of methacrylic acid/methyl methacrylate (1:1) copolymer to methacrylic acid/methyl methacrylate (1:2). Preferably, the mixture comprises a weight ratio of about 2:1 of methacrylic acid/methyl methacrylate (1:1) copolymer to methacrylic acid/methyl methacrylate (1:2).

Optionally, the gastro-resistant coating of the unit dose starts breaking or dissolving at or above a pH of 5, optionally, at or about a pH of 6, more preferably at or above a pH of 6.5, much more preferably at a pH of 6.8.

Optionally, the tablet core has a friability of less than about 1.0%, optionally, less than about 0.5%, preferably less than about 0.1% when measured in accordance with US Pharmacopeia monograph <1216> or with European Pharmacopoeia monograph 2.9.7. For example, the tablet core may have a friability of 0.001 to 1%, or from 0.01 to 0.5%, such as from 0.01 to 0.1% when measured in accordance with US Pharmacopeia monograph <1216> or with European Pharmacopoeia monograph 2.9.7.

Optionally, the tablet core has a hardness of greater than about 70 N, optionally, greater than about 80 N, optionally, greater than about 90 N, when measured in accordance with US Pharmacopeia monograph <1217> or with European Pharmacopoeia monograph 2.9.8. The tablet core may have a hardness in the range of from 70 N to 450 N, such as from 80 N to 400 N, optionally from 90 N to 370 N, such as from 100 N to 330 N when measured in accordance with US Pharmacopeia monograph <1217> or with European Pharmacopoeia monograph 2.9.8.

Suitably, in the unit dose pharmaceutical composition, the colesevelam and/or the pharmaceutically acceptable salts thereof is present in the granulate component in an amount of from 30 to 50 wt% of the tablet core, more preferably 35 to 45 wt% of the tablet core, and suitably, the colesevelam and/or the pharmaceutically acceptable salts thereof is present in the non-granulate component in an amount of from 30 to 50 wt% of the tablet core.

Suitably, in the unit dose pharmaceutical composition, the colesevelam and/or the pharmaceutically acceptable salts thereof is present in the granulate component in an amount of from 30 to 50 wt% of the tablet core, more preferably 35 to 45 wt% of the tablet core, and the colesevelam and/or the pharmaceutically acceptable salts thereof is present in the non-granulate component in an amount of from 30 to 50 wt% of the tablet core, more preferably 35 to 45 wt% of the tablet core, and suitably the total amount of colesevelam and/or pharmaceutically acceptable salts present in the unit dose pharmaceutical composition is present in an amount of at least 76 wt% of the tablet core, and suitably, the total amount of colesevelam and/or pharmaceutically acceptable salts thereof is present in an amount of from 700 mg to 1200 mg in the tablet core, preferably from 850 mg to 1100 mg in the tablet core, such as about 900 mg in the tablet core.

Suitably, the diluent is selected from one or more of mannitol, sucrose and cellulose, and is present in an amount of from 6 wt% to 18 wt% of the tablet core, and the binder is polyvinyl pyrrolidone and is present in an amount of from 2 wt% to 4 wt% of the tablet core.

Suitably, the colesevelam and/or pharmaceutically acceptable salt thereof is colesevelam hydrochloride.

Suitably, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 45% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 40% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 45% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 40% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 45% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 40% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Any of the oral pharmaceutical compositions disclosed herein may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the oral pharmaceutical compositions of the present invention have a dissolution rate lower than about 50% over about 1 hour, when measured at about pH 6.8.

Optionally, the oral pharmaceutical compositions of the present invention have a dissolution rate lower than about 45% over about 1 hour, when measured at about pH 6.8.

Optionally, the oral pharmaceutical compositions of the present invention have a dissolution rate lower than about 40% over about 1 hour, when measured at about pH 6.8.

Optionally, the oral pharmaceutical compositions of the present invention have a dissolution rate lower than about 35% over about 1 hour, when measured at about pH 6.8.

Optionally, the oral pharmaceutical compositions of the present invention have a dissolution rate lower than about 30% over about 1 hour, when measured at about pH 6.8.

Optionally, the oral pharmaceutical compositions of the present invention have a dissolution rate lower than about 25% over about 1 hour, when measured at about pH 6.8.

Optionally, the oral pharmaceutical compositions of the present invention have a dissolution rate lower than about 20% over about 1 hour, when measured at about pH 6.8.

Optionally, the oral pharmaceutical compositions of the present invention have a dissolution rate of greater than or equal to about 80% of colesevelam, or a pharmaceutically acceptable salt thereof, over about 2 hours, when measured at about pH 6.8.

Optionally, the oral pharmaceutical compositions of the present invention have a dissolution rate of greater than or equal to about 80% of colesevelam, or a pharmaceutically acceptable salt thereof, over about 3 hours, when measured at about pH 6.8.

Optionally, the oral pharmaceutical compositions of the present invention have a dissolution rate of greater than or equal to about 80% of colesevelam, or a pharmaceutically acceptable salt thereof, over about 4 hours, when measured at about pH 6.8.

According to the invention, the dissolutions are measured with the with the USP dissolution apparatus type II equipped with a paddle at 100 rpm at 37°±2° C.

In another aspect the present invention provides a method of manufacturing a unit dose pharmaceutical composition for oral administration comprising:
coating a tablet core with a gastro resistant coating, wherein the tablet core comprises:
a granulate component and a non-granulate component, wherein the granulate component comprises colesevelam and/or a pharmaceutically acceptable salt thereof, a diluent and a binder,
wherein the non-granulate component comprises colesevelam or a pharmaceutically acceptable salt thereof, and optionally, one or more of a diluent, a disintegrant, a glidant and a lubricant, and
wherein the colesevelam and/or the pharmaceutically acceptable salts thereof is present in an amount of at least 70 wt% of the tablet core.

In another aspect the present invention provides a method of manufacturing a unit dose pharmaceutical composition for oral administration comprising the steps of:
(i) providing a tablet core wherein the tablet core comprises:
   a granulate component and a non-granulate component, wherein the granulate component comprises colesevelam and/or a pharmaceutically acceptable salt thereof, a diluent and a binder,
   wherein the non-granulate component comprises colesevelam or a pharmaceutically acceptable salt thereof, and optionally, one or more of a diluent, a disintegrant, a glidant and a lubricant, and wherein the colesevelam and/or the pharmaceutically acceptable salts thereof is present in an amount of at least 70 wt% of the tablet core; and
(ii) coating the tablet core with a gastro resistant coating.

The non-granulate component may comprise one or more of a diluent, a disintegrant, a glidant and a lubricant. For example, the non-granulate component may comprise two or more, or three or more, or all of a diluent, a disintegrant, a glidant and a lubricant.

Suitably, the non-granulate component comprises a diluent. Suitably, the non-granulate component comprises a disintegrant. Suitably, the non-granulate component comprises a glidant. Suitably, the non-granulate component comprises a lubricant. Optionally, the non-granulate component comprises a diluent and a disintegrant. Optionally, the non-granulate component comprises a diluent, a disintegrant and a glidant. Optionally, the non-granulate component comprises a diluent, a disintegrant, a glidant and a lubricant.

The method may optionally comprise the step of providing a sub-coating between the non-granulate component and the gastro-resistant coating.

Suitably, the method may comprise the steps of:
(i) combining colesevelam and/or pharmaceutically acceptable salts thereof, a diluent and a binder, to form granules;
(ii) combining colesevelam and/or pharmaceutically acceptable salts thereof and, optionally one or more of a diluent, a disintegrant, a glidant and a lubricant, with the granules of step (i), to form a tablet core having a granulate component and a non-granulate component:
   wherein the granulate component comprises colesevelam and/or a pharmaceutically acceptable salt thereof, a diluent and a binder;
   wherein the non-granulate component comprises colesevelam or a pharmaceutically acceptable salt thereof, and, optionally, a diluent, a disintegrant, a glidant and lubricants; wherein the colesevelam and/or the pharmaceutically acceptable salts thereof is present in an amount of at least 70 wt% of the tablet core; and coating the tablet core with a gastro resistant coating and optionally with a sub-coating between the tablet core and the gastro resistant coating.

The method may comprise dry or wet granulation, preferably wet granulation.

The wet granulation may be carried out using an aqueous or non-aqueous solvent, preferably a non-aqueous solvent, such as an organic solvent. Optionally, the organic solvent may be an alcohol, a ketone or a chlorinated solvent. Suitably, the alcohol is selected from methanol, ethanol, propanol and isopropanol, or mixtures thereof. Preferably, the alcohol is ethanol.

The method may involve bulk manufacture of a plurality of unit doses.

For example, the method may involve bulk manufacture of a plurality of unit doses comprising a pharmaceutical composition for oral administration, wherein the plurality of unit doses has a uniformity of mass of less than ±2%, preferably less than ±1%, more preferably less than ±0.8%.

The obtained tablets are tested in a dissolution test USP <711> apparatus II, 0.1N HCl for 2 hours and then 8 hours at pH 6.8 (phosphate buffer). To 1000 ml of phosphate buffer at pH 6.8 a fixed amount of sodium taurodeoxycholate is added. Coated tablets remain intact for 2 hours in 0.1 N HCl. Thereafter the tablets are withdrawn from the above medium and after draining are placed in the pH 6.8 medium. The coating starts to dissolve, the colesevelam starts to swell and binds the taurodeoxycholate in the medium. The dissolution of colesevelam along time is calculated by difference on the residual amount of free, non-bound taurodeoxycholate in the medium.

The present disclosure provides a unit dose oral pharmaceutical composition (or one or more unit doses) as described herein for use in a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering more than one of said unit dose oral pharmaceutical compositions to said patient.

The present disclosure provides further provides a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, comprising administering to said patient a unit does oral pharmaceutical composition as described herein, optionally, wherein the method comprises administering more than one of said unit dose oral pharmaceutical compositions to said patient.

The pharmaceutical compositions of the present invention can be used in the treatment of bile acid diarrhea. This bile acid diarrhea may be idiopathic (e.g., primary bile acid diarrhea) or secondary to an underlying disease. The underlying disease can be: Crohn's disease, ileal resection or radiation ileitis, chronic pancreatitis, celiac disease, cholecystectomy, small intestine bacterial overgrowth, irritable bowel syndrome subtype diarrhea (IBS-D) and similar.

Suitably, the method of treating bile acid diarrhea comprises administering to a subject in need thereof, a therapeutically effective amount of a pharmaceutical composition described herein. The unit dose oral pharmaceutical composition can be administered either in a single non-fractionated administration or in multiple fractionated administrations.

The present invention allows for a reduction in the number of tablets and frequency of administration per day (e.g., two tablets twice per day, or one tablet twice per day) in comparison to current treatments, thus advantageously leading to an improvement in patient compliance. The pharmaceutical compositions of the present invention can be used in the treatment of irritable bowel syndrome subtype diarrhea (IBS-D).

Suitably, the method of treating irritable bowel syndrome subtype diarrhea (IBS-D) comprises administering to a subject in need thereof, a therapeutically effective amount of a pharmaceutical composition described herein.

The pharmaceutical compositions of the present invention can be used in the treatment of microscopic colitis.

Suitably, the method of treating microscopic colitis comprises administering to a subject in need thereof, a therapeutically effective amount of a pharmaceutical composition described herein. The pharmaceutical compositions of the present invention can be used in the treatment of bile acid malabsorption.

Suitably, the method of treating bile acid malabsorption comprises administering to a subject in need thereof, a therapeutically effective amount of a pharmaceutical composition described herein. The tablets can be administered either in a single non-fractionated administration or in multiple fractionated administrations (e.g., six tablets once per day, or three tablets twice per day, or two tablets thrice per day).

The present invention also provides for the use of a unit dose pharmaceutical composition for oral administration as described herein, for treating bile acid diarrhea, irritable bowel syndrome, optionally, irritable bowel syndrome subtype diarrhea (IBS-D), colitis, such as microscopic colitis, and/or bile acid malabsorption in a patient in need thereof, wherein optionally said use comprises administering more than one of said unit dose oral pharmaceutical compositions to said patient.

Furthermore, the present invention provides for use of a unit dose pharmaceutical composition for oral administration as described herein in the manufacture of a medicament for treating bile acid diarrhea, irritable bowel syndrome, optionally, irritable bowel syndrome subtype diarrhea (IBS-D), colitis, such as microscopic colitis, and/or bile acid malabsorption.

The present invention provides:
a unit dose pharmaceutical composition for oral administration comprising a tablet core and a gastro-resistant coating covering the core,
wherein the tablet core comprises a granulate component and a non-granulate component:
   wherein the granulate component comprises colesevelam and/or a pharmaceutically acceptable salt thereof, a diluent and a binder;
   wherein the non-granulate component comprises colesevelam or a pharmaceutically acceptable salt thereof and optionally one or more of: a diluent, a disintegrant, a glidant and a lubricant;
   wherein the colesevelam and/or the pharmaceutically acceptable salts thereof is present in an amount of at least 66 wt% of the tablet core, such as at least about 70 wt% of the tablet core.

The colesevelam and/or pharmaceutically acceptable salts thereof may be present in the granulate component in an amount of from 30 to 60 wt% of the tablet core, preferably 30 to 50 wt% of the tablet core, such as 35 to 50 wt% of the tablet core, more preferably 35 to 45 wt% of the tablet core, optionally, in an amount of from 36 to 45 wt% of the tablet core, such as from 37 to 45 wt% of the tablet core.

The colesevelam and/or pharmaceutically acceptable salts thereof may be present in the non-granulate component in an amount of from 30 to 60 wt% of the tablet core, preferably 30 to 50 wt% of the tablet core, such as 35 to 50 wt% of the tablet core, more preferably 35 to 45 wt% of the tablet core, optionally, in an amount of from 36 to 45 wt% of the tablet core, such as from 37 to 45 wt% of the tablet core.

The colesevelam and/or pharmaceutically acceptable salts thereof may be present in an amount of at least 76 wt% of the tablet core, preferably at least 80 wt% of the tablet core, more preferably at least 81 wt% of the tablet core, optionally at least 82 wt% of the tablet core.

The colesevelam and/or pharmaceutically acceptable salts thereof is present in an amount of from 400 mg to 1250 mg in the tablet core, preferably in an amount of from 400 mg to 1200 mg in the tablet core, such as from 700 to 1200 mg in the tablet core, more preferably in an amount of from 850 mg to 1100 mg in the tablet core.

The colesevelam and/or the pharmaceutically acceptable salts thereof may be present in the granulate component in an amount of from 30 to 50 wt% of the tablet core, more preferably 35 to 45 wt% of the tablet core, and wherein the colesevelam is present in the non-granulate component in an amount of from 30 to 50 wt% of the tablet core, more preferably 35 to 45 wt% of the tablet core, and wherein colesevelam and/or pharmaceutically acceptable salts thereof is present in a total amount of at least 76 wt% of the tablet core, and wherein the colesevelam and/or pharmaceutically acceptable salts thereof is present in an amount of from 700 mg to 1200 mg in the tablet core, preferably from 850 mg to 1100 mg in the tablet core, such as about 900 mg in the tablet core.

The tablet core may have a friability of < 1.0%, optionally, < 0.5%, preferably < 0.1% when measured in accordance with US Pharmacopeia monograph <1216> or with European Pharmacopoeia monograph 2.9.7.

The tablet core may have a hardness of > 70 N, optionally > 80 N, optionally, > 90 N, when measured in accordance with US Pharmacopeia monograph <1217> or with European Pharmacopoeia monograph 2.9.8.

Suitably, in the unit dose pharmaceutical composition of the present invention, colesevelam and/or the pharmaceutically acceptable salts thereof is present in an amount of from 76 wt% to 90 wt%, suitably from 80 wt% to 90 wt%;
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, such as in an amount of from 850 mg to 1100 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Suitably, the colesevelam and/or pharmaceutically acceptable salts thereof is present in an amount of about 900 mg in the tablet core.

Suitably, the non-granulate component comprises one or more of a diluent, a disintegrant, a glidant and a lubricant;
optionally, wherein the diluent is selected from one or more of polyols such as mannitol, lactose, sugars such as sucrose, dextrose, dextrates, sorbitol, fructose, inorganic salts such as dibasic and tribasic calcium phosphate, sodium chloride, starch, modified starch and derivatives thereof, cellulose, a cellulose derivative (i.e. cellulose derivatives such as, for example, methyl, ethyl, or hydroxyethyl cellulose), and kaolin; further optionally, wherein the diluent is present in an amount of from 5 to 20 wt% of the tablet core, such as from 6 to 18 wt% of the tablet core, preferably from 7 to 16 wt% of the tablet core, more preferably from 10 to 15 wt% of the tablet core;
optionally, wherein the binder is selected from one or more of polyvinyl pyrrolidone, polyvinyl alcohol, copovidone, cellulose, a cellulose derivative (e.g. hydroxypropyl cellulose, hypromellose, ethyl cellulose), acacia gum, starch, and a polymethylacrylate, preferably polyvinyl pyrrolidone and/or poly vinyl alcohol; further optionally, wherein the binder is present in an amount of from 1 wt% to 5 wt% of the tablet core, preferably from 2 wt % to 4 wt% of the tablet core;
optionally, wherein the disintegrant is selected from one or more of sodium carboxy methyl cellulose, such as cross-linked sodium carboxy methyl cellulose, crospovidone, bentonite, an algin (e.g., alginic acid or a salt thereof, such a sodium alginate), a gum and modified starch, preferably, wherein the disintegrant is cross-linked sodium carboxy methyl cellulose, further optionally, wherein the disintegrant is present in an amount of from 0.1 wt% to 7 wt% of the tablet core, preferably from 0.5 wt % to 5 wt%, such as from 1 to 2.5 wt% of the tablet core, more preferably from 1 to 2 wt% of the tablet core.

For example, a unit dose pharmaceutical composition of the present invention may comprise colesevelam and/or the pharmaceutically acceptable salts thereof is present in an amount of from 81 wt% to 90 wt% of the tablet core,
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein said diluent is selected from one or more of mannitol, sucrose, cellulose, and a cellulose derivative, wherein the diluent is present in an amount of from about 7 to about 16 wt% of the tablet core;
wherein said binder is selected from one or more of polyvinyl pyrrolidone, polyvinyl alcohol and related graft copolymers, such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 4 wt % of the tablet core;
wherein the non-granulate component further comprises a lubricant and a glidant;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8, and wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Suitably, the gastro-resistant coating of the unit dose starts breaking or dissolving at or above a pH of 6.0, preferably at or above a pH of 6.5, more preferably at a pH of 6.8.

Preferably, the colesevelam and/or pharmaceutically acceptable salt thereof is colesevelam hydrochloride.

Also disclosed herein is:
a method of manufacturing a unit dose pharmaceutical composition for oral administration comprising the steps of:
(a) providing a tablet core wherein the tablet core comprises:
   a granulate component and a non-granulate component,
   wherein the granulate component comprises colesevelam and/or a pharmaceutically acceptable salt thereof, a diluent and a binder,
   wherein the non-granulate component comprises colesevelam or a pharmaceutically acceptable salt thereof, and optionally, one or more of a diluent, a disintegrant, a glidant and a lubricant, and
   wherein the colesevelam and/or the pharmaceutically acceptable salts thereof is present in an amount of at least 66 wt% of the tablet core, preferably at least 70 wt% of the tablet core; and
(b) coating the tablet core with a gastro resistant coating and optionally a sub-coating.

The method may comprise the steps of:
(i) combining colesevelam and/or pharmaceutically acceptable salts thereof, a diluent and a binder, to form a granulate;
(ii) combining colesevelam and/or pharmaceutically acceptable salts thereof and, optionally, a diluent and a disintegrant, with the granulate of step (i), to form a tablet core having a granulate component and a non-granulate component:
   wherein the granulate component comprises colesevelam and/or a pharmaceutically acceptable salt thereof, a diluent and a binder;
   wherein the non-granulate component comprises colesevelam or a pharmaceutically acceptable salt thereof, and, optionally, one or more of a diluent, a disintegrant, a glidant and lubricants;
   wherein the colesevelam and/or the pharmaceutically acceptable salts thereof is present in an amount of at least 66 wt% of the tablet core, preferably at least 70 wt% of the tablet core; and
(iii) coating the tablet core with a gastro resistant coating and optionally a sub-coating;
   wherein optionally, step (i) involves wet granulation, further optionally,
   the wet granulation is carried out using water or a non-aqueous solvent, preferably a non-aqueous solvent, such as an organic solvent, such as an alcohol, preferably ethanol, a ketone or a chlorinated solvent.

The method may comprise bulk manufacture of a plurality of unit doses comprising a pharmaceutical composition for oral administration, wherein the plurality of unit doses has a uniformity of mass of less than ±2%, preferably less than ±1%, more preferably less than ±0.8%.

Also disclosed is a unit dose pharmaceutical composition for oral administration as described herein for use in a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering more than one of said unit dose oral pharmaceutical compositions to said patient.

Disclosed herein is a unit dose pharmaceutical composition for oral administration comprising a tablet core and a gastro-resistant coating covering the core,
wherein the tablet core comprises a granulate component and a non-granulate component:
wherein the granulate component comprises colesevelam hydrochloride, a diluent and a binder;
wherein the non-granulate component comprises colesevelam hydrochloride and optionally one or more of: a diluent, a disintegrant, a glidant and a lubricant;
wherein the colesevelam hydrochloride is present in an amount of about 66 wt% to about 90 wt% of the tablet core.

For example, colesevelam hydrochloride may be present in the granulate component in an amount of from about 37 wt% to about 45 wt% of the tablet core. Colesevelam hydrochloride may be present in the non-granulate component in an amount of from about 37 wt% to about 45 wt% of the tablet core.

The colesevelam hydrochloride is present in an amount of from about 700 to about 1200 mg in the tablet core. Suitably, colesevelam hydrochloride is present in an amount of about 900 mg in the tablet core.

### DEFINITIONS

As used herein the term "active pharmaceutical ingredient" ("API") or "pharmaceutically active agent" is a drug or agent which can be employed for the compositions and methods of the disclosure and is intended to be used in the human or animal body in order to heal, to alleviate, to prevent or to diagnose diseases, ailments, physical damage or pathological symptoms; allow the state, the condition or the functions of the body or mental states to be identified; to replace active substances produced by the human or animal body, or body fluids; to defend against, to eliminate or to render innocuous pathogens, parasites or exogenous substances or to influence the state, the condition or the functions of the body or mental states. Drugs in use can be found in reference works such as, for example, the Rote Liste or the Merck Index. Examples which may be mentioned include, for example, colesevelam.

As used herein, "pharmaceutically acceptable salts" refer to derivatives of the disclosed compounds wherein the therapeutic compound is modified by making acid or base salts thereof. Examples of pharmaceutically acceptable salts include, but are not limited to, mineral or organic acid salts of the active agent. The pharmaceutically acceptable salts include the conventional non-toxic salts, for example, from non-toxic inorganic or organic acids. For example, such conventional non-toxic salts include those derived from inorganic acids such as hydrochloric, hydrobromic, sulfuric, sulfonic, sulfamic, phosphoric, nitric and the like; and the salts prepared from organic acids such as amino acids, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and other known to those of ordinary skill in the pharmaceutical sciences. Lists of suitable salts are found in texts such as Remington 's Pharmaceutical Sciences, 18th Ed. (Alfonso R. Gennaro, ed.; Mack Publishing Company, Easton, Pa., 1990); Remington: the Science and Practice of Pharmacy 19th Ed. (Lippincott, Williams & Wilkins, 1995); Handbook of Pharmaceutical Excipients, 3K| Ed. (Arthur H. Kibbe, ed.; Amer. Pharmaceutical Assoc., 1999); the Pharmaceutical Codex: Principles and Practice of Pharmaceutics 12th Ed. (Walter Lund ed.; Pharmaceutical Press, London, 1994); The United States Pharmacopeia: The National Formulary (United States Pharmacopeial Convention); and Goodman and Gilman's: the Pharmacological Basis of Therapeutics (Louis S. Goodman and Lee E. Limbird, eds.; McGraw Hill, 1992), the disclosures of which are hereby incorporated by reference.

An amount is "effective" as used herein, when the amount provides an effect in the subject. As used herein, the term "effective amount" means an amount of a compound or composition sufficient to significantly induce a positive benefit, including independently or in combinations the benefits disclosed herein, but low enough to avoid serious side effects, i.e., to provide a reasonable benefit to risk ratio, within the scope of sound judgment of the skilled artisan. For those skilled in the art, the effective amount, as well as dosage and frequency of administration, may be determined according to their knowledge and standard methodology of merely routine experimentation based on the present disclosure.

As used herein, the terms "subject" and "patient" are used interchangeably. As used herein, the term "patient" refers to an animal, preferably a mammal such as a non-primate (e.g., cows, pigs, horses, cats, dogs, rats etc.) and a primate (e.g., monkey and human), and most preferably a human. In some embodiments, the subject is a non-human animal such as a farm animal (e.g., a horse, pig, or cow) or a pet (e.g., a dog or cat). In a specific embodiment, the subject is an elderly human. In another embodiment, the subject is a human adult. In another embodiment, the subject is a human child. In yet another embodiment, the subject is a human infant.

As used herein, the phrase "pharmaceutically acceptable" means approved by a regulatory agency of the federal or a state government, or listed in the U.S. Pharmacopeia, European Pharmacopeia, or other generally recognized pharmacopeia for use in animals, and more particularly, in humans.

As used herein, the terms "prevent," "preventing" and "prevention" in the context of the administration of a therapy to a subject refer to the prevention or inhibition of the recurrence, onset, and/or development of a disease or condition, or a combination of therapies (e.g., a combination of prophylactic or therapeutic agents).

As used herein, the terms "therapies" and "therapy" can refer to any method(s), composition(s), and/or agent(s) that can be used in the prevention, treatment and/or management of a disease or condition, or one or more symptoms thereof.

As used herein, the terms "treat," "treatment," and "treating" in the context of the administration of a therapy to a subject refer to the reduction or inhibition of the progression and/or duration of a disease or condition, the reduction or amelioration of the severity of a disease or condition, and/or the amelioration of one or more symptoms thereof resulting from the administration of one or more therapies.

As used herein, the term "about" when used in conjunction with a stated numerical value or range has the meaning reasonably ascribed to it by a person skilled in the art, i.e. denoting somewhat more or somewhat less than the stated value or range. For example, for each specified value "about" may be ±10%, suitably ±5%, suitably ±2%, suitably ±1%.

As used herein, the symbol "wt%" or "% w/w" indicates the weight percent of a substance in a mixture (e.g., a mixture of solid substances, or in a solution), which is calculated as the mass of the component divided by the total mass of the mixture and then multiplied by 100 to provide the percentage. Usually, mass is expressed in grams, but any unit of measurement is acceptable as long as the same units for both the component or solute mass and the total or solution mass are used in the calculation.

Colesevelam hydrochloride is poly(allylamine hydrochloride) cross-linked with epichlorohydrin and alkylated with 1-bromodecane and (6-bromohexyl)-trimethylammonium bromide. The chemical name (IUPAC) of colesevelam hydrochloride is allylamine polymer with 1-chloro-2,3-epoxypropane,[6-(allylamino)-hexyl]trimethylammonium chloride and N-allyldecylamine, hydrochloride. The chemical structure of colesevelam hydrochloride is represented by the following formula: 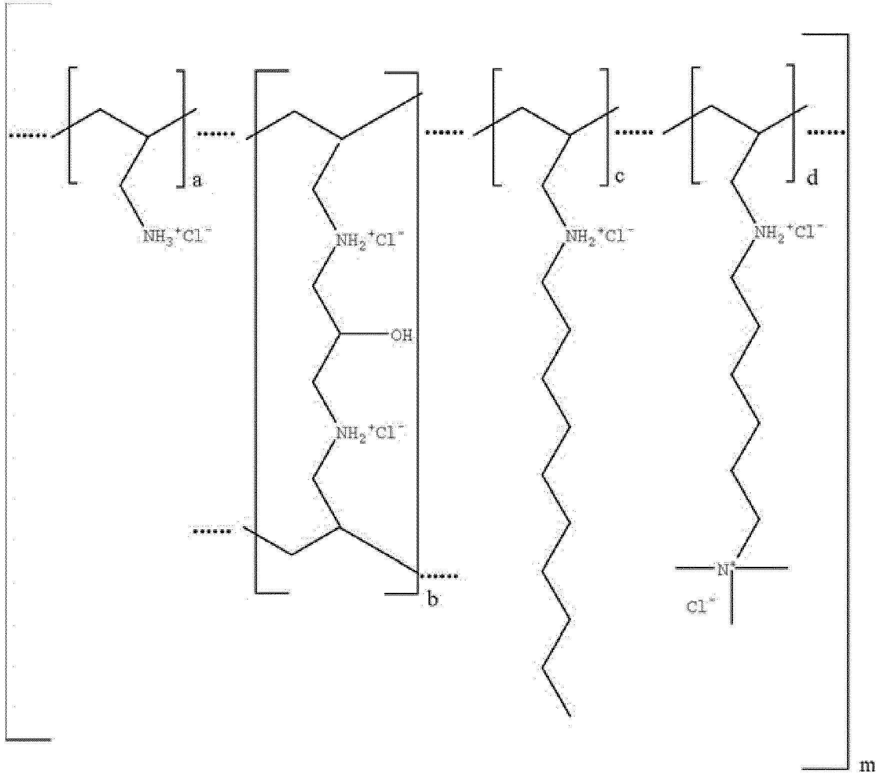 wherein (a) represents allyl amine monomer units that have not been alkylated by either of the 1-bromodecane or (6-bromohexyl)-trimethylammonium bromide alkylating agents or cross-linked by epichlorohydrin; (b) represents allyl amine units that have undergone cross-linking with epichlorohydrin; (c) represents allyl amine units that have been alkylated with a decyl group; (d) represents allyl amine units that have been alkylated with a (6-trimethylammonium) hexyl group, and m represents a number ≥ 100 to indicate an extended polymer network. A small amount of the amines are dialkylated and are not depicted in the formula above. No regular order of the groups is implied by the structure; cross-linking and alkylation are expected to occur randomly along the polymer chains. A large amount of the amines are protonated. The polymer is depicted in the hydrochloride form; a small amount of the halides are bromide. Colesevelam hydrochloride is hydrophilic and insoluble in water. Preferably, the present invention employs colesevelam hydrochloride, however, the person skilled in the art will appreciate that alternative pharmaceutically acceptable salts, or the free base version of colesevelam may be employed in the present invention.

### Brief Description of the Drawings

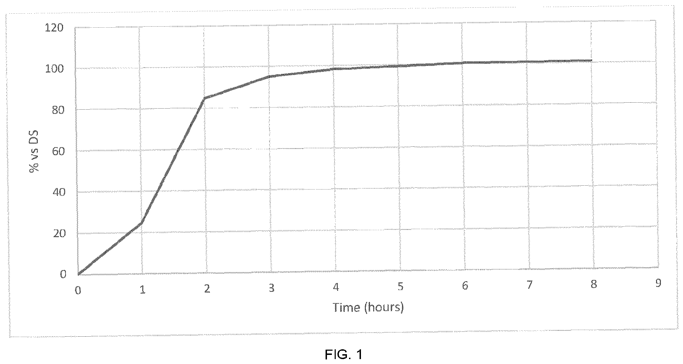

FIG. 1 shows the binding capacity of colesevelam HCl for taurodeoxycholate (DS) over time. The Y axis label "% vs DS", refers to the binding capacity of colesevelam hydrochloride versus the maximum binding capacity of the drug substance (colesevelam raw material). A unit dose pharmaceutical composition tablet according to the invention as described in Example 2B (Formulation G2, coated in the same manner as Formulation F described below) was added to 0.1 N HCl for 2 hours, and then the tablets (which remained intact during the course of the acid treatment and were drained of any residual acid) were added to phosphate buffer medium at pH 6.8 comprising a known amount of taurodeoxycholate. As the gastric resistant coating of the tablet dissolves (at pH 6.8) colesevelam HCl is released and swells and binds the taurodeoxycholate in the medium. The amount of unbound taurodeoxycholate is monitored over time, and the binding capacity of colesevelam is calculated by the consumption of the free non-bound taurodeoxycholate.

### DETAILED DESCRIPTION

The present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof and optionally one or more of a diluent, a disintegrant, a glidant and a lubricant; and
wherein the colesevelam and/or the pharmaceutically acceptable salts thereof is present in an amount of at least 66 wt% of the tablet core.

The present inventors discovered that tablet cores having a surprisingly high loading of colesevelam and/or a pharmaceutically acceptable salt thereof could unexpectedly be manufactured by splitting a high dose of colesevelam and/or pharmaceutically acceptable salts thereof into a granulate component and a non-granulate component, and tabletting said granulate and non-granulate components. In particular, and without wishing to be bound by a particular theory, the inventors believe that providing a unit dose pharmaceutical composition comprising colesevelam hydrochloride in granulate and non-granulate components enables preparation of tablets having excellent hardness and low friability. Tablets having these qualities are suitable for manufacturing, transportation, and storage, and as discussed elsewhere herein, due to their high API loading, enable physicians to reduce the number of tablets patients are required to consume. Because patients are required to consume fewer tablets throughout the course of the day, there is an increased likelihood of patient compliance with a prescribed dosing regimen, and relatedly, increased efficacy. Importantly, preparing tablets having an increased concentration of colesevlam hydrochloride and the presently noted mechanical properties was not a mere optimization or revision of certain prior art formulation disclosed, for example, in WO2021/163007. As described in detail elsewhere herein, the formulations described in WO2021/163007 were either unsuitable for tableting altogether at the desired API loading or were unable to provide tablets having both the desired API loading and mechanical properties necessary for manufacturing, transportation, and storage. Thus, the inventors, through the extensive research and development described herein, solved the problem of providing colesevelam unit dosage forms for oral administration having high colesevlam loading and suitable mechanical properties - a problem the prior art did not recognize or provide any guidance on solving. In particular, the inventors, through the extensive trial and error detailed elsewhere herein, solved the problem of providing colesevelam hydrochloride unit dosage forms having high colesevlam hydrochloride loading and suitable mechanical properties - a problem the prior art did not recognize or provide any guidance on solving.

The total amount of colesevelam and/or pharmaceutically acceptable salts thereof in the unit dose pharmaceutical composition for oral administration accounts for at least 66 wt% of the tablet core, such as at least about 70 wt% of the tablet core. The total amount of colesevelam and or pharmaceutically acceptable salts thereof in the unit dose includes the amount of colesevelam and/or pharmaceutically acceptable salts thereof in the granulate component and in the non-granulate component. The granulate component colesevelam and/or a pharmaceutically acceptable salt thereof, diluent, and a binder. For example, the granulate component comprises granules comprising colesevelam and/or a pharmaceutically acceptable salt thereof, diluent, and a binder. The granulate is manufactured by granulation using a granulator.

The unit dose oral pharmaceutical composition (or one or more unit doses) of the present invention may be used to treat diseases or conditions such as bile acid diarrhea, irritable bowel syndrome, optionally, irritable bowel syndrome subtype diarrhea (IBS-D), colitis, such as microscopic colitis, and/or bile acid malabsorption in a patient in need thereof. The treatment may comprise administering more than one of said unit dose oral pharmaceutical compositions to said patient.

Given the chronicity of the aforementioned diseases/conditions, treatment with prior art or commercially available colesevelam tablets which comprise lower amounts and loadings of colesevelam requires the administration of a large number of tablets per day. This often leads to patient compliance issues. Advantageously, the unit dose oral pharmaceutical composition of the present invention allows for a reduction in the number of tablets and a reduction in the frequency of administration per day (e.g, two tablets twice per day, or one tablet twice per day), which leads to an improvement in patient compliance.

As outlined above, the amounts of colesevelam or pharmaceutically acceptable salts thereof refer to amounts of the anhydrous substances (i.e., dry weight percentages). The colesevelam or pharmaceutically acceptable salts thereof suitable for the present invention may comprise some amount of water, i.e., hydrated colesevelam or pharmaceutically acceptable salts thereof may be used, however, the amount of hydrated colesevelam or pharmaceutically acceptable salts thereof used is determined based on a dry weight percentage. Suitably, such hydrated colesevelam or pharmaceutically acceptable salts thereof comprise less than about 20 wt% of the total weight of the hydrated substance, preferably less than about 10 wt%, e.g., 1 wt%, or 2 wt%, or 3 wt%, or 4 wt%, or 5 wt%, or 6 wt%, or 7 wt%, or 8 wt%, or 9 wt%, or any fractions of the above integers. As known in the art, such amount of water shall be compensated through an overweight of colesevelam or salts thereof in order to achieve the target amounts according to the present disclosure.

Suitably, the non-granulate component is a powder or pulverulent substance.

Suitably, the granulate component is a granulate formed by a granulation method, for example using a granulator.

Suitably, the granulate component comprises granules having a particle size distribution with a D50 between 125 and 250 µm and a D90 of not more than 850 µm as determined in accordance with European Pharmacopoeia monograph 2.9.38 or US Pharmacopoeia monograph <786>.

The colesevelam and/or pharmaceutically acceptable salts thereof may be present in the granulate component in an amount of from 30 to 60 wt% of the tablet core, optionally 30 to 50 wt% of the tablet core, such as 35 to 50 wt% of the tablet core, optionally 35 to 45 wt% of the tablet core, further optionally in an amount of from 36 to 45 wt% of the tablet core, or 37 to 45 wt% of the tablet core. For example, the colesevelam and/or pharmaceutically acceptable salts thereof may be present in the granulate component in an amount of from 35 to 45 wt% of the tablet core, such as in an amount of from 36 to 44 wt% of the tablet core, such as in an amount of from 37 to 43 wt% of the tablet core. By way of further example, the colesevelam hydrochloride may be present in the granulate component in an amount of from 35 to 45 wt% of the tablet core, such as in an amount of from 36 to 44 wt% of the tablet core, such as in an amount of from 37 to 43 wt% of the tablet core.

The colesevelam and/or pharmaceutically acceptable salts thereof may be present in the granulate component in an amount of from 30 to 60 wt% of the tablet core, such as 30 wt%, or 31 wt%, or 32 wt% or 33 wt%, or 34 wt%, or 35 wt%, or 36 wt%, or 37 wt%, or 38 wt%, or 39 wt%, or 40 wt%, or 41 wt%, or 42 wt%, or 43 wt%, or 44 wt%, or 45 wt%, or 46 wt%, or 47 wt%, or 48 wt%, or 49 wt%, or 50 wt%, or 51 wt%, or 52 wt%, or 53 wt%, or 54 wt%, or 55 wt%, or 56 wt%, or 57 wt%, or 58 wt%, or 59 wt%, or 60 wt%.

Preferably, the colesevelam and/or pharmaceutically acceptable salts thereof may be present in the granulate component in an amount of 45 wt% of the tablet core, preferably of 47.5 wt%, more preferably of 50 wt%. For example, colesevelam hydrochloride may be present in the granulate component in an amount of 45 wt% of the tablet core, preferably of 47.5 wt%, more preferably of 50 wt%.

The colesevelam and/or pharmaceutically acceptable salts thereof may be present in the non-granulate component in an amount of from 30 to 60 wt% of the tablet core, optionally 30 to 50 wt% of the tablet core, optionally 35 to 45 wt% of the tablet core, further optionally in an amount of from 36 to 45 wt% of the tablet core, such as in an amount of from 37 to 45 wt% of the tablet core. For example, the colesevelam and/or pharmaceutically acceptable salts thereof may be present in the granulate component in an amount of from 35 to 45 wt% of the tablet core, such as in an amount of from 36 to 44 wt% of the tablet core, such as in an amount of from 37 to 43 wt% of the tablet core. By way of further example, colesevelam hydrochloride may be present in the granulate component in an amount of from 35 to 45 wt% of the tablet core, such as in an amount of from 36 to 44 wt% of the tablet core, such as in an amount of from 37 to 43 wt% of the tablet core.

The colesevelam and/or pharmaceutically acceptable salts thereof may be present in the non-granulate component in an amount of from 30 to 60 wt% of the tablet core, such as 30 wt%, or 31 wt%, or 32 wt% or 33 wt%, or 34 wt%, or 35 wt%, or 36 wt%, or 37 wt%, or 38 wt%, or 39 wt%, or 40 wt%, or 41 wt%, or 42 wt%, or 43 wt%, or 44 wt%, or 45 wt%, or 46 wt%, or 47 wt%, or 48 wt%, or 49 wt%, or 50 wt%, or 51 wt%, or 52 wt%, or 53 wt%, or 54 wt%, or 55 wt%, or 56 wt%, or 57 wt%, or 58 wt%, or 59 wt%, or 60 wt%.

Preferably, the colesevelam and/or pharmaceutically acceptable salts thereof may be present in the non-granulate component in an amount of 45 wt% of the tablet core, preferably of 47.5 wt%, more preferably of 50 wt%. For example, colesevelam hydrochloride may be present in the non-granulate in an amount of 45 wt% of the tablet core, preferably of 47.5 wt%, more preferably of 50 wt%.

Friability of the tablet cores is reduced (i.e., the tablet cores are less friable) when the colesevelam and/or pharmaceutically acceptable salts thereof is present in the granulate component in an amount of from 30 to 60 wt% of the tablet core, optionally 30 to 50 wt% of the tablet core, optionally 35 to 45 wt% of the tablet core, such as from 36 wt% to 45 wt%, for example 37 wt% to 45 wt% of the tablet core; and when the colesevelam and/or pharmaceutically acceptable salts thereof is present in the non-granulate component in an amount of from 30 to 60 wt% of the tablet core, optionally 30 to 50 wt% of the tablet core, optionally 35 to 45 wt% of the tablet core, such as from 36 wt% to 45 wt%, for example 37 wt% to 45 wt% of the tablet core. In particular aspects, friability of the tablet cores is reduced when the colesevelam hydrochloride is present in the granulate component in an amount of from 30 to 60 wt% of the tablet core, optionally 30 to 50 wt% of the tablet core, optionally 35 to 45 wt% of the tablet core, such as from 36 wt% to 45 wt%, for example 37 wt% to 45 wt% of the tablet core; and when colesevelam hydrochloride is present in the non-granulate component in an amount of from 30 to 60 wt% of the tablet core, optionally 30 to 50 wt% of the tablet core, optionally 35 to 45 wt% of the tablet core, such as from 36 wt% to 45 wt%, for example 37 wt% to 45 wt% of the tablet core.

If less than about 30 wt% of the tablet core of colesevelam is contained in the non-granulate component hardness is reduced and friability is increased.

Together the amount of colesevelam and/or pharmaceutically acceptable salts thereof in the non-granulate component and the amount in the granulate component account for at least 70 wt% of the tablet core. For example, the amount of colesevelam hydrochloride in the non-granulate component and the amount in the granulate component account for at least 70 wt% of the tablet core.

Suitably, colesevelam and/or pharmaceutically acceptable salts thereof is present (in the unit dose pharmaceutical composition for oral administration) in an amount of at least 70 wt% or at least 75 wt% or at least 76 wt% of the tablet core, suitably at least 80 wt% of the tablet core, more suitably at least 81 wt% of the tablet core, optionally at least 82 wt% of the tablet core. For example, and in some aspects, colesevelam hydrochloride is present (in the unit dose pharmaceutical composition for oral administration) in an amount of at least 70 wt% or at least 75 wt% or at least 76 wt% of the tablet core, suitably at least 80 wt% of the tablet core, more suitably at least 81 wt% of the tablet core, optionally at least 82 wt% of the tablet core.

Suitably, colesevelam and/or pharmaceutically acceptable salts thereof is present (in the unit dose pharmaceutical composition for oral administration) form in an amount of at least 70 wt% of the tablet core, such as at least 71 wt%, or at least 72 wt%, or at least 73 wt%, or at least 74 wt%, or at least 75 wt%, or at least 76 wt%, or at least 77 wt%, or at least 78 wt%, or at least 79 wt%, or at least 80 wt%, or at least 81 wt%, or at least 82 wt%, or at least 83 wt%, or at least 84 wt%, or at least 85 wt%, or at least 86 wt%, or at least 87 wt%, or at least 88 wt%, or at least 89 wt%, or at least 90 wt%, of the tablet core. For example, and in some aspects, colesevelam hydrochloride is present (in the unit dose pharmaceutical composition for oral administration) in an amount of at least 70 wt% of the tablet core, such as at least 71 wt%, or at least 72 wt%, or at least 73 wt%, or at least 74 wt%, or at least 75 wt%, or at least 76 wt%, or at least 77 wt%, or at least 78 wt%, or at least 79 wt%, or at least 80 wt%, or at least 81 wt%, or at least 82 wt%, or at least 83 wt%, or at least 84 wt%, or at least 85 wt%, or at least 86 wt%, or at least 87 wt%, or at least 88 wt%, or at least 89 wt%, or at least 90 wt%, of the tablet core.

Preferably, colesevelam and/or pharmaceutically acceptable salts thereof is present in the unit dose pharmaceutical composition for oral administration in an amount of at least 75 wt% of the tablet core, such as in an amount of at least 76 wt% of the tablet core, preferably at least 80 wt% of the tablet core, more preferably at least 81 wt% of the tablet core, optionally, at least 82 wt% of the tablet core. In typical aspects, the colesevelam present in the unit dose pharmaceutical composition for oral administration in the amounts noted herein is colesevelam hydrochloride.

For example, colesevelam and/or pharmaceutically acceptable salts thereof is present in the unit dose pharmaceutical composition for oral administration in an amount of 70 wt% to 90 wt% of the tablet core, or in an amount of 75 wt% to 90 wt% of the tablet core, or in an amount of 76 wt% to 90 wt% of the tablet core, suitably in an amount of 80 wt% to 90 wt% of the tablet core, more suitably 81 wt% to 90 wt% of the tablet core, optionally 82 wt% to 90 wt% of the tablet core. Optionally, colesevelam and/or pharmaceutically acceptable salts thereof is present in the unit dose pharmaceutical composition for oral administration in an amount of 70 wt% to 88 wt% of the tablet core, or in an amount of 75 wt% to 88 wt% of the tablet core, or in an amount of 76 wt% to 88 wt% of the tablet core, suitably in an amount of 80 wt% to 88 wt% of the tablet core, more suitably 81 wt% to 88 wt% of the tablet core, optionally 82 wt% to 88 wt% of the tablet core. Further optionally, colesevelam and/or pharmaceutically acceptable salts thereof is present in the unit dose pharmaceutical composition for oral administration in an amount of 70 wt% to 85 wt% of the tablet core, or in an amount of 75 wt% to 85 wt% of the tablet core, or in an amount of 76 wt% to 85 wt% of the tablet core, suitably in an amount of 80 wt% to 85 wt% of the tablet core, more suitably 81 wt% to 85 wt% of the tablet core, optionally 82 wt% to 85 wt% of the tablet core.

In another example, colesevelam hydrochloride is present in the unit dose pharmaceutical composition for oral administration in an amount of 70 wt% to 90 wt% of the tablet core, or in an amount of 75 wt% to 90 wt% of the tablet core, or in an amount of 76 wt% to 90 wt% of the tablet core, suitably in an amount of 80 wt% to 90 wt% of the tablet core, more suitably 81 wt% to 90 wt% of the tablet core, optionally 82 wt% to 90 wt% of the tablet core. Optionally, colesevelam hydrochloride is present in the unit dose pharmaceutical composition for oral administration in an amount of 70 wt% to 88 wt% of the tablet core, or in an amount of 75 wt% to 88 wt% of the tablet core, or in an amount of 76 wt% to 88 wt% of the tablet core, suitably in an amount of 80 wt% to 88 wt% of the tablet core, more suitably 81 wt% to 88 wt% of the tablet core, optionally 82 wt% to 88 wt% of the tablet core. Further optionally, colesevelam hydrochloride is present in the unit dose pharmaceutical composition for oral administration in an amount of 70 wt% to 85 wt% of the tablet core, or in an amount of 75 wt% to 85 wt% of the tablet core, or in an amount of 76 wt% to 85 wt% of the tablet core, suitably in an amount of 80 wt% to 85 wt% of the tablet core, more suitably 81 wt% to 85 wt% of the tablet core, optionally 82 wt% to 85 wt% of the tablet core.

Preferably, colesevelam and/or pharmaceutically acceptable salts thereof is present in the unit dose pharmaceutical composition for oral administration in an amount of from 75 wt% to 90 wt% of the tablet core, preferably in an amount of from 80 wt% to 90 wt% of the tablet core, more preferably in an amount of from 81 wt% to 90 wt% of the tablet core. For example, colesevelam and/or pharmaceutically acceptable salts thereof may be present in the unit dose pharmaceutical composition for oral administration in an amount of from 75 wt% to 90 wt% of the tablet core, optionally, in an amount of from 80 wt% to 88 wt% of the tablet core, optionally in an amount of from 81 wt% to 85 wt% of the tablet core, optionally 82 wt% to 85 wt% of the tablet core. For example, colesevelam hydrochloride is present in the unit dose pharmaceutical composition for oral administration in an amount of from 75 wt% to 90 wt% of the tablet core, preferably in an amount of from 80 wt% to 90 wt% of the tablet core, more preferably in an amount of from 81 wt% to 90 wt% of the tablet core. By way of further example, colesevelam hydrochloride may be present in the unit dose pharmaceutical composition for oral administration in an amount of from 75 wt% to 90 wt% of the tablet core, optionally, in an amount of from 80 wt% to 88 wt% of the tablet core, optionally in an amount of from 81 wt% to 85 wt% of the tablet core, optionally 82 wt% to 85 wt% of the tablet core.

Suitably, the colesevelam and/or pharmaceutically acceptable salts thereof is present in an amount of from 400 mg to 1250 mg in the tablet core, preferably in an amount of from 400 mg to 1200 mg in the tablet core, such as from 700 to 1200 mg in the tablet core, more preferably in an amount of from 850 mg to 1100 mg in the tablet core. Suitably, the colesevelam and/or pharmaceutically acceptable salts thereof is present in an amount of about 900 mg in the tablet core. For example, the colesevelam hydrochloride is present in an amount of from 400 mg to 1250 mg in the tablet core, preferably in an amount of from 400 mg to 1200 mg in the tablet core, such as from 700 to 1200 mg in the tablet core, more preferably in an amount of from 850 mg to 1100 mg in the tablet core. Suitably, the colesevelam hydrochloride is present in an amount of about 900 mg in the tablet core.

The unit dose may be formulated for delayed release, optionally for delayed release in the ileum. The unit dose may be formulated for extended release, optionally for extended release starting in the ileum and continuing into the initial portion of the colon. The unit dose may be formulated for delayed/extended release, optionally for delayed release in the ileum and extended release continuing into the initial portion of the colon.

The unit dose may be formulated for release starting and reaching 100% release in the ileum prior to entering the ascending colon. The unit dose may be formulated for release starting in the ileum and continuing into the initial portion of the ascending colon. The unit dose may be formulated for release starting in the ileum and continuing throughout the ascending colon. The unit dose may be formulated for release starting in the ileum and continuing throughout the ascending and transverse colon. The unit dose may be formulated for release starting in the ileum and continuing throughout the ascending colon, transverse colon and descending colon. The unit dose may be formulated for release starting in the ileum and continuing throughout the ascending colon, transverse colon, descending colon and sigmoid colon. The unit dose may be formulated for release starting in the ileum and continuing throughout the ascending colon, transverse colon, descending colon, sigmoid colon and rectum.

The non-granulate component of the unit dose pharmaceutical composition may also comprise a diluent.

The diluent (of the granulate component and/or non-granulate component) may be selected from one or more of polyols such as mannitol, lactose, sugars as sucrose, dextrose, dextrates, sorbitol, fructose; inorganic salts such as dibasic and tribasic calcium phosphate sodium chloride, starch, modified starch and derivatives thereof, cellulose, and a cellulose derivative (i.e. cellulose derivatives such as, for example, methyl, ethyl or hydroxyethyl cellulose or similar), and kaolin. Suitably, wherein the diluent is selected from one or more of mannitol, sucrose, cellulose, a cellulose derivative. The diluent may be present in an amount of from 5 wt% to 20 wt% of the tablet core, such as from 6 wt% to 18 wt% of the tablet core, optionally from 7 wt% to 16 wt% of the tablet core, optionally from 10 wt% to 15 wt% of the tablet core.

The binder may be selected from one or more of polyvinyl pyrrolidone, polyvinyl alcohol and related graft copolymers, such as polyvinyl alcohol graft polyethylene glycol copolymer, copovidone, acacia gum, cellulose, and a cellulose derivative (e.g., hydroxypropyl cellulose, hypromellose, or ethyl cellulose), a polymethacrylate and starch, preferably polyvinyl pyrrolidone and/or polyvinyl alcohol.

The binder may be present in an amount of from 1 wt% to 5 wt% of the tablet core, optionally from 1 wt% to 4 wt%, such as from 2 wt % to 4 wt% of the tablet core.

The composition may optionally include a disintegrant. The disintegrant may be selected from one or more of sodium carboxy methyl cellulose, such as cross-linked sodium carboxy methyl cellulose, crospovidone, bentonite, an algin (e.g., alginic acid or a salt thereof, such as sodium alginate), a gum, starch, and modified starch, preferably, wherein the disintegrant is cross-linked sodium carboxy methyl cellulose or modified starch.

The unit dose pharmaceutical composition may comprise a disintegrant.

The disintegrant may be present in an amount of from 0.1 wt% to 7 wt%, optionally, from 0.5 to 5 wt% of the tablet core, optionally from 1 wt % to 2.5 wt% of the tablet core, optionally from 1 to 2 wt% of the tablet core.

Suitably, the disintegrant is present in the non-granulate component of the unit dose pharmaceutical composition.

The unit dose pharmaceutical composition may further comprise a glidant and/or lubricant.

The glidant may be selected from the group comprising: talc, starch, magnesium oxide, silica (silicon dioxide) and a silicate, such as magnesium or calcium silicate. Preferably, the glidant is colloidal silicon dioxide, silica, talc or magnesium oxide.

The lubricant may be selected from the group comprising: stearic acid and salts thereof (e.g. magnesium, calcium or zinc stearate), polyethylene glycol, fumaric acid and salts thereof, glyceryl behenate, glyceryl palmitostearate, wax, poloxamer, sodium benzoate, and any combination thereof. Preferably, the lubricant is stearic acid or a salt thereof (e.g., magnesium stearate), fumaric acid or a salt thereof, or polyethylene glycol.

The glidant may be present in an amount of from 0.1 to 2 wt% of the tablet core, optionally the glidant is present in an amount of from 0.5 to 1 wt% of the tablet core.

The lubricant may be present in an amount of from 0.1 to 2 wt% of the tablet core, optionally the lubricant is present in an amount of from 0.5 to 1 wt% of the tablet core.

The unit dose pharmaceutical composition may further comprise a sub-coating between the tablet core and the gastro-resistant coating, wherein optionally, the sub-coating comprises hydroxypropyl cellulose.

The gastro-resistant coating (film) covering the tablet core may contain polymethacrylates, acrylic and/or methacrylic acids polymers or copolymers or cellulose derivatives, such as cellulose acetophthalate. Optionally, the gastro-protective coating comprises methacrylic acid/methyl methacrylate (1:1) copolymer (e.g., commercially known as EUDRAGIT L). Optionally, the gastro-resistant coating comprises methacrylic acid/methyl methacrylate (1:2) copolymer (e.g., commercially known as EUDRAGIT S). Optionally, the gastro-protective coating comprises methacrylic acid-ethylacrylate (1:1) copolymer (e.g., commercially known as EUDRAGIT L30 D55). Optionally, the gastro-protective coating comprises polymethacrylate copolymers including, but not limited, to compounds known commercially as EUDRAGIT S (methacrylic acid-methyl methacrylate copolymer 1:2), EUDRAGIT L (methacrylic acid- methyl methacrylate 1:1 copolymer) and/or EUDRAGIT L30 D55 (methacrylic acid-ethylacrylate 1:1 copolymer), and/or mixtures thereof. The gastro resistant coating may comprise a methacrylic acid -(meth)acrylate copolymer. Suitably, the gastro resistant coating comprises methacrylic acid/methyl methacrylate copolymer.

The gastro-resistant coating may comprise methacrylic acid/methyl methacrylate (1:1) copolymer (EUDRAGIT L), or methacrylic acid/methyl methacrylate (1:2) copolymer (EUDRAGIT S).

The gastro-resistant coating may comprise a mixture of methacrylic acid/methyl methacrylate (1:1) copolymer and of methacrylic acid/methyl methacrylate (1:2) copolymer. Optionally, the mixture comprises a weight ratio of 0.5:1 to 3:1 of methacrylic acid/methyl methacrylate (1:1) copolymer to methacrylic acid/methyl methacrylate (1:2). Optionally, the mixture comprises a weight ratio of 1:1 to 3:1 of methacrylic acid/methyl methacrylate (1:1) copolymer to methacrylic acid/methyl methacrylate (1:2). Preferably, the mixture comprises a weight ratio of about 2:1 of methacrylic acid/methyl methacrylate (1:1) copolymer to methacrylic acid/methyl methacrylate (1:2).

The gastro-resistant coating of the unit dose suitably starts breaking or dissolving at or above a pH of 5, preferably at or above a pH of 6, more preferably at or above a pH of 6.5, much more preferably at a pH at or about of 6.8.

Suitably, the active substance is released from the tablet core at pH of at least 5, such as 5.1, or 5.2, or 5.3, or 5.4, or 5.5, or 5.6, or5.7, or 5.8, or 5.9, or6, or6.1, or6.2, or6.3, or 6.4, or 6.5, or 6.6, or 6.7, or 6.8, or 6.9, or 7, or 7.1, or 7.2, or 7.3, or 7.4, or 7.5. Preferably, the active substance is released from the tablet core at pH in the range from about 6.5 to about 7.5, optionally, at a pH in the range of from about 6.8 to about 7. According to a preferred aspect, the active substance is released from the tablet core at pH of 6.8. According to another preferred aspect, the active substance is released from the tablet core at pH of 7. According to a further preferred aspect, the active substance is released from the tablet core at pH of 7.2.

The tablet core has a friability of less than about 1.0%, optionally, less than about 0.5%, preferably less than about 0.1% when measured in accordance with US Pharmacopeia monograph <1216> or with European Pharmacopoeia monograph 2.9.7. For example, the tablet core may have a friability of 0.001 to 1%, or from 0.01 to 0.5%, such as from 0.01 to 0.1% when measured in accordance with US Pharmacopeia monograph <1216> or with European Pharmacopoeia monograph 2.9.7. In particular, the tablet core has a friability of less than about 1.0%, optionally, less than about 0.5%, preferably less than about 0.1% when measured in accordance with US Pharmacopeia monograph <1216> or with European Pharmacopoeia monograph 2.9.7 when the colesevelam is colsevelam hydrochloride. For example, the tablet core may have a friability of 0.001 to 1%, or from 0.01 to 0.5%, such as from 0.01 to 0.1% when measured in accordance with US Pharmacopeia monograph <1216> or with European Pharmacopoeia monograph 2.9.7 when the colesevelam is colsevelam hydrochloride.

The tablet core has a hardness of greater than about 70 N, optionally, greater than about 80 N, optionally, greater than about 90 N, when measured in accordance with US Pharmacopeia monograph <1217> or with European Pharmacopoeia monograph 2.9.8. In particular, the tablet core has a hardness of greater than about 70 N, optionally, greater than about 80 N, optionally, greater than about 90 N, when measured in accordance with US Pharmacopeia monograph <1217> or with European Pharmacopoeia monograph 2.9.8 when the colesevelam is colsevelam hydrochloride.

The tablet core may have a hardness in the range of from 70 N to 450 N, such as from 80 N to 400 N, optionally from 90 N to 370 N, such as from 100 N to 330 N when measured in accordance with US Pharmacopeia monograph <1217> or with European Pharmacopoeia monograph 2.9.8. In particular, the tablet core may have a hardness in the range of from 70 N to 450 N, such as from 80 N to 400 N, optionally from 90 N to 370 N, such as from 100 N to 330 N when measured in accordance with US Pharmacopeia monograph <1217> or with European Pharmacopoeia monograph 2.9.8 when the colesevelam is colsevelam hydrochloride.

For example, the tablet core may have a hardness in the range of from 70 N to 450 N, optionally, from 80 N to 450 N, optionally, from 90 N to 450 N, optionally from 100 N to 450 N when measured in accordance with US Pharmacopeia monograph <1217> or with European Pharmacopoeia monograph 2.9.8. Optionally, the tablet core may have a hardness in the range of from 70 N to 400 N, optionally, from 80 N to 400 N, optionally, from 90 N to 400 N, optionally from 100 N to 400 N when measured in accordance with US Pharmacopeia monograph <1217> or with European Pharmacopoeia monograph 2.9.8. Further optionally, the tablet core may have a hardness in the range of from 70 N to 370 N, optionally, from 80 N to 370 N, optionally, from 90 N to 370 N, optionally from 100 N to 370 N when measured in accordance with US Pharmacopeia monograph <1217> or with European Pharmacopoeia monograph 2.9.8. Further optionally, the tablet core may have a hardness in the range of from 70 N to 330 N, optionally, from 80 N to 330 N, optionally, from 90 N to 330 N, optionally from 100 N to 330 N when measured in accordance with US Pharmacopeia monograph <1217> or with European Pharmacopoeia monograph 2.9.8.

By way of further example, the tablet core may have a hardness in the range of from 70 N to 450 N, optionally, from 80 N to 450 N, optionally, from 90 N to 450 N, optionally from 100 N to 450 N when measured in accordance with US Pharmacopeia monograph <1217> or with European Pharmacopoeia monograph 2.9.8 when the colesevelam is colsevelam hydrochloride. Optionally, the tablet core may have a hardness in the range of from 70 N to 400 N, optionally, from 80 N to 400 N, optionally, from 90 N to 400 N, optionally from 100 N to 400 N when measured in accordance with US Pharmacopeia monograph <1217> or with European Pharmacopoeia monograph 2.9.8 when the colesevelam is colsevelam hydrochloride. Further optionally, the tablet core may have a hardness in the range of from 70 N to 370 N, optionally, from 80 N to 370 N, optionally, from 90 N to 370 N, optionally from 100 N to 370 N when measured in accordance with US Pharmacopeia monograph <1217> or with European Pharmacopoeia monograph 2.9.8 when the colesevelam is colsevelam hydrochloride. Further optionally, the tablet core may have a hardness in the range of from 70 N to 330 N, optionally, from 80 N to 330 N, optionally, from 90 N to 330 N, optionally from 100 N to 330 N when measured in accordance with US Pharmacopeia monograph <1217> or with European Pharmacopoeia monograph 2.9.8 when the colesevelam is colsevelam hydrochloride.

Suitably, in the unit dose pharmaceutical composition, the colesevelam and/or the pharmaceutically acceptable salts thereof is present in the granulate component in an amount of from 30 to 50 wt% of the tablet core, more preferably 35 to 45 wt% of the tablet core, and suitably, the colesevelam and/or the pharmaceutically acceptable salts thereof is present in the non-granulate component in an amount of from 30 to 50 wt% of the tablet core, more preferably in an amount of from 35 to 45 wt% of the tablet core. For example, the colesevelam hydrochloride is present in the granulate component in an amount of from 30 to 50 wt% of the tablet core, more preferably 35 to 45 wt% of the tablet core, and suitably, the colesevelam hydrochloride is present in the non-granulate component in an amount of from 30 to 50 wt% of the tablet core, more preferably in an amount of from 35 to 45 wt% of the tablet core.

Suitably, in the unit dose pharmaceutical composition, the colesevelam and/or the pharmaceutically acceptable salts thereof is present in the granulate component in an amount of from 30 to 50 wt% of the tablet core, more preferably 35 to 45 wt% of the tablet core, and the colesevelam and/or the pharmaceutically acceptable salts thereof is present in the non-granulate component in an amount of from 30 to 50 wt% of the tablet core, more preferably 35 to 45 wt% of the tablet core, and suitably the total amount of colesevelam and/or pharmaceutically acceptable salts present in the unit dose pharmaceutical composition is present in an amount of at least 76 wt% of the tablet core, and suitably, the total amount of colesevelam and/or pharmaceutically acceptable salts thereof is present in an amount of from 700 mg to 1200 mg in the tablet core, preferably from 850 mg to 1100 mg in the tablet core, such as about 900 mg in the tablet core.

Suitably in the unit dose pharmaceutical composition, the colesevelam hydrochloride is present in the granulate component in an amount of from 30 to 50 wt% of the tablet core, more preferably 35 to 45 wt% of the tablet core, and the colesevelam hydrochloride is present in the non-granulate component in an amount of from 30 to 50 wt% of the tablet core, more preferably 35 to 45 wt% of the tablet core, and suitably the total amount of colesevelam hydrochloride present in the unit dose pharmaceutical composition is present in an amount of at least 76 wt% of the tablet core, and suitably, the total amount of colesevelam hydrochloride is present in an amount of from 700 mg to 1200 mg in the tablet core, preferably from 850 mg to 1100 mg in the tablet core, such as about 900 mg in the tablet core.

Suitably, the diluent is selected from one or more of mannitol, sucrose and cellulose, and is present in an amount of from 6 wt% to 18 wt% of the tablet core, and the binder is polyvinyl pyrrolidone and is present in an amount of from 2 wt% to 4 wt% of the tablet core.

Suitably, the colesevelam and/or pharmaceutically acceptable salt thereof is colesevelam hydrochloride.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 70 wt% of the tablet core;
wherein colesevelam and/or pharmaceutically acceptable salts thereof is present in the granulate component in an amount of from 30 to 60 wt% of the tablet core; and
colesevelam and/or pharmaceutically acceptable salts thereof is present in the non-granulate component in an amount of from 30 to 60 wt% of the tablet core;
wherein the (total) colesevelam and/or pharmaceutically acceptable salts thereof (in the unit dose) is present in an amount of from 400 mg to 1250 mg in the tablet core.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrocholoride; and wherein the colesevelam hydrochloride is present in an amount of at least 70 wt% of the tablet core;
wherein colesevelam hydrochloride is present in the granulate component in an amount of from 30 to 60 wt% of the tablet core; and
colesevelam hydrochloride is present in the non-granulate component in an amount of from 30 to 60 wt% of the tablet core;
wherein the (total) colesevelam hydrochloride (in the unit dose) is present in an amount of from 400 mg to 1250 mg in the tablet core.

Suitably, the tablet core has a hardness of greater than about 70 N, optionally greater than about 80 N, optionally, greater than about 90 N, when measured in accordance with US Pharmacopeia monograph <1217> or with European Pharmacopoeia monograph 2.9.8. More suitably, the tablet core has a hardness in the range of from 70 N to 450 N, such as from 80 N to 400 N, optionally from 90 N to 370 N, such as from 100 N to 330 N when measured in accordance with US Pharmacopeia monograph <1217> or with European Pharmacopoeia monograph 2.9.8. Suitably, the tablet core has a friability of less than about 1%, optionally, less than about 0.5%, preferably less than about 0.1% when measured in accordance with US Pharmacopeia monograph <1216> or with European Pharmacopoeia monograph 2.9.7. For example, the tablet core may have a friability of 0.001 to 1%, or from 0.01 to 0.5%, such as from 0.01 to 0.1% when measured in accordance with US Pharmacopeia monograph <1216> or with European Pharmacopoeia monograph 2.9.7. Suitably, the tablet core has the hardness and/or friability properties noted above when the colesevelam is colesevelam hydrochloride

For example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 70 wt% of the tablet core;
wherein colesevelam and/or pharmaceutically acceptable salts thereof is present in the granulate component in an amount of from 30 to 60 wt% of the tablet core; and
colesevelam and/or pharmaceutically acceptable salts thereof is present in the non-granulate component in an amount of from 30 to 60 wt% of the tablet core;
wherein the (total) colesevelam and/or pharmaceutically acceptable salts thereof (in the unit dose) is present in an amount of from 400 mg to 1250 mg in the tablet core;
wherein the tablet core has a hardness of greater than about 70 N, optionally > greater than 80 N, optionally, > greater than 90 N, when measured in accordance with US Pharmacopeia monograph <1217> or with European Pharmacopoeia monograph 2.9.8; and
wherein the tablet core has a friability of less than about 1%, optionally, less than 0.5%, preferably less than 0.1% when measured in accordance with US Pharmacopeia monograph <1216> or with European Pharmacopoeia monograph 2.9.7.

By way of further example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 70 wt% of the tablet core;
wherein colesevelam hydrochloride is present in the granulate component in an amount of from 30 to 60 wt% of the tablet core; and
colesevelam hydrochloride is present in the non-granulate component in an amount of from 30 to 60 wt% of the tablet core;
wherein the (total) colesevelam hydrochloride (in the unit dose) is present in an amount of from 400 mg to 1250 mg in the tablet core;
wherein the tablet core has a hardness of greater than about 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than about 1%, optionally, less than 0.5%, preferably less than 0.1% when measured in accordance with US Pharmacopeia monograph <1216> or with European Pharmacopoeia monograph 2.9.7.

For example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 70 wt% of the tablet core, such as from 70 wt% to 90 wt% of the tablet core;
wherein colesevelam and/or pharmaceutically acceptable salts thereof is present in the granulate component in an amount of from 30 to 60 wt% of the tablet core; and
colesevelam and/or pharmaceutically acceptable salts thereof is present in the non-granulate component in an amount of from 30 to 60 wt% of the tablet core;
wherein the (total) colesevelam and/or pharmaceutically acceptable salts thereof (in the unit dose) is present in an amount of from 400 mg to 1250 mg in the tablet core;
wherein the tablet core has a hardness of greater than about 70 N, such as from 70 N to 450 N when measured in accordance with US Pharmacopeia monograph <1217> or with European Pharmacopoeia monograph 2.9.8; and
wherein the tablet core has a friability of less than about 1%, such as from 0.001% to 1% when measured in accordance with US Pharmacopeia monograph <1216> or with European Pharmacopoeia monograph 2.9.7.

By way of further example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride thereof, a diluent and a binder;
wherein the non-granulate component comprises colesevelam hydrochloride; and wherein the colesevelam hydrochloride is present in an amount of at least 70 wt% of the tablet core, such as from 70 wt% to 90 wt% of the tablet core;
wherein colesevelam hydrochloride is present in the granulate component in an amount of from 30 to 60 wt% of the tablet core; and
colesevelam hydrochloride is present in the non-granulate component in an amount of from 30 to 60 wt% of the tablet core;
wherein the (total) colesevelam hydrochloride (in the unit dose) is present in an amount of from 400 mg to 1250 mg in the tablet core;
wherein the tablet core has a hardness of greater than about 70 N, such as from 70 N to 450 N when measured in accordance with US Pharmacopeia monograph <1217> or with European Pharmacopoeia monograph 2.9.8; and
wherein the tablet core has a friability of less than about 1%, such as from 0.001% to 1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 76 wt%, suitably at least 80 wt% more suitably at least 81 wt% of the tablet core, such as at least 82 wt% of the tablet core;
wherein colesevelam and/or pharmaceutically acceptable salts thereof is present in the granulate component in an amount of from 30 to 50 wt% of the tablet core; and
colesevelam and/or pharmaceutically acceptable salts thereof is present in the non-granulate component in an amount of from 30 to 50 wt% of the tablet core;
wherein the (total) colesevelam and/or pharmaceutically acceptable salts thereof (in the unit dose) is present in an amount of from 700 mg to 1200 mg in the tablet core.

Suitably, the tablet core has a hardness of greater than 70 N, optionally greater than 80 N, optionally, greater than 90 N, when measured in accordance with US Pharmacopeia monograph <1217> or with European Pharmacopoeia monograph 2.9.8. Suitably, the tablet core has a friability of less than 1.0%, optionally, less than 0.5%, preferably less than 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 76 wt%, suitably at least 80 wt% more suitably at least 81 wt% of the tablet core, such as at least 82 wt% of the tablet core;
wherein colesevelam hydrochloride is present in the granulate component in an amount of from 30 to 50 wt% of the tablet core; and
colesevelam hydrochloride is present in the non-granulate component in an amount of from 30 to 50 wt% of the tablet core;
wherein the (total) colesevelam hydrochloride (in the unit dose) is present in an amount of from 700 mg to 1200 mg in the tablet core.

Suitably, the tablet core has a hardness of greater than 70 N, optionally greater than 80 N, optionally, greater than 90 N, when measured in accordance with US Pharmacopeia monograph <1217> or with European Pharmacopoeia monograph 2.9.8. Suitably, the tablet core has a friability of less than 1.0%, optionally, less than 0.5%, preferably less than 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount from 76 wt% to 90 wt%, suitably from 80 wt% to 90 wt%, more suitably from 81 wt% to 90 wt% of the tablet core, optionally, 82 wt% to 90 wt% of the tablet core;
wherein colesevelam and/or pharmaceutically acceptable salts thereof is present in the granulate component in an amount of from 30 to 50 wt% of the tablet core; and
colesevelam and/or pharmaceutically acceptable salts thereof is present in the non-granulate component in an amount of from 30 to 50 wt% of the tablet core;
wherein the (total) colesevelam and/or pharmaceutically acceptable salts thereof (in the unit dose) is present in an amount of from 700 mg to 1200 mg in the tablet core.

Suitably, the tablet core has a hardness of 70 N to 400 N, optionally 80 N to 400 N, optionally, 90 N to 400 N, such as 100 N to 400 N when measured in accordance with US Pharmacopeia monograph <1217> or with European Pharmacopoeia monograph 2.9.8. Suitably, the tablet core has a friability of 0.001% to 1%, optionally, 0.01 to 0.5%, preferably 0.1% to 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride salt thereof, a diluent and a binder;
wherein the non-granulate component comprises colesevelam hydrochloride; and wherein the colesevelam hydrochloride is present in an amount from 76 wt% to 90 wt%, suitably from 80 wt% to 90 wt%, more suitably from 81 wt% to 90 wt% of the tablet core, optionally, 82 wt% to 90 wt% of the tablet core;
wherein colesevelam hydrochloride is present in the granulate component in an amount of from 30 to 50 wt% of the tablet core; and
colesevelam hydrochloride is present in the non-granulate component in an amount of from 30 to 50 wt% of the tablet core;
wherein the (total) colesevelam hydrochloride (in the unit dose) is present in an amount of from 700 mg to 1200 mg in the tablet core.

Suitably, the tablet core has a hardness of 70 N to 400 N, optionally 80 N to 400 N, optionally, 90 N to 400 N, such as 100 N to 400 N, when measured in accordance with US Pharmacopeia monograph <1217> or with European Pharmacopoeia monograph 2.9.8. Suitably, the tablet core has a friability of 0.001% to 1%, optionally, 0.01 to 0.5%, preferably 0.1% to 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount from 81 wt% to 90 wt% of the tablet core, optionally, 82 wt% to 90 wt% of the tablet core;
wherein colesevelam and/or pharmaceutically acceptable salts thereof is present in the granulate component in an amount of from 35 to 50 wt% of the tablet core; and
colesevelam and/or pharmaceutically acceptable salts thereof is present in the non-granulate component in an amount of from 35 to 50 wt% of the tablet core;
wherein the (total) colesevelam and/or pharmaceutically acceptable salts thereof (in the unit dose) is present in an amount of from 700 mg to 1200 mg in the tablet core.

Suitably, the tablet core has a hardness of 70 N to 400 N, optionally 80 N to 400 N, optionally, 90 N to 400 N, such as 100 N to 400 N when measured in accordance with US Pharmacopeia monograph <1217> or with European Pharmacopoeia monograph 2.9.8. Suitably, the tablet core has a friability of 0.001% to 1%, optionally, 0.01 to 0.5%, preferably 0.1% to 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount from 81 wt% to 90 wt% of the tablet core, optionally, 82 wt% to 90 wt% of the tablet core;
wherein colesevelam hydrochloride is present in the granulate component in an amount of from 35 to 50 wt% of the tablet core; and
colesevelam hydrochloride is present in the non-granulate component in an amount of from 35 to 50 wt% of the tablet core;
wherein the (total) colesevelam hydrochloride (in the unit dose) is present in an amount of from 700 mg to 1200 mg in the tablet core.

Suitably, the tablet core has a hardness of 70 N to 400 N, optionally 80 N to 400 N, optionally, 90 N to 400 N, such as 100 N to 400 N when measured in accordance with US Pharmacopeia monograph <1217> or with European Pharmacopoeia monograph 2.9.8. Suitably, the tablet core has a friability of 0.001 % to 1%, optionally, 0.01 to 0.5%, preferably 0.1% to 0.1% when measured in accordance with US Pharmacopeia monograph <1216> or with European Pharmacopoeia monograph 2.9.7.

For example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 76 wt%, suitably at least 80 wt% more suitably at least 81 wt% of the tablet core, optionally at least 82 wt% of the tablet core;
wherein colesevelam and/or pharmaceutically acceptable salts thereof is present in the granulate component in an amount of from 30 to 50 wt% of the tablet core; and
colesevelam and/or pharmaceutically acceptable salts thereof is present in the non-granulate component in an amount of from 30 to 50 wt% of the tablet core;
wherein the (total) colesevelam and/or pharmaceutically acceptable salts thereof (in the unit dose) is present in an amount of from 700 mg to 1200 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably 0.1% when measured in accordance with US Pharmacopeia monograph <1216> or with European Pharmacopoeia monograph 2.9.7.

By way of further example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 76 wt%, suitably at least 80 wt% more suitably at least 81 wt% of the tablet core, optionally at least 82 wt% of the tablet core;
wherein colesevelam hydrochloride thereof is present in the granulate component in an amount of from 30 to 50 wt% of the tablet core; and
colesevelam hydrochloride is present in the non-granulate component in an amount of from 30 to 50 wt% of the tablet core;
wherein the (total) colesevelam hydrchloride (in the unit dose) is present in an amount of from 700 mg to 1200 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably 0.1% when measured in accordance with US Pharmacopeia monograph <1216> or with European Pharmacopoeia monograph 2.9.7.

For example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 81 wt% of the tablet core, optionally at least 82 wt% of the tablet core;
wherein colesevelam and/or pharmaceutically acceptable salts thereof is present in the granulate component in an amount of from 35 to 50 wt% of the tablet core; and
colesevelam and/or pharmaceutically acceptable salts thereof is present in the non-granulate component in an amount of from 35 to 50 wt% of the tablet core;
wherein the (total) colesevelam and/or pharmaceutically acceptable salts thereof (in the unit dose) is present in an amount of from 700 mg to 1200 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably 0.1% when measured in accordance with US Pharmacopeia monograph <1216> or with European Pharmacopoeia monograph 2.9.7.

By way of further example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 81 wt% of the tablet core, optionally at least 82 wt% of the tablet core;
wherein colesevelam hydrochloride is present in the granulate component in an amount of from 35 to 50 wt% of the tablet core; and
colesevelam hydrochloride is present in the non-granulate component in an amount of from 35 to 50 wt% of the tablet core;
wherein the (total) colesevelam hydrochloride (in the unit dose) is present in an amount of from 700 mg to 1200 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably 0.1% when measured in accordance with US Pharmacopeia monograph <1216> or with European Pharmacopoeia monograph 2.9.7.

For example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of 76 wt% to 90 wt% of the tablet core, suitably from 80 wt% to 90 wt% of the tablet core;
wherein colesevelam and/or pharmaceutically acceptable salts thereof is present in the granulate component in an amount of from 30 to 50 wt% of the tablet core; and
colesevelam and/or pharmaceutically acceptable salts thereof is present in the non-granulate component in an amount of from 30 to 50 wt% of the tablet core;
wherein the (total) colesevelam and/or pharmaceutically acceptable salts thereof (in the unit dose) is present in an amount of from 700 mg to 1200 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

By way of example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of 76 wt% to 90 wt% of the tablet core, suitably from 80 wt% to 90 wt% of the tablet core,;
wherein colesevelam hydrochloride is present in the granulate component in an amount of from 30 to 50 wt% of the tablet core; and
colesevelam hydrochloride is present in the non-granulate component in an amount of from 30 to 50 wt% of the tablet core;
wherein the (total) colesevelam hydrochloride (in the unit dose) is present in an amount of from 700 mg to 1200 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

For example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of 81 wt% to 90 wt% of the tablet core, suitably from 82 wt% to 90 wt% of the tablet core;
wherein colesevelam and/or pharmaceutically acceptable salts thereof is present in the granulate component in an amount of from 35 to 50 wt% of the tablet core; and
colesevelam and/or pharmaceutically acceptable salts thereof is present in the non-granulate component in an amount of from 35 to 50 wt% of the tablet core;
wherein the (total) colesevelam and/or pharmaceutically acceptable salts thereof (in the unit dose) is present in an amount of from 700 mg to 1200 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

By way of further example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam and/or the pharmaceutically acceptable salts thereof is present in an amount of 81 wt% to 90 wt% of the tablet core, suitably from 82 wt% to 90 wt% of the tablet core;
wherein colesevelam hydrochloride is present in the granulate component in an amount of from 35 to 50 wt% of the tablet core; and
colesevelam hydrochloride is present in the non-granulate component in an amount of from 35 to 50 wt% of the tablet core;
wherein the (total) colesevelam hydrochloride (in the unit dose) is present in an amount of from 700 mg to 1200 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

For example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 76 wt%, suitably at least 80 wt% of the tablet core;
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, such as in an amount of from 850 mg to 1100 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably less than 0.1% when measured in accordance with US Pharmacopeia monograph <1216> or with European Pharmacopoeia monograph 2.9.7.

By way of further example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 76 wt%, suitably at least 80 wt% of the tablet core;
wherein colesevelam hydrochloride is present in the granulate component in an amount of from 35 to 45 wt% of the tablet core; and
colesevelam hydrochloride is present in the non-granulate component in an amount of from 35 to 45 wt% of the tablet core;
wherein the (total) colesevelam hydrochloride (in the unit dose) is present in an amount of from 700 mg to 1200 mg in the tablet core, such as in an amount of from 850 mg to 1100 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably less than 0.1% when measured in accordance with US Pharmacopeia monograph <1216> or with European Pharmacopoeia monograph 2.9.7.

Also disclosed is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
Wherein the non-granulate component comprises colesevelam or a pharmaceutically acceptable salt thereof; and wherein the colesevelam and/or the pharmaceutically acceptable salts thereof is present in an amount of at least 81 wt% of the tablet core,
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the (total) colesevelam and/or pharmaceutically acceptable salts thereof (in the unit dose) is present in an amount of from 700 mg to 1200 mg in the tablet core, such as in an amount of from 850 mg to 1100 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably less than 0.1% when measured in accordance with US Pharmacopeia monograph <1216> or with European Pharmacopoeia monograph 2.9.7.

Also disclosed is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
Wherein the non-granulate component comprises colesevelam hydrochloride; and wherein the colesevelam hydrochloride is present in an amount of at least 81 wt% of the tablet core,
wherein colesevelam hydrochloride is present in the granulate component in an amount of from 36 to 45 wt% of the tablet core; and
colesevelam hydrochloride is present in the non-granulate component in an amount of from 36 to 45 wt% of the tablet core;
wherein the (total) colesevelam hydrochloride (in the unit dose) is present in an amount of from 700 mg to 1200 mg in the tablet core, such as in an amount of from 850 mg to 1100 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably less than 0.1% when measured in accordance with US Pharmacopeia monograph <1216> or with European Pharmacopoeia monograph 2.9.7.

Also disclosed is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
Wherein the non-granulate component comprises colesevelam or a pharmaceutically acceptable salt thereof; and wherein the colesevelam and/or the pharmaceutically acceptable salts thereof is present in an amount of at least 82 wt% of the tablet core,
wherein colesevelam and/or pharmaceutically acceptable salts thereof is present in the granulate component in an amount of from 37 to 45 wt% of the tablet core; and
colesevelam and/or pharmaceutically acceptable salts thereof is present in the non-granulate component in an amount of from 37 to 45 wt% of the tablet core;
wherein the (total) colesevelam and/or pharmaceutically acceptable salts thereof (in the unit dose) is present in an amount of from 700 mg to 1200 mg in the tablet core, such as in an amount of from 850 mg to 1100 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably less than 0.1% when measured in accordance with US Pharmacopeia monograph <1216> or with European Pharmacopoeia monograph 2.9.7.

Also disclosed is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 82 wt% of the tablet core,
wherein colesevelam hydrochloride is present in the granulate component in an amount of from 37 to 45 wt% of the tablet core; and
colesevelam hydrochloride is present in the non-granulate component in an amount of from 37 to 45 wt% of the tablet core;
wherein the (total) colesevelam hydrochloride (in the unit dose) is present in an amount of from 700 mg to 1200 mg in the tablet core, such as in an amount of from 850 mg to 1100 mg in the tablet core;
wherein the tablet core has a hardness of greater than 70 N, optionally greater than 80 N, optionally, greater than 90 N, when measured in accordance with US Pharmacopeia monograph <1217> or with European Pharmacopoeia monograph 2.9.8; and
wherein the tablet core has a friability of less than 1%, optionally, less than 0.5%, preferably less than 0.1% when measured in accordance with US Pharmacopeia monograph <1216> or with European Pharmacopoeia monograph 2.9.7.

For example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of from 76 wt% to 90 wt%, suitably from 80 wt% to 90 wt%;
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, such as in an amount of from 850 mg to 1100 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

By way of further example, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of from 76 wt% to 90 wt%, suitably from 80 wt% to 90 wt%;
wherein colesevelam hydrochloride is present in the granulate component in an amount of from 35 to 45 wt% of the tablet core; and
colesevelam hydrochloride is present in the non-granulate component in an amount of from 35 to 45 wt% of the tablet core;
wherein the (total) colesevelam hydrochloride (in the unit dose) is present in an amount of from 700 mg to 1200 mg in the tablet core, such as in an amount of from 850 mg to 1100 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of from 81 wt% to 90 wt% of the tablet core,
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the (total) colesevelam and/or pharmaceutically acceptable salts thereof (in the unit dose) is present in an amount of from 700 mg to 1200 mg in the tablet core, such as in an amount of from 850 mg to 1100 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of from 81 wt% to 90 wt% of the tablet core,
wherein colesevelam hydrochloride is present in the granulate component in an amount of from 36 to 45 wt% of the tablet core; and
colesevelam hydrochloride is present in the non-granulate component in an amount of from 36 to 45 wt% of the tablet core;
wherein the (total) colesevelam hydrochloride (in the unit dose) is present in an amount of from 700 mg to 1200 mg in the tablet core, such as in an amount of from 850 mg to 1100 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of from 82 wt% to 90 wt% of the tablet core,
wherein colesevelam and/or pharmaceutically acceptable salts thereof is present in the granulate component in an amount of from 37 to 45 wt% of the tablet core; and
colesevelam and/or pharmaceutically acceptable salts thereof is present in the non-granulate component in an amount of from 37 to 45 wt% of the tablet core;
wherein the (total) colesevelam and/or pharmaceutically acceptable salts thereof (in the unit dose) is present in an amount of from 700 mg to 1200 mg in the tablet core, such as in an amount of from 850 mg to 1100 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of from 82 wt% to 90 wt% of the tablet core,
wherein colesevelam hydrochloride is present in the granulate component in an amount of from 37 to 45 wt% of the tablet core; and
colesevelam hydrochloride is present in the non-granulate component in an amount of from 37 to 45 wt% of the tablet core;
wherein the (total) colesevelam hydrochloride (in the unit dose) is present in an amount of from 700 mg to 1200 mg in the tablet core, such as in an amount of from 850 mg to 1100 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 76 wt%, suitably at least 80 wt% of the tablet core;
wherein colesevelam and/or pharmaceutically acceptable salts thereof is present in the granulate component in an amount of from 30 to 50 wt% of the tablet core; and
colesevelam and/or pharmaceutically acceptable salts thereof is present in the non-granulate component in an amount of from 30 to 50 wt% of the tablet core;
wherein the (total) colesevelam and/or pharmaceutically acceptable salts thereof (in the unit dose) is present in an amount of from 700 mg to 1200 mg in the tablet core;
wherein the diluent is selected from one or more of mannitol, sucrose, cellulose, and a cellulose derivative,
wherein the binder is selected from one or more of polyvinyl pyrrolidone, polyvinyl alcohol and related graft copolymers, such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 76 wt%, suitably at least 80 wt% of the tablet core;
wherein colesevelam hydrochloride is present in the granulate component in an amount of from 30 to 50 wt% of the tablet core; and
colesevelam hydrochloride is present in the non-granulate component in an amount of from 30 to 50 wt% of the tablet core;
wherein the (total) colesevelam hydrochloride (in the unit dose) is present in an amount of from 700 mg to 1200 mg in the tablet core;
wherein the diluent is selected from one or more of mannitol, sucrose, cellulose, and a cellulose derivative,
wherein the binder is selected from one or more of polyvinyl pyrrolidone, polyvinyl alcohol and related graft copolymers, such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Disclosed herein is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 81 wt% of the tablet core, optionally at least 82 wt% of the tablet core;
wherein colesevelam and/or pharmaceutically acceptable salts thereof is present in the granulate component in an amount of from 35 to 50 wt% of the tablet core; and
colesevelam and/or pharmaceutically acceptable salts thereof is present in the non-granulate component in an amount of from 35 to 50 wt% of the tablet core;
wherein the (total) colesevelam and/or pharmaceutically acceptable salts thereof (in the unit dose) is present in an amount of from 700 mg to 1200 mg in the tablet core;
wherein the diluent is selected from one or more of mannitol, sucrose, cellulose, and a cellulose derivative,
wherein the binder is selected from one or more of polyvinyl pyrrolidone, polyvinyl alcohol and related graft copolymers, such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Disclosed herein is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 81 wt% of the tablet core, optionally at least 82 wt% of the tablet core;
wherein colesevelam hydrochloride is present in the granulate component in an amount of from 35 to 50 wt% of the tablet core; and
colesevelam hydrochloride is present in the non-granulate component in an amount of from 35 to 50 wt% of the tablet core;
wherein the (total) colesevelam hydrochloride (in the unit dose) is present in an amount of from 700 mg to 1200 mg in the tablet core;
wherein the diluent is selected from one or more of mannitol, sucrose, cellulose, and a cellulose derivative,
wherein the binder is selected from one or more of polyvinyl pyrrolidone, polyvinyl alcohol and related graft copolymers, such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
Wherein the non-granulate component comprises colesevelam or a pharmaceutically acceptable salt thereof; and wherein the colesevelam and/or the pharmaceutically acceptable salts thereof is present in an amount of 76 wt% to 90 wt%, suitably from 80 wt% to 90 wt%, of the tablet core;
wherein colesevelam and/or pharmaceutically acceptable salts thereof is present in the granulate component in an amount of from 30 to 50 wt% of the tablet core; and
colesevelam and/or pharmaceutically acceptable salts thereof is present in the non-granulate component in an amount of from 30 to 50 wt% of the tablet core;
wherein the (total) colesevelam and/or pharmaceutically acceptable salts thereof (in the unit dose) is present in an amount of from 700 mg to 1200 mg in the tablet core;
wherein the diluent is selected from one or more of mannitol, sucrose, cellulose and a cellulose derivative,
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
Wherein the non-granulate component comprises colesevelam hydrochloride; and wherein the colesevelam hydrochloride is present in an amount of 76 wt% to 90 wt%, suitably from 80 wt% to 90 wt%, of the tablet core;
wherein colesevelam hydrochloride is present in the granulate component in an amount of from 30 to 50 wt% of the tablet core; and
colesevelam hydrochloride is present in the non-granulate component in an amount of from 30 to 50 wt% of the tablet core;
wherein the (total) colesevelam hydrochloride (in the unit dose) is present in an amount of from 700 mg to 1200 mg in the tablet core;
wherein the diluent is selected from one or more of mannitol, sucrose, cellulose and a cellulose derivative,
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Provided herein is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
Wherein the non-granulate component comprises colesevelam or a pharmaceutically acceptable salt thereof; and wherein the colesevelam and/or the pharmaceutically acceptable salts thereof is present in an amount of 81 wt% to 90 wt%, suitably from 82 wt% to 90 wt%, of the tablet core;
wherein colesevelam and/or pharmaceutically acceptable salts thereof is present in the granulate component in an amount of from 35 to 50 wt% of the tablet core; and
colesevelam and/or pharmaceutically acceptable salts thereof is present in the non-granulate component in an amount of from 35 to 50 wt% of the tablet core;
wherein the (total) colesevelam and/or pharmaceutically acceptable salts thereof (in the unit dose) is present in an amount of from 700 mg to 1200 mg in the tablet core;
wherein the diluent is selected from one or more of mannitol, sucrose, cellulose and a cellulose derivative,
wherein the binder is selected from one or more of polyvinyl pyrrolidone, polyvinyl alcohol and related graft copolymers, such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Also provided herein is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
Wherein the non-granulate component comprises colesevelam hydrochloride; and wherein the colesevelam hydrochloride is present in an amount of 81 wt% to 90 wt%, suitably from 82 wt% to 90 wt%, of the tablet core;
wherein colesevelam hydrochloride is present in the granulate component in an amount of from 35 to 50 wt% of the tablet core; and
colesevelam hydrochloride is present in the non-granulate component in an amount of from 35 to 50 wt% of the tablet core;
wherein the (total) colesevelam hydrochloride (in the unit dose) is present in an amount of from 700 mg to 1200 mg in the tablet core;
wherein the diluent is selected from one or more of mannitol, sucrose, cellulose and a cellulose derivative,
wherein the binder is selected from one or more of polyvinyl pyrrolidone, polyvinyl alcohol and related graft copolymers, such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 70 wt% of the tablet core, suitably at least 75 wt% of the tablet core, or 76 wt% of the tablet core more suitably at least 80 or at least 81 wt% of the tablet core, optionally at least 82 wt% of the tablet core;
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose and a cellulose derivative,
wherein the binder is selected from one or more of polyvinyl pyrrolidone, polyvinyl alcohol and related graft copolymers, such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;

and wherein the non-granulate component comprises a lubricant and a glidant.Suitably, the tablet core has a hardness of greater than 70 N, optionally greater than 80 N, optionally, greater than 90 N, when measured in accordance with US Pharmacopeia monograph <1217> or with European Pharmacopoeia monograph 2.9.8. Suitably, the tablet core has a friability of less than 1%, optionally, less than 0.5%, preferably less than 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 70 wt% of the tablet core, suitably at least 75 wt% of the tablet core, or 76 wt% of the tablet core more suitably at least 80 or at least 81 wt% of the tablet core, optionally at least 82 wt% of the tablet core;
wherein colesevelam hydrochloride is present in the granulate component in an amount of from 35 to 45 wt% of the tablet core; and
colesevelam hydrochloride is present in the non-granulate component in an amount of from 35 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose and a cellulose derivative ,
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Suitably, the tablet core has a hardness of greater than 70 N, optionally greater than 80 N, optionally, greater than 90 N, when measured in accordance with US Pharmacopeia monograph <1217> or with European Pharmacopoeia monograph 2.9.8. Suitably, the tablet core has a friability of less than 1%, optionally, less than 0.5%, preferably less than 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of from 70 wt% to 90 wt% of the tablet core, suitably in an amount of from 75 wt% to 90 wt% of the tablet core, such as from 76 wt% to 90 wt% of the tablet core, more suitably from 80 wt% to 90 wt% of the tablet core,
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative,
wherein the binder is selected from one or more of polyvinyl pyrrolidone, polyvinyl alcohol and related graft copolymers, such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Suitably, the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8. Suitably, the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of from 70 wt% to 90 wt% of the tablet core, suitably in an amount of from 75 wt% to 90 wt% of the tablet core, such as from 76 wt% to 90 wt% of the tablet core, more suitably from 80 wt% to 90 wt% of the tablet core,
wherein colesevelam hydrochloride is present in the granulate component in an amount of from 35 to 45 wt% of the tablet core; and
colesevelam hydrochloride is present in the non-granulate component in an amount of from 35 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose and a cellulose derivative,
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol;
and wherein the non-granulate component comprises a lubricant and a glidant.

Suitably, the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8. Suitably, the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 76 wt%, suitably at least 80 wt%;
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 6 to about 18 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate acacia gum and starch, preferably polyvinyl pyrrolidone and/or polyvinyl alcohol; wherein the binder is present in an amount of from about 1 wt% to 5 wt % of the tablet core;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 76 wt%, suitably at least 80 wt%;
wherein colesevelam hydrochloride is present in the granulate component in an amount of from 35 to 45 wt% of the tablet core; and
colesevelam hydrochloride is present in the non-granulate component in an amount of from 35 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 6 to about 18 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate acacia gum and starch, preferably polyvinyl pyrrolidone and/or polyvinyl alcohol; wherein the binder is present in an amount of from about 1 wt% to 5 wt % of the tablet core;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 81 wt%;
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 5 wt % of the tablet core;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
Wherein the non-granulate component comprises colesevelam hydrochloride; and wherein the colesevelam hydrochloride is present in an amount of at least 81 wt%;
wherein colesevelam hydrochloride is present in the granulate component in an amount of from 36 to 45 wt% of the tablet core; and
colesevelam hydrochloride is present in the non-granulate component in an amount of from 36 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 5 wt % of the tablet core;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
Wherein the non-granulate component comprises colesevelam or a pharmaceutically acceptable salt thereof; and wherein the colesevelam and/or the pharmaceutically acceptable salts thereof is present in an amount of at least 82 wt% of the tablet core;
wherein colesevelam and/or pharmaceutically acceptable salts thereof is present in the granulate component in an amount of from 37 to 45 wt% of the tablet core; and
colesevelam and/or pharmaceutically acceptable salts thereof is present in the non-granulate component in an amount of from 37 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 5 wt % of the tablet core;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
Wherein the non-granulate component comprises colesevelam hydrochloride; and wherein the colesevelam hydrochloride is present in an amount of at least 82 wt% of the tablet core;
wherein colesevelam hydrochloride is present in the granulate component in an amount of from 37 to 45 wt% of the tablet core; and
colesevelam hydrochloride is present in the non-granulate component in an amount of from 37 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 5 wt % of the tablet core;
and wherein the non-granulate component comprises a lubricant and a glidant.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of from 76 wt% to 90 wt% of the tablet core, such as from 80 wt% to 90 wt% of the tablet core,;
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 4 wt % of the tablet core;
wherein the non-granulate component comprises a lubricant and a glidant;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8, and wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of from 76 wt% to 90 wt% of the tablet core, such as from 80 wt% to 90 wt% of the tablet core,;
wherein colesevelam hydrochloride is present in the granulate component in an amount of from 35 to 45 wt% of the tablet core; and
colesevelam hydrochloride is present in the non-granulate component in an amount of from 35 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 4 wt % of the tablet core;
wherein the non-granulate component comprises a lubricant and a glidant;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8, and wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of from 81 wt% to 90 wt% of the tablet core,
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 4 wt % of the tablet core;
wherein the non-granulate component comprises a lubricant and a glidant;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8, and wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of from 81 wt% to 90 wt% of the tablet core,
wherein colesevelam hydrochloride is present in the granulate component in an amount of from 36 to 45 wt% of the tablet core; and
colesevelam hydrochloride is present in the non-granulate component in an amount of from 36 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 4 wt % of the tablet core;
wherein the non-granulate component comprises a lubricant and a glidant;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8, and wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of from 82 wt% to 90 wt% of the tablet core,
wherein colesevelam and/or pharmaceutically acceptable salts thereof is present in the granulate component in an amount of from 37 to 45 wt% of the tablet core; and
colesevelam and/or pharmaceutically acceptable salts thereof is present in the non-granulate component in an amount of from 37 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 5 wt % of the tablet core;
wherein the non-granulate component comprises a lubricant and a glidant;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8, and wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of from 82 wt% to 90 wt% of the tablet core,
wherein colesevelam hydrochloride is present in the granulate component in an amount of from 37 to 45 wt% of the tablet core; and
colesevelam hydrochloride is present in the non-granulate component in an amount of from 37 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 5 wt % of the tablet core;
wherein the non-granulate component comprises a lubricant and a glidant;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8, and wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
Wherein the non-granulate component comprises colesevelam or a pharmaceutically acceptable salt thereof; and wherein the colesevelam and/or the pharmaceutically acceptable salts thereof is present in an amount of at least 76 wt%, suitably at least 80 wt% of the tablet core;
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
Wherein the non-granulate component comprises colesevelam hydrochloride; and wherein the colesevelam hydrochloride is present in an amount of at least 76 wt%, suitably at least 80 wt% of the tablet core;
wherein colesevelam hydrochloride is present in the granulate component in an amount of from 35 to 45 wt% of the tablet core; and
colesevelam hydrochloride is present in the non-granulate component in an amount of from 35 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
Wherein the non-granulate component comprises colesevelam or a pharmaceutically acceptable salt thereof; and wherein the colesevelam and/or the pharmaceutically acceptable salts thereof is present in an amount of at least 81 wt% of the tablet core;
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
Wherein the non-granulate component comprises colesevelam hydrochloride; and wherein the colesevelam hydrochloride is present in an amount of at least 81 wt% of the tablet core;
wherein colesevelam hydrochloride is present in the granulate component in an amount of from 36 to 45 wt% of the tablet core; and
colesevelam hydrochloride is present in the non-granulate component in an amount of from 36 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

The present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 82 wt% of the tablet core;
wherein colesevelam and/or pharmaceutically acceptable salts thereof is present in the granulate component in an amount of from 37 to 45 wt% of the tablet core; and
colesevelam and/or pharmaceutically acceptable salts thereof is present in the non-granulate component in an amount of from 37 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

The present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 82 wt% of the tablet core;
wherein colesevelam hydrochloride is present in the granulate component in an amount of from 37 to 45 wt% of the tablet core; and
colesevelam hydrochloride is present in the non-granulate component in an amount of from 37 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of 76 wt% to 88 wt% of the tablet core, such as from 80 wt% to 88 wt% of the tablet core;
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of 76 wt% to 88 wt% of the tablet core, such as from 80 wt% to 88 wt% of the tablet core;
wherein colesevelam hydrochloride is present in the granulate component in an amount of from 35 to 45 wt% of the tablet core; and
colesevelam hydrochloride is present in the non-granulate component in an amount of from 35 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of 81 wt% to 88 wt% of the tablet core;
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Also provided is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of 81 wt% to 88 wt% of the tablet core;
wherein colesevelam hydrochloride is present in the granulate component in an amount of from 36 to 45 wt% of the tablet core; and
colesevelam hydrochloride is present in the non-granulate component in an amount of from 36 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Further provided is provided is a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of 82 wt% to 88 wt% of the tablet core;
wherein colesevelam and/or pharmaceutically acceptable salts thereof is present in the granulate component in an amount of from 37 to 45 wt% of the tablet core; and
colesevelam and/or pharmaceutically acceptable salts thereof is present in the non-granulate component in an amount of from 37 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Further provided is provided is a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of 82 wt% to 88 wt% of the tablet core;
wherein colesevelam hydrochloride is present in the granulate component in an amount of from 37 to 45 wt% of the tablet core; and
colesevelam hydrochloride is present in the non-granulate component in an amount of from 37 to 45 wt% of the tablet core;
wherein the (total) colesevelam hydrochloride (in the unit dose) is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
and wherein the non-granulate component comprises a lubricant and a glidant, wherein the lubricant comprises magnesium stearate; and wherein the glidant comprises colloidal silicon dioxide.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 81 wt% of the tablet core;
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the total colesevelam and/or pharmaceutically acceptable salts thereof in the unit dose is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
wherein the non-granulate component comprises a lubricant and a glidant, wherein the lubricant comprises magnesium stearate; and wherein the glidant comprises colloidal silicon dioxide;
wherein the tablet core has a hardness of greater than about 70 N, optionally greater than about 80 N, optionally, greater than about 90 N, when measured in accordance with US Pharmacopeia monograph <1217> or with European Pharmacopoeia monograph 2.9.8; and wherein the tablet core has a friability of less than about 1%, optionally, less than about 0.5%, preferably less than about 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 81 wt% of the tablet core;
wherein colesevelam hydrochloride is present in the granulate component in an amount of from 36 to 45 wt% of the tablet core; and
colesevelam hydrochloride is present in the non-granulate component in an amount of from 36 to 45 wt% of the tablet core;
wherein the total colesevelam hydrochloride in the unit dose is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
wherein the non-granulate component comprises a lubricant and a glidant, wherein the lubricant comprises magnesium stearate; and wherein the glidant comprises colloidal silicon dioxide;
wherein the tablet core has a hardness of greater than about 70 N, optionally greater than about 80 N, optionally, greater than about 90 N, when measured in accordance with US Pharmacopeia monograph <1217> or with European Pharmacopoeia monograph 2.9.8; and wherein the tablet core has a friability of less than about 1%, optionally, less than about 0.5%, preferably less than about 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of at least 82 wt% of the tablet core;
wherein colesevelam and/or pharmaceutically acceptable salts thereof is present in the granulate component in an amount of from 37 to 45 wt% of the tablet core; and
colesevelam and/or pharmaceutically acceptable salts thereof is present in the non-granulate component in an amount of from 37 to 45 wt% of the tablet core;
wherein the total colesevelam and/or pharmaceutically acceptable salts thereof in the unit dose is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
wherein the non-granulate component comprises a lubricant and a glidant, wherein the lubricant comprises magnesium stearate; and wherein the glidant comprises colloidal silicon dioxide;
wherein the tablet core has a hardness of greater than about 70 N, optionally greater than about 80 N, optionally, greater than about 90 N, when measured in accordance with US Pharmacopeia monograph <1217> or with European Pharmacopoeia monograph 2.9.8; and wherein the tablet core has a friability of less than about 1%, optionally, less than about 0.5%, preferably less than about 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of at least 82 wt% of the tablet core;
wherein colesevelam hydrochloride is present in the granulate component in an amount of from 37 to 45 wt% of the tablet core; and
colesevelam hydrochloride is present in the non-granulate component in an amount of from 37 to 45 wt% of the tablet core;
wherein the total colesevelam hydrochloride in the unit dose is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
wherein the non-granulate component comprises a lubricant and a glidant, wherein the lubricant comprises magnesium stearate; and wherein the glidant comprises colloidal silicon dioxide;
wherein the tablet core has a hardness of greater than about 70 N, optionally greater than about 80 N, optionally, greater than about 90 N, when measured in accordance with US Pharmacopeia monograph <1217> or with European Pharmacopoeia monograph 2.9.8; and wherein the tablet core has a friability of less than about 1%, optionally, less than about 0.5%, preferably less than about 0.1% when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of from 81 wt% to 90 wt% of the tablet core;
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the total colesevelam and/or pharmaceutically acceptable salts thereof in the unit dose is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
wherein the non-granulate component comprises a lubricant and a glidant, wherein the lubricant comprises magnesium stearate; and wherein the glidant comprises colloidal silicon dioxide;
wherein the tablet core has a hardness of 70 N to 450 N, optionally from 80 N to 400 N, optionally, from 90 N to 370 N, such as from 100 N to 330 N, when measured in accordance with US Pharmacopeia monograph <1217> or with European Pharmacopoeia monograph 2.9.8; and wherein the tablet core has a friability from 0.001% to 1%, optionally, from 0.01% to 0.5%, such as from 0.01 to 0.1 % when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of from 81 wt% to 90 wt% of the tablet core;
wherein colesevelam hydrochloride is present in the granulate component in an amount of from 36 to 45 wt% of the tablet core; and
colesevelam hydrochloride is present in the non-granulate component in an amount of from 36 to 45 wt% of the tablet core;
wherein the total colesevelam hydrochloride in the unit dose is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
wherein the non-granulate component comprises a lubricant and a glidant, wherein the lubricant comprises magnesium stearate; and wherein the glidant comprises colloidal silicon dioxide;
wherein the tablet core has a hardness of 70 N to 450 N, optionally from 80 N to 400 N, optionally, from 90 N to 370 N, such as from 100 N to 330 N, when measured in accordance with US Pharmacopeia monograph <1217> or with European Pharmacopoeia monograph 2.9.8; and wherein the tablet core has a friability from 0.001% to 1%, optionally, from 0.01% to 0.5%, such as from 0.01 to 0.1 % when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam and/or a pharmaceutically acceptable salt thereof, a diluent and a binder;
wherein the non-granulate component comprises colesevelam or a pharmaceutically acceptable salt thereof; and wherein the colesevelam and/or the pharmaceutically acceptable salts thereof is present in an amount of from 82 wt% to 90 wt% of the tablet core;
wherein colesevelam and/or pharmaceutically acceptable salts thereof is present in the granulate component in an amount of from 37 to 45 wt% of the tablet core; and
colesevelam and/or pharmaceutically acceptable salts thereof is present in the non-granulate component in an amount of from 37 to 45 wt% of the tablet core;
wherein the total colesevelam and/or pharmaceutically acceptable salts thereof in the unit dose is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
wherein the non-granulate component comprises a lubricant and a glidant, wherein the lubricant comprises magnesium stearate; and wherein the glidant comprises colloidal silicon dioxide;
wherein the tablet core has a hardness of 70 N to 450 N, optionally from 80 N to 400 N, optionally, from 90 N to 370 N, such as from 100 N to 330 N, when measured in accordance with US Pharmacopeia monograph <1217> or with European Pharmacopoeia monograph 2.9.8; and wherein the tablet core has a friability from 0.001% to 1%, optionally, from 0.01% to 0.5%, such as from 0.01 to 0.1 % when measured in accordance with US Pharmacopeia monograph <1216> or with European Pharmacopoeia monograph 2.9.7.

Suitably, the present invention provides a unit dose pharmaceutical composition for oral administration comprising a tablet core and a gastro-resistant coating covering the core, wherein the tablet core comprises a granulate component and a non-granulate component: wherein the granulate component comprises colesevelam hydrochloride, a diluent and a binder;
wherein the non-granulate component comprises colesevelam hydrochloride; and wherein the colesevelam hydrochloride is present in an amount of from 82 wt% to 90 wt% of the tablet core;
wherein colesevelam hydrochloride is present in the granulate component in an amount of from 37 to 45 wt% of the tablet core; and
colesevelam hydrochloride is present in the non-granulate component in an amount of from 37 to 45 wt% of the tablet core;
wherein the total colesevelam hydrochloride in the unit dose is present in an amount of from 700 mg to 1200 mg in the tablet core, optionally from about 850 to about 1100 mg in the tablet core;
wherein the diluent is selected from one or more of mannitol, sucrose, cellulose, and a cellulose derivative, wherein the diluent is present in an amount of from about 7 to about 16 wt% of the tablet core;
wherein the binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl acohol; wherein the binder is present in an amount of from about 1 wt% to 5 wt % of the tablet core;
wherein the non-granulate component comprises a lubricant and a glidant, wherein the lubricant comprises magnesium stearate; and wherein the glidant comprises colloidal silicon dioxide;
wherein the tablet core has a hardness of 70 N to 450 N, optionally from 80 N to 400 N, optionally, from 90 N to 370 N, such as from 100 N to 330 N, when measured in accordance with US Pharmacopeia monograph <1217> or with European Pharmacopoeia monograph 2.9.8; and wherein the tablet core has a friability from 0.001% to 1%, optionally, from 0.01% to 0.5%, such as from 0.01 to 0.1 % when measured in accordance with US Pharmacopeia monograph <1216> or with European Pharmacopoeia monograph 2.9.7.

In another aspect the present invention provides a method of manufacturing a unit dose pharmaceutical composition for oral administration comprising coating a tablet core as described herein with a gastro resistant coating.

For example, the present invention provides a method of manufacturing a unit dose pharmaceutical composition for oral administration comprising:
coating a tablet core with a gastro resistant coating, wherein the tablet core comprises:
a granulate component and a non-granulate component, wherein the granulate component comprises colesevelam and/or a pharmaceutically acceptable salt thereof, a diluent and a binder,
wherein the non-granulate component comprises colesevelam or a pharmaceutically acceptable salt thereof, and optionally, one or more of a diluent, a disintegrant, a glidant and a lubricant, and
wherein the colesevelam and/or the pharmaceutically acceptable salts thereof is present in an amount of at least 70 wt% of the tablet core.

The oral pharmaceutical compositions disclosed herein may release not more than about 50% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

The oral pharmaceutical compositions disclosed herein may release not more than about 45% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

The oral pharmaceutical compositions disclosed herein may release not more than about 40% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

The oral pharmaceutical compositions disclosed herein may release not more than about 35% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

The oral pharmaceutical compositions disclosed herein may release not more than about 30% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

The oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

The oral pharmaceutical compositions disclosed herein, may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

The oral pharmaceutical compositions disclosed may release not more than about 50% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 45% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 40% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 25% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

The oral pharmaceutical compositions disclosed herein, may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 45% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 40% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the oral pharmaceutical compositions of the present invention have a dissolution rate lower than about 50% over about 1 hour, when measured at about pH 6.8.

Optionally, the oral pharmaceutical compositions of the present invention have a dissolution rate lower than about 45% over about 1 hour, when measured at about pH 6.8.

Optionally, the oral pharmaceutical compositions of the present invention have a dissolution rate lower than about 40% over about 1 hour, when measured at about pH 6.8.

Optionally, the oral pharmaceutical compositions of the present invention have a dissolution rate lower than about 35% over about 1 hour, when measured at about pH 6.8.

Optionally, the oral pharmaceutical compositions of the present invention have a dissolution rate lower than about 30% over about 1 hour, when measured at about pH 6.8.

Optionally, the oral pharmaceutical compositions of the present invention have a dissolution rate lower than about 25% over about 1 hour, when measured at about pH 6.8.

Optionally, the oral pharmaceutical compositions of the present invention have a dissolution rate lower than about 20% over about 1 hour, when measured at about pH 6.8.

Optionally, the oral pharmaceutical compositions of the present invention have a dissolution rate of greater than or equal to about 80% of colesevelam, or a pharmaceutically acceptable salt thereof, over about 2 hours, when measured at about pH 6.8.

Optionally, the oral pharmaceutical compositions of the present invention have a dissolution rate of greater than or equal to about 80% of colesevelam, or a pharmaceutically acceptable salt thereof, over about 3 hours, when measured at about pH 6.8.

Optionally, the oral pharmaceutical compositions of the present invention have a dissolution rate of greater than or equal to about 80% of colesevelam, or a pharmaceutically acceptable salt thereof, over about 4 hours, when measured at about pH 6.8.

According to the invention, the dissolutions are measured with the with the USP dissolution apparatus type II equipped with a paddle at 100 rpm at 37°±2° C.

The oral pharmaceutical compositions of the present invention may have a dissolution rate of less than about 50% over about 1 hour and equal to or greater than about 80% over about 2 hours, when measured at about pH 6.8 with the USP dissolution apparatus type II equipped with a paddle at 100 rpm at 37°±2° C.

The oral pharmaceutical compositions of the present invention may have a dissolution rate of less than about 45% over about 1 hour and equal to or greater than about 80% over about 2 hours, when measured at about pH 6.8 with the USP dissolution apparatus type II equipped with a paddle at 100 rpm at 37°±2° C.

The oral pharmaceutical compositions of the present invention may have a dissolution rate of less than about 40% over about 1 hour and equal to or greater than about 80% over about 2 hours, when measured at about pH 6.8 with the USP dissolution apparatus type II equipped with a paddle at 100 rpm at 37°±2° C.

The oral pharmaceutical compositions of the present invention may have a dissolution rate of less than about 35% over about 1 hour and equal to or greater than about 80% over about 2 hours, when measured at about pH 6.8 with the USP dissolution apparatus type II equipped with a paddle at 100 rpm at 37°±2° C.

The oral pharmaceutical compositions of the present invention may have a dissolution rate of less than about 30% over about 1 hour and equal to or greater than about 80% over about 2 hours, when measured at about pH 6.8 with the USP dissolution apparatus type II equipped with a paddle at 100 rpm at 37°±2° C.

The colesevelam, or a pharmaceutically acceptable salt thereof, may be released in the ileum, thus ensuring release of the colesevelam or the salt thereof, where it is needed to maximize the treatment and the efficacy.

In another aspect the present invention provides a method of manufacturing a unit dose pharmaceutical composition for oral administration comprising the steps of:
(i) providing a tablet core wherein the tablet core is a tablet core as described herein; and
(ii) coating the tablet core with a gastro resistant coating and optionally a sub coating.

For example, the present invention provides a method of manufacturing a unit dose pharmaceutical composition for oral administration comprising the steps of:
(i) providing a tablet core wherein the tablet core comprises:
   a granulate component and a non-granulate component, wherein the granulate component comprises colesevelam and/or a pharmaceutically acceptable salt thereof, a diluent and a binder,
   wherein the non-granulate component comprises colesevelam or a pharmaceutically acceptable salt thereof, and optionally, one or more of a diluent, a disintegrant, a glidant and a lubricant, and wherein the colesevelam and/or the pharmaceutically acceptable salts thereof is present in an amount of at least 66 wt% such as in an amount of at least 70 wt% of the tablet core; and
(ii) coating the tablet core with a gastro resistant coating and optionally a sub coating.

The granulate component comprises granules comprising colesevelam and/or a pharmaceutically acceptable salt thereof, diluent, and a binder. The granulate is manufactured by granulation using a granulator.

The non-granulate component may comprise one or more of a diluent, a disintegrant, a glidant and a lubricant. For example, the non-granulate component may comprise two or more, or three or more, or all of a diluent, a disintegrant, a glidant and a lubricant.

Suitably, the non-granulate component comprises a diluent. Suitably, the non-granulate component comprises a disintegrant. Suitably, the non-granulate component comprises a glidant. Suitably, the non-granulate component comprises a lubricant. Optionally, the non-granulate component comprises a diluent and a disintegrant. Optionally, the non-granulate component comprises a diluent, a disintegrant and a glidant. Optionally, the non-granulate component comprises a diluent, a disintegrant, a glidant and a lubricant.

The method may optionally comprise the step of providing a sub-coating between the non-granulate component and the gastro-resistant coating.

Suitably, the method may comprise the steps of:
(i) combining colesevelam and/or pharmaceutically acceptable salts thereof, a diluent and a binder, to form granules;
(ii) combining colesevelam and/or pharmaceutically acceptable salts thereof and, optionally one or more of a diluent, a disintegrant, a glidant and a lubricant, with the granules of step (i), to form a tablet core having a granulate component and a non-granulate component:
   wherein the granulate component comprises colesevelam and/or a pharmaceutically acceptable salt thereof, a diluent and a binder;
   wherein the non-granulate component comprises colesevelam or a pharmaceutically acceptable salt thereof, and, optionally, a diluent, a disintegrant, a glidant and a lubricant; wherein the colesevelam and/or the pharmaceutically acceptable salts thereof is present in an amount of at least 70 wt% of the tablet core; and coating the tablet core with a gastro-resistant coating and optionally with a sub-coating between the tablet core and the gastro-resistant coating.

The method may comprise dry or wet granulation, preferably wet granulation.

The wet granulation may be carried out using an aqueous or non-aqueous solvent, preferably a non-aqueous solvent, such as an organic solvent. Optionally, the organic solvent may be an alcohol, a ketone or a chlorinated solvent. Suitably, the alcohol is selected from methanol, ethanol, propanol and isopropanol, or mixtures thereof. Preferably, the alcohol is ethanol.

The method may involve bulk manufacture of a plurality of unit doses.

For example, the method may involve bulk manufacture of a plurality of unit doses comprising a pharmaceutical composition for oral administration, wherein the plurality of unit doses has a uniformity of mass of less than ±2%, preferably less than ±1%, more preferably less than ±0.8%.

The present disclosure provides for a unit dose pharmaceutical composition for oral administration as described herein for use in treating for use in a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering more than one of said unit dose oral pharmaceutical compositions to said patient.

In addition, the present disclosure provides for use of a unit dose pharmaceutical composition for oral administration as described herein for treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering more than one of said unit dose oral pharmaceutical compositions to said patient.

Further disclosed is use of a unit dose pharmaceutical composition for oral administration as described herein in the manufacture of a medicament for treating bile acid diarrhea, irritable bowel syndrome, optionally, irritable bowel syndrome subtype diarrhea (IBS-D), colitis, such as microscopic colitis, and/or bile acid malabsorption.

Also disclosed is a method for treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering more than one of said unit dose oral pharmaceutical compositions to said patient.

The pharmaceutical compositions of the present invention can be used in the treatment of bile acid diarrhea (BAD). This bile acid diarrhea may be idiopathic (e.g., primary bile acid diarrhea) or secondary to an underlying disease. The underlying disease can be: Crohn's disease, ileal resection or radiation ileitis, chronic pancreatitis, celiac disease, cholecystectomy, small intestine bacterial overgrowth, irritable bowel syndrome subtype diarrhea (IBS-D) and similar.

Suitably, the method of treating bile acid diarrhea comprises administering to a subject in need thereof, a therapeutically effective amount of a pharmaceutical composition described herein. The tablets can be administered either in a single non-fractionated administration or in multiple fractionated administrations, e.g., three tablets once per day, or one tablet three times per day. In comparison to prior art colesevelam formulations, the unit dose pharmaceutical compositions of the present invention, comprise a higher loading and a higher dose of colesevelam, thus fewertablets are required fortreatment patient compliance is improved.

Suitably, the method of treating irritable bowel syndrome subtype diarrhea (IBS-D) comprises administering to a subject in need thereof, a therapeutically effective amount of a pharmaceutical composition described herein.

The pharmaceutical compositions of the present invention can be used in the treatment of microscopic colitis.

Suitably, the method of treating microscopic colitis comprises administering to a subject in need thereof, a therapeutically effective amount of a pharmaceutical composition described herein. The pharmaceutical compositions of the present invention can be used in the treatment of bile acid malabsorption.

Suitably, the method of treating bile acid malabsorption comprises administering to a subject in need thereof, a therapeutically effective amount of a pharmaceutical composition described herein. The tablets can be administered either in a single non-fractionated administration or in multiple fractionated administrations, e.g., three tablets once per day, or one tablet three times per day, or for example, six tablets once per day, or three tablets twice per day, or two tablets thrice per day.

It will be appreciated that any of the unit dose pharmaceutical compositions for oral administration described herein may be manufactured by the methods of manufacture described herein. Furthermore, any of the unit dose pharmaceutical compositions for oral administration described herein may be employed in the methods of treatment described herein.

The invention will be more readily appreciated by a review of the examples which follow.

### EXAMPLES

### Examples

Obtaining tablets with a very high loading of colesevelam is difficult, in particular due to the physical properties of colesevelam. Colesevelam is a cross linked polymer which is insoluble in aqueous and organic solvents, and has poor flowability and compressibility properties.

WO 2021/163007 discloses a colesevelam hydrochloride formulation having the following composition:

| **Ingredient** | **Qty/mg** | **%w/w of tablet core** |
|---|---|---|
| Colesevelam hydrochloride (anhydrous) | 625 | 63.13 |
| Microcrystalline cellulose (avicel pH101) | 220 | 22.22 |
| Hydroxy propyl methylcellulose (HPMC E5LV) | 20 | 2.02 |
| Hyrdoxy propyl methylcellulose (K4M) | 100 | 10.10 |
| Colloidal silicon dioxide | 15 | 1.52 |
| Magnesium stearate | 10 | 1.01 |
| **Core tablet weight** | **990** | **100** |

| **Coating** | | |
|---|---|---|
| | **Qty/mg** | **%w/w of coated tablet** |
| Hydroxypropyl methyl cellulose (HPMC E50SLV) | 69.8 | 6.53 |
| Deiacylated monoglyceride | 9.4 | 0.88 |
| **Coated tablet total weight** | **1069.2** | **100** |

Using the same components as described in WO 2021/163007 and similar weight percentage amounts, the present inventors assessed whether a higher dose formulation (i.e., a unit dose comprising more than 625 mg colesevelam hydrochloride (anhydrous)) could be manufactured.

### Comparative Example 1: Preparation of Compositions

| | **Comparative Example 1** | |
|---|---|---|
| **Components** | **% w/w** | **Tablet (mg)** |
| Colesevelam HCl (expressed as weight of anhydrous substance) | 63.13 | 900.00 |
| Microcrystalline cellulose (Avicel pH 101) | 22.22 | 316.80 |
| Hydroxy propyl methyl cellulose E5LV | 2.02 | 28.80 |
| Hydroxy propyl methyl cellulose K4M | 10.10 | 144.00 |
| Colloidal silicon dioxide | 1.52 | 21.60 |
| Magnesium stearate | 1.01 | 14.40 |
| **Total Cores** | 100.00 | 1425.60 |

The inventors attempted to follow the same manufacturing process as is described in WO 2021/163007 in order to manufacture tablet cores comprising 900 mg colesevelam HCl (anhydrous):

### Tablet cores

1. Sift colesevelam hydrochloride, microcrystalline cellulose, HPMC ES LV(R), Hydroxypropyl methyl cellulose (K4M(R) and Aerosil R-200 through #40 mesh and mix for 10 minutes in bin blender. Sift Magnesium stearate through #60 mesh and add to the above blend and mix for another 5 min.
2. The above lubricated blend is then compressed into tablet cores.
   Coating of Tablet cores
3. Prepare coating solution by dissolving acetylated monoglyceride in purified water under stirring followed addition of HPMC E50LV(R) into it, under stirring until uniform dispersion formed.
4. The coating solution of 3) is sprayed onto the tablet cores of 2) using a Suitable coating machine to achieve a weight gain between 6-4% w/w per tablet.

However, during tableting of the composition of Comparative Example 1 (i.e., during step 2 above), the target tablet weight for tablet cores was not achieved.

The formulation of Comparative Example 1 could not be tableted at 900 mg strength in a punch size having the following dimensions: 22.5 x 10.5 mm. In fact, the maximum weight reached corresponded to 91.1% of theoretical amount of Colesevelam HCl, for each tablet.

The theoretical weight could only be achieved by increasing the tableting punch dimension. In doing so, the tablet size would be increased which would negatively impact patient compliance. Furthermore, the resulting tablet had poor friability, and crumbled during the hardness test, thereby precluding forming a high dose, high loading colesevelam tablet having suitable friability and hardness.

In order to overcome the tableting issues, and in the hope of achieving a higher drug loading of colesevelam, the inventors investigated a plethora of different excipients, combinations, drug loadings, and formulating methodologies.

### Comparative Example 2: Direct Compression of Colesevelam using alternative excipients

The use of sodium carboxymethyl starch, and poloxamer or xanthan gum was investigated in this example.

| | **Formulation A** | | **Formulation B** | |
|---|---|---|---|---|
| **Components** | **% w/w** | **Tablet (mg)** | **% w/w** | **Tablet (mg)** |
| Colesevelam HCl (expressed as anhydrous substance) | 78.00 | 900.00 | 78.00 | 900.00 |
| Silicified Microcrystalline Cellulose | 12.00 | 138.46 | 11.00 | 126.92 |
| Sodium Carboxymethyl Starch | 6.50 | 75.00 | 6.50 | 75.00 |
| Poloxamer | 3.50 | 40.38 | 0.00 | 0.00 |
| Xanthan gum | 0.00 | 0.00 | 3.50 | 40.38 |
| Magnesium Stearate | 0.00 | 0.00 | 1.00 | 11.54 |
| **Total** | 100.00 | 1153.85 | 100.00 | 1153.85 |

In comparative example 2, a similar method of manufacture was employed as in comparative example 1. The components of each composition were sifted, sieved and blended prior to tabletting to form tablet cores. In each of the compositions (of formulations A and B), the powder mixture formed after combining the components flowed very poorly, and tablet cores could only be formed with mandatory use of forced loading. Though tablet cores could be formed by direct compression, the tablet cores formed were friable with damage at the edges. Thus, the tablet cores were unsuitable for bulk manufacture.

### Test methods employed:

- Tablet breaking force is measured according to United States Pharmacopoeia (USP) <1217> and European Pharmacopoeia (EP) 2.9.8.
- Tablet friability is measured according to USP <1216> and EP 2.9.7.
- Uniformity of mass is measured according to EP 2.9.5.

### Characterization of tablet cores of Comparative Example 2

| | **Tablet cores** | |
|---|---|---|
| | **Formulation A** | **Formulation B** |
| **Targeted Dose (mg)** | 900.0 | 900.0 |
| **Dose obtained (mg)** | 900.0 | 900.0 |
| **Hardness (N)** | 49.7 | 42.2 |
| **Thickness (mm)** | 9.40 | 9.25 |
| **Friability (%)** | Tablet crumbled during the test - test failed | Tablet crumbled during the test - test failed |

### Comparative Example 3: hot melt granulation

In comparative example 3, to try to overcome the flowability issues experienced in the direct compression examples (comparative examples 1 and 2), compositions were granulated using hot melt granulation.

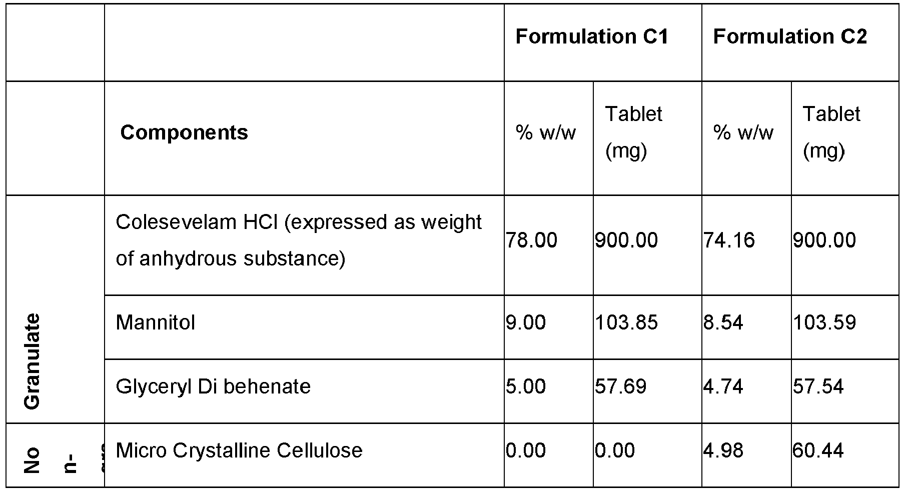

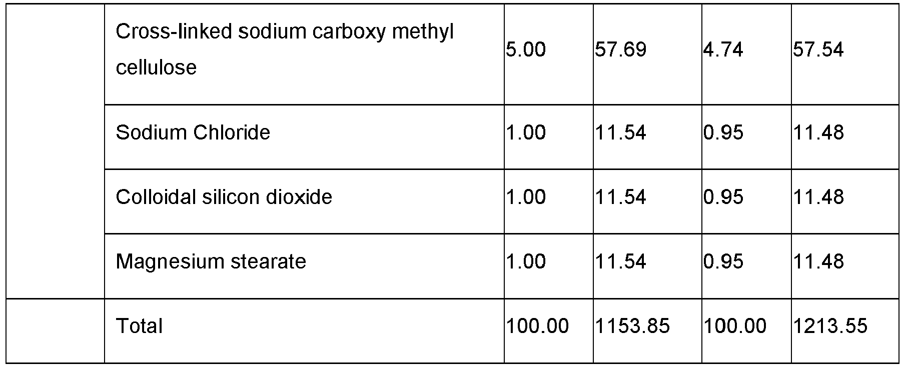

### Characterization of tablet cores of Comparative Example 3

| | **Tablet cores** | |
|---|---|---|
| | **Formulation C1** | **Formulation C2** |
| **Targeted Dose (mg)** | 900.0 | 900.0 |
| **Dose obtained (mg)** | 900.0 | 900.0 |
| **Hardness (N)** | 37 | 44.6 |
| **Thickness (mm)** | 9.03 | 9.12 |
| **Friability (%)** | Tablet crumbled during the test - test failed | Tablet crumbled during the test - test failed |

Manufacturing process: Colesevelam hydrochloride, Mannitol, and Glyceryl Di behenate were weighed, loaded in a granulator and mix while heating at 85°C until glyceryl di behenate reach its melting point. The mixture was maintained at 85°C for 2 minutes. The mixture was then spread onto a tray and the granule was kept at room temperature for at least 30 minutes. The granulate was then calibrated through a 850 µm sieve. Micro crystalline cellulose was weighed and sieved through a 710 µm sieve. The granulate and MCC was then mixed for 10 minutes in a bin blender. Cross linked sodium carboxy methyl cellulose (SCMC) and Sodium chloride (NaCl) were weighed and sieved through a 710 µm sieve. The mixture of SCMC and NaCl were combined with the mixture of granulate and MCC and the resulting mixture was blended for 5 minutes in a bin blender. Colloidal silica was weighed and sieved through an 850 µm sieve, prior to adding to a bin blender, and further blending for 5 minutes. Magnesium stearate was weighed and sieved through a 500 µm sieve, and combined with the mixture in the blender, followed by blending for an additional 3 minutes in the bin blender, thereby forming a blended mixture. The blended mixture was subsequently tabletted to form tablet cores.

Though tablet cores could be manufactured from the compositions of formulations C1 and C2, the hardness and friability of said tablet cores was not satisfactory. The tablet cores were insufficiently hard, and the tablet cores crumbled during the friability assessment.

### Comparative Example 4: Wet granulation of colesevelam

Wet granulation was also attempted. Several compositions were prepared and formulated using wet granulation in comparative example 4. Representative compositions and formulations assessed using wet granulation include formulations D1, D2, E1 and E2.

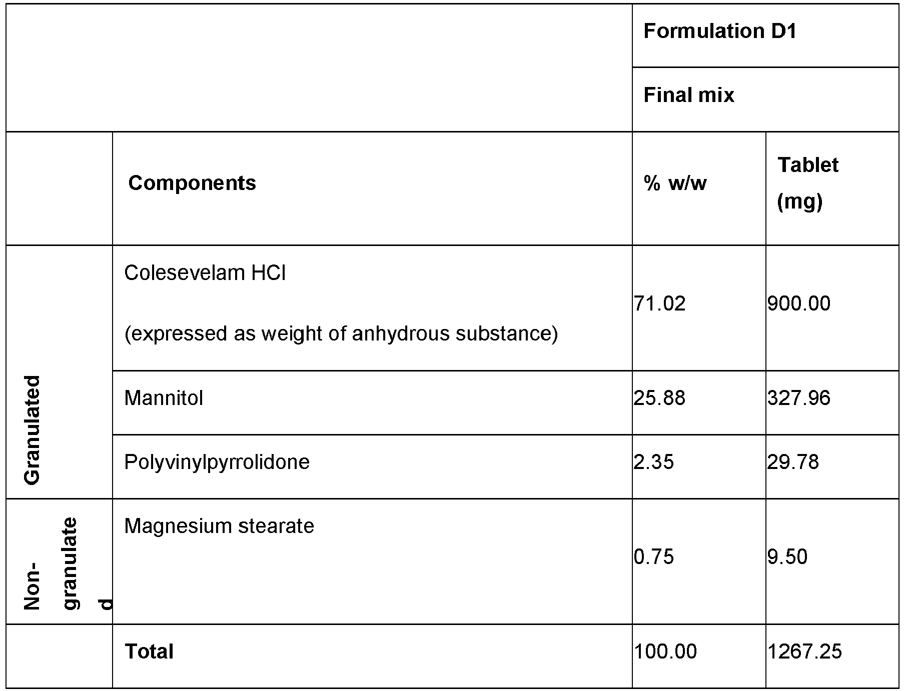

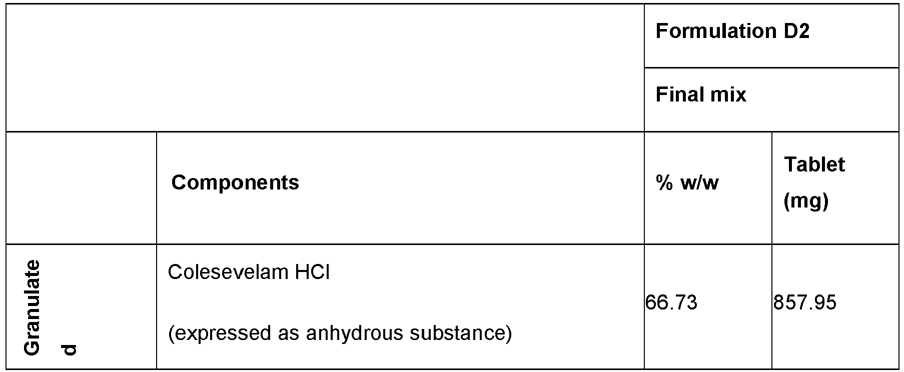

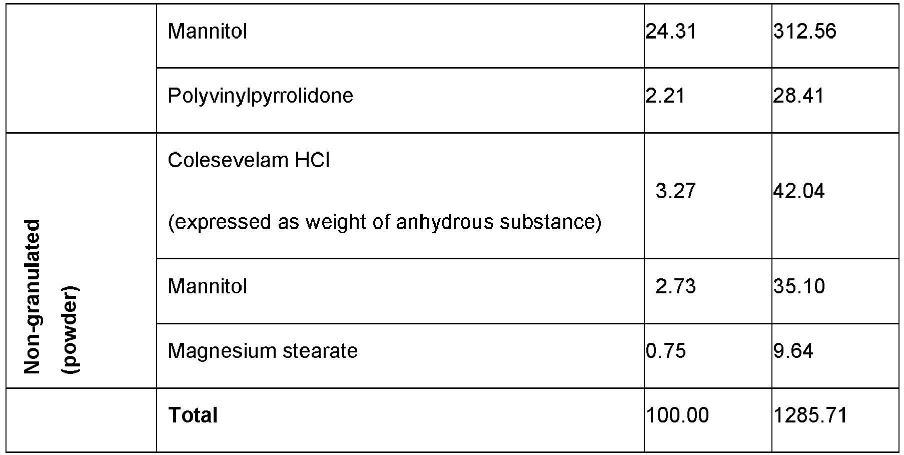

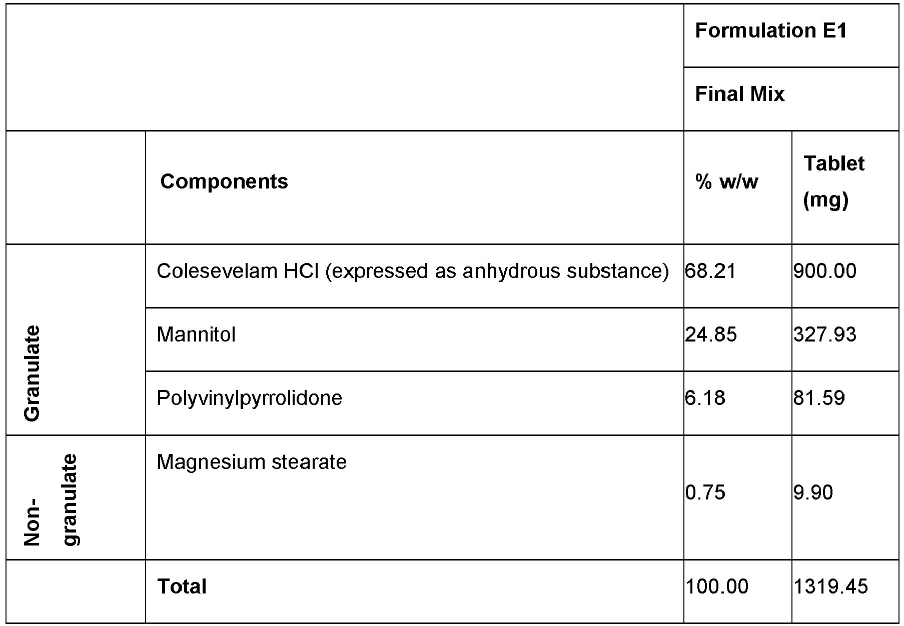

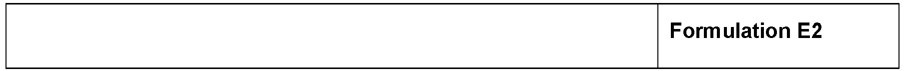

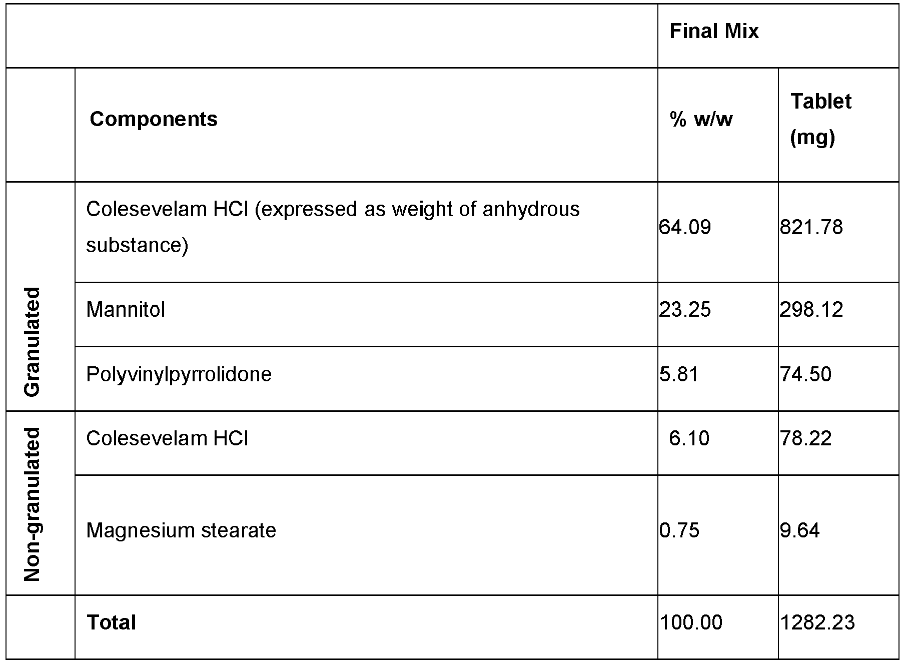

Formulation D1 involved wet granulation of colesevelam and diluent using an alcoholic solvent (ethanol, EtOH). The diluent was mannitol and PVP was also included as a binder. A solution of ethanol and PVP was added to the granulator on a bed of mannitol and colesevelam to obtain a drug loading of approximately 71%. The granulate thus formed was blended with lubricant (magnesium stearate) and tablet cores were formed with the resulting blend.

The total amount of colesevelam was divided in Formulation D2, with a first portion being granulated with diluent and binder and the remaining second portion being in powder form (i.e., non-granulated). To manufacture the granulate, wet granulation of colesevelam and diluent was conducted using an alcoholic solvent (ethanol, EtOH). The diluent was mannitol and PVP was also included as a binder. A solution of ethanol and PVP was added to the granulator on a bed of mannitol and colesevelam to obtain a granulate having a drug loading of approximately 67%. The granulate was blended with a non-granulated mixture of colesevelam (3.27 wt%), diluent (mannitol) and lubricant (magnesium stearate) and tablet cores were formed with the resulting blend.

The composition of Formulation E1, includes more binder than that of D1 and was formulated in a manner similar to that of composition D1.

The composition of Formulation E2 was similar to composition of Formulation D2, however, the split of granulated and non-granulated components was modified. In addition, diluent was included in the non-granulated portion of the composition.

Characterization of wet granulation tablet cores of Comparative example 4:

| | **Tablet cores** | | | |
|---|---|---|---|---|
| | **Formulation D1** | **Formulation D2** | **Formulation E1** | **Formulation E2** |
| **Hardness (N)** | 61 | 67.30 | ∼ 50 | ∼ 30 |
| **Thickness (mm)** | 9.27 | 8.93 | 8.94 | NA |
| **Friability (%)** | Test failed | Test failed | Test failed | Test failed |

All formulations reached the target tablet weight.

The hardness of the tablet cores of formulations D1 and D2 were satisfactory, however, the tablet cores of each of formulations, D1, D2, E1 and E2 were overly friable.

Compositions D2 and E2 were formulated by splitting the load of colesevelam into granulated and non-granulated forms. The non-granulated mixture of formulation D2 included colesevelam, diluent (in the form of mannitol) and magnesium stearate. The hardness achieved for tablet cores from formulation D2 was about 67 N, however, the tablet cores were also overly friable, and not suitable for commercial production.

### Example 1: combination of granulated and non-granulated components

Persisting with wet granulation, and formulating with granulated and non-granulated forms of colesevelam, the inventors modified the split of the colesevelam in granulate and non-granulate form. In Formulation F a total drug loading of approximately 76 wt% (of 900 mg colesevelam HCl) was split into approximately 38 wt% to be granulated and approximately 38 wt% in non-granulated (or powder) form.

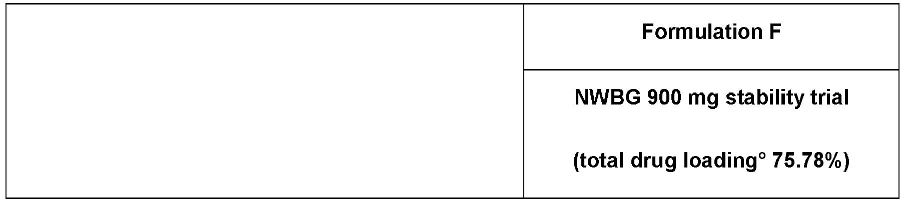

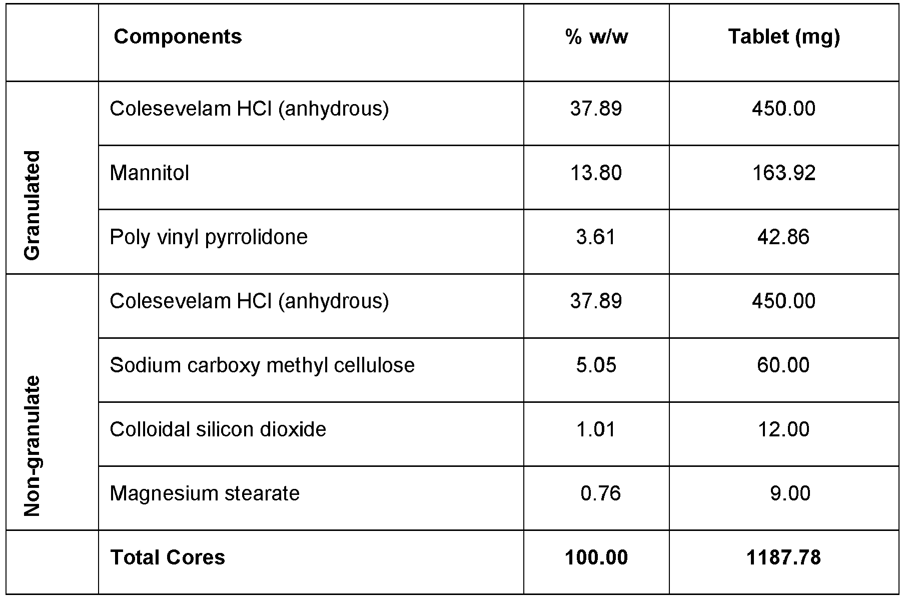

Formation of Granulate components: Colesevelam hydrochloride and mannitol were combined and sieved through an 18-mesh sieve, the sieved mixture was then blended using a high shear mixer to form a blend mixture. Polyvinyl pyrrolidone was dissolved in ethanol under stirring to form a binding solution, which was added to the blend mixture in a granulation machine, and the resulting granules were then dried in a fluid bed dryer. The dry granulate was then sifted (for example sifting through a 1 mm comill). The granulate had a particle size distribution with a D50 between 125 and 250 µm and a D90 of not more than 710 µm as determined in accordance with European Pharmacopoeia monograph 2.9.38 or US Pharmacopoeia monograph <786>.

Formation of non-granulate components: colesevelam hydrochloride, cross-linked sodium carboxy methyl cellulose and colloidal silicon dioxide were combined and sieved through a 20-mesh to form a non-granulated mixture.

Formation of tablet cores: The granulate was combined with the non-granulated mixture and the combination was added to a blender to which sieved magnesium stearate was also added. The magnesium stearate had been sieved through a 30-mesh prior to introduction into the blender. The components were then blended. The resulting blend was then tableted to form tablet cores using 22.5 × 10.5 mm punches for 900 mg strength tablets.

The tablet cores were also assessed:

| | **Formulation F** |
|---|---|
| | **NWBG 900mg** |
| | **(Total drug loading 75.78%)** |
| **Average mass (mg)** | 1194.4 |
| **Uniformity of mass** | Complies (-0.4%/+0.6%) |
| **hardness (N)** | 146 (131-157) |
| **Thickness (mm)** | 8.37 (8.32-8.43) |
| **Friability (%)** | 0.2 |

Advantageously, by reducing the amount of colesevelam in the granulate mixture and by increasing the amount of colesevelam in the non-granulated mixture tablet cores having excellent hardness properties and low friability were achieved. Furthermore, the batch production (i.e. the plurality) of tablet cores had excellent uniformity of mass. Thus, unexpectedly by splitting the colesevelam into granulate and non-granulate forms, tablet cores having a surprisingly high loading of colesevelam could be manufactured. The tablet cores had excellent hardness and low friability when the 30 wt% to 60 wt% of total colesevelam (or a pharmaceutically acceptable salt thereof) was included in the granulate form, and 30 wt% to 60 wt% of the total colesevelam (or a pharmaceutically acceptable salt thereof) was included in the non-granulate form of the tablet core.

Advantageously, the tablet cores of Example 1 comprised a high drug loading of 900 mg, which accounted for approximately 76 wt% of the total tablet core. The tablet cores of Example 1 comprised 30 to 50 wt%, such as 35 to 45 wt% of total colesevelam as a granulate component, and 30 to 50 wt%, such as 35 to 45 wt% of total colesevelam as a non-granulate component. The tablet cores had excellent hardness properties, with hardness exceeding > 90 N when measured in accordance with US Pharmacopeia monograph <1217> or with European Pharmacopoeia monograph 2.9.8, and the tablet cores had excellent friability properties, with friability of less than 0.5% when measured in accordance with US Pharmacopeia monograph <1216> or with European Pharmacopoeia monograph 2.9.7.

Buoyed by this success, the inventors ascertained if higher drug loadings could be achieved. In Example 2, a drug loading of 82% was investigated:

### Example 2A: Combination of granulated components and non-granulate components

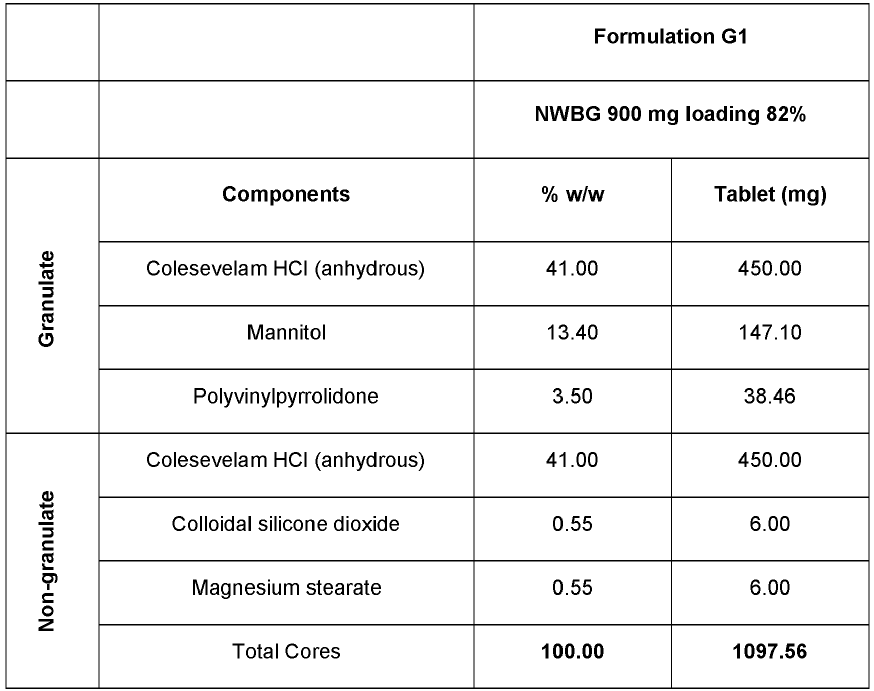

### Analysis of tablet cores of Formulation G1:

| | **Formulation G1** | |
|---|---|---|
| | **NWBG 900 mg loading 82%** | |
| | **Set 1** | **Set 2** |
| **Average mass (mg)** | 1128.7 | 1130.4 |
| **Uniformity of mass** | Complies | Complies |
| | (-0.4%/+0.5%) | (-0.3%/+0.5%) |

| | | |
|---|---|---|
| **Tablets breaking force (N)** | **119** | **149** |
| | (117-122) | (148-151) |
| **Thickness (mm)** | 7.86 | 7.91 |
| | (7.82-7.89) | (7.90-7.93) |
| **Friability (%)** | <0.1 | <0.1 |

Advantageously, the tablet cores of Example 2A comprising a total loading of 900 mg colesevelam or a pharmaceutically acceptable salt thereof (based on anhydrous weight) had excellent hardness and low friability, despite having a drug loading of 82 wt%. Furthermore, the uniformity of mass of the batches produced in Example 2A was excellent. Thus, the present inventors have identified a surprisingly effective formulation approach that facilitates the formation of unit dose pharmaceutical compositions comprising very high loadings of colesevelam. The inventors discovered that by splitting the colesevelam dose into granulate and non-granulate forms, and in particular by splitting the total colesevelam dose into from 30 to 60 wt% of the tablet core in the granulate component and the 30 to 60 wt% in the non-granulate component, tablet cores having excellent hardness and low friability could be successfully manufactured. Moreover, bulk manufacture of such tablet cores had excellent uniformity of mass. The formation of a unit dose pharmaceutical composition for oral administration comprising a tablet core and a gastro-resistant coating covering the core, comprising a high loading of colesevelam is thus achieved by forming tablet cores from a blend of a granulate and non-granulate mixture, wherein each of the granulate and non-granulate comprise colesevelam. The inventors discovered that by using a blend of granulate and non-granulate colesevelam that high drug loading unit dose tablets of colesevelam (e.g., containing 850 - 1100 mg colesevelam) could be manufactured. Advantageously, the compositions had good hardness, and a plurality of the tablet cores had excellent uniformity of mass. It is particularly surprising that by splitting the colesevelam drug loading into a mixture of granulate and non-granulate yielded high-quality tablet cores of sufficient hardness and robustness (i.e., having low friability).

The inventors also investigated if lower amounts of colesevelam could be employed in the same manner to manufacture high drug loading lower dose tablets.

### Example 2B: Combination of granulated components and non-granulate components

Tablet cores were manufactured using a 22.5 x 10.5 mm punch from a blend of granulate and non-granulate components in the same manner as for the above Example 2A.

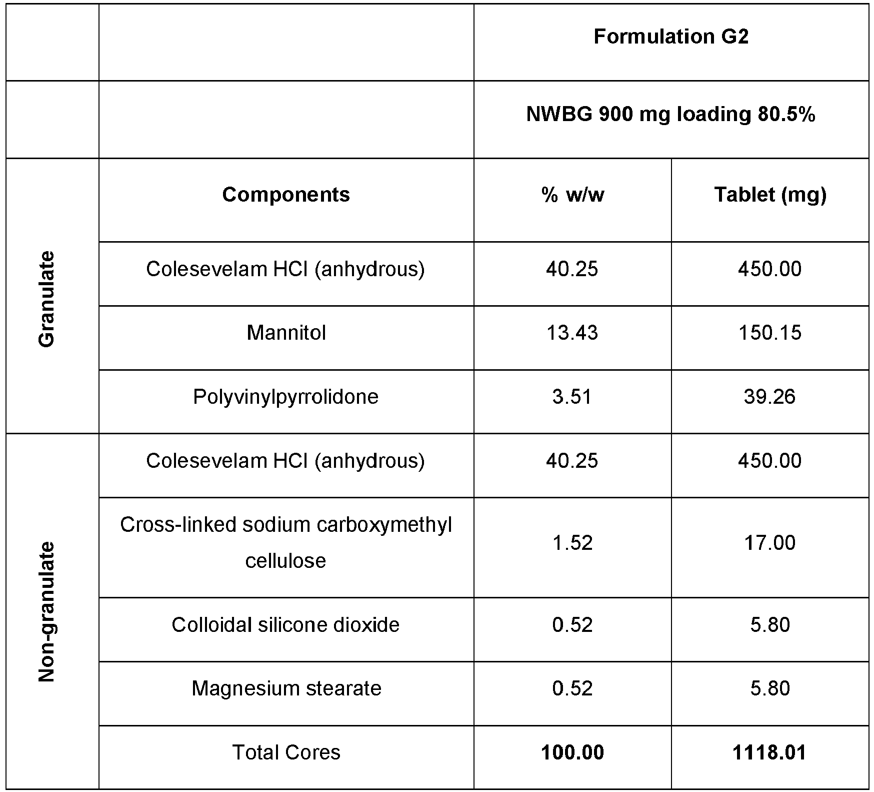

### Analysis of tablet cores of Formulation G2:

| | **Formulation G2** |
|---|---|
| | **NWBG 900 mg loading 80.5%** |
| **Average mass (mg)** | 1151.0 |
| **Uniformity of mass** | Complies |
| | (-0.99%/+1.01%) |
| **Hardness (N)** | 193 |
| | (182-208) |
| **Thickness (mm)** | 8.00 |
| | (7.92-8.07) |
| **Friability (%)** | < 0.1% |

### Example 3: combination of granulated and non-granulated colesevelam

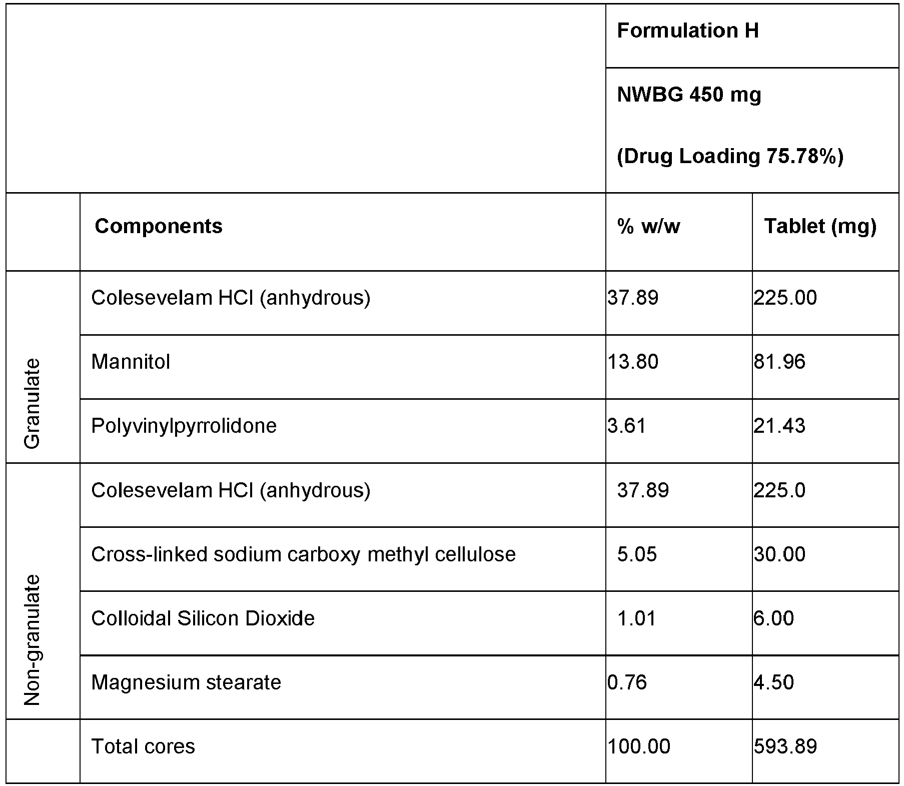

Tablet cores were manufactured using a 16.8 x 7.0 mm punch from a blend of granulate and non-granulate components in the same manner as for the higher dose high drug loading tablet cores. The tablet cores of example 3 were analysed:

| | **Formulation H** |
|---|---|
| | **NWBG 450 mg** |
| | **(Drug Loading 75.77%)** |
| **Average mass (mg)** | 610.9 |
| **Uniformity of mass** | Complies |
| | (-0.5%/+0.6%) |
| **Hardness (N)** | 170 |
| | (162-181) |
| **Thickness (mm)** | 7.12 |
| | (7.03-7.21) |
| **Friability (%)** | < 0.1% |

Pleasingly, the high loading lower dose tablet cores had excellent properties, with hardness values of about 170 N achieved, and very low friability. In addition, the batch of tablet cores had excellent uniformity of mass.

### Tablet coating

A coating solution can be prepared by dissolving the functional coatings e.g. methacrylic acid/methyl methacrylate copolymers in solvent, such as ethanol, and applying to the tablet cores using a suitable coating machine.

For example, a coating comprising: methacrylic acid/methyl methacrylate (1:2) copolymers (EUDRAGIT S100), methacrylic acid/methylmethacrylate (1:1) copolymer (EUDRAGIT L100), triethyl citrate and talc was prepared in ethanol, and sprayed onto tablet cores to form unit dose pharmaceutical composition for oral administration according to the invention.

An optional sub-coating can also be applied, between the tablet cores and the gastro-resistant coating. For example, a sub-coating comprising hydroxypropylcellulose and ethanol may be sprayed onto tablet cores and dried, prior to adding a gastro-resistant coating thereto.

Example unit dose formulations of Formulation F and Formulation H are shown in the below table:

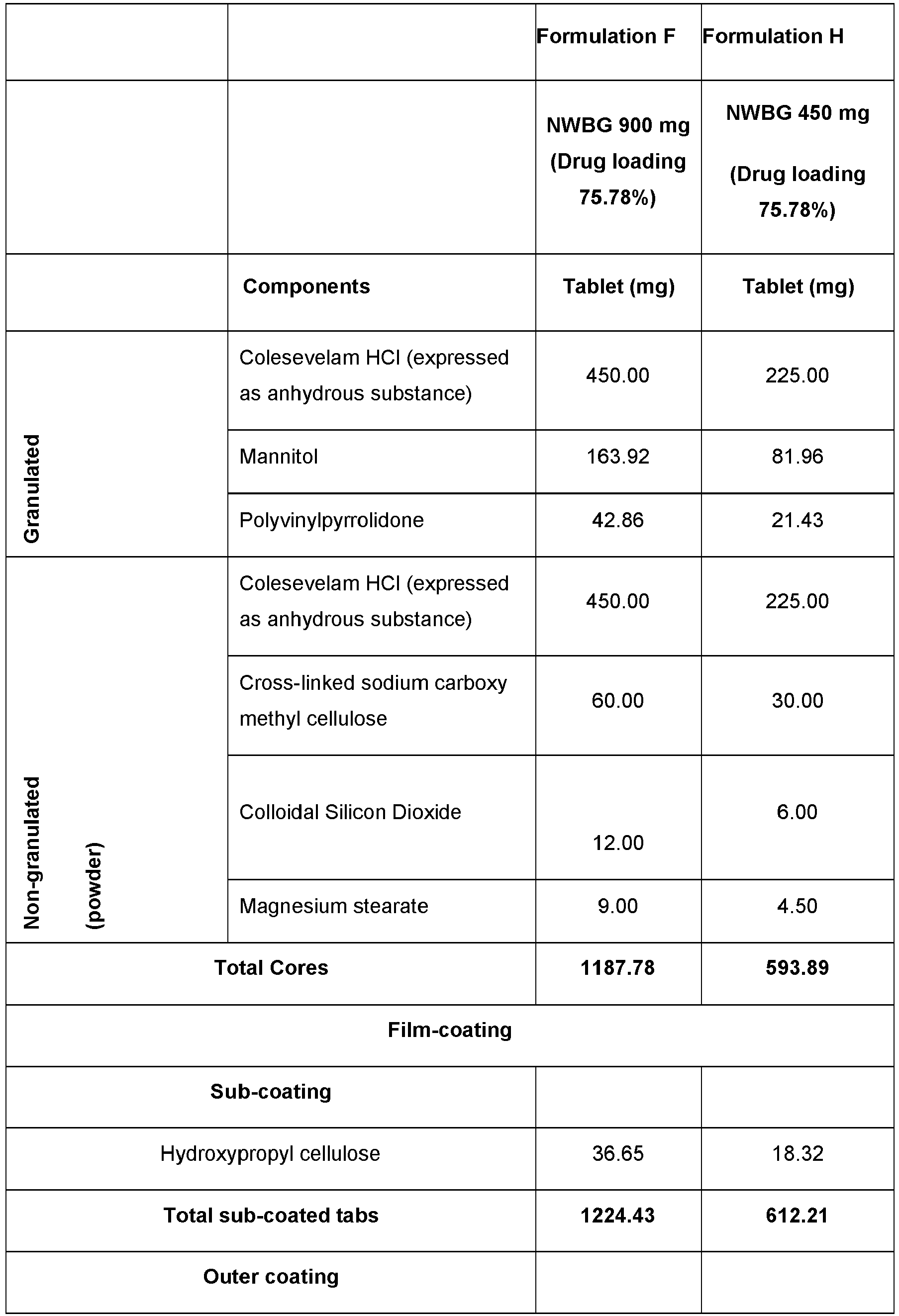

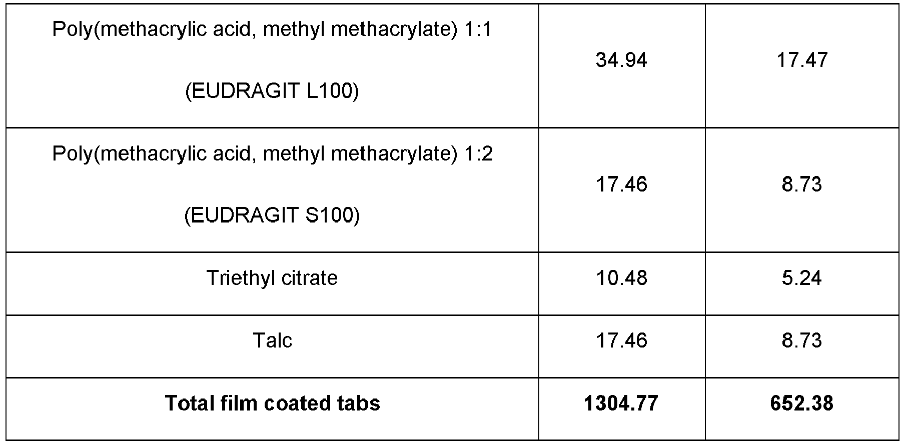

Advantageously, the tablets have excellent hardness and low friability. Furthermore, the high loading and high dose of colesevelam implies that less tablets are required for treatment of diseases such as Bile Acid Diarrhea, IBS, colitis, and/or bile acid malabsorption, thereby improving patient compliance.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable subcombination.

### Embodiments

1. A unit dose pharmaceutical composition for oral administration comprising a tablet core and a gastro-resistant coating covering the core,
   wherein the tablet core comprises a granulate component and a non-granulate component:
   wherein the granulate component comprises colesevelam and/or a pharmaceutically acceptable salt thereof, a diluent and a binder;
   wherein the non-granulate component comprises colesevelam or a pharmaceutically acceptable salt thereof and optionally one or more of: a diluent, a disintegrant, a glidant and a lubricant;
   wherein the colesevelam and/or the pharmaceutically acceptable salts thereof is present in an amount of at least 66 wt% of the tablet core, such as at least about 70 wt% of the tablet core.
2. The unit dose pharmaceutical composition of embodiment 1, wherein colesevelam and/or pharmaceutically acceptable salts thereof is present in the granulate component in an amount of from 30 to 60 wt% of the tablet core, preferably 30 to 50 wt% of the tablet core, such as 35 to 50 wt% of the tablet core, more preferably 35 to 45 wt% of the tablet core, optionally, in an amount of from 36 to 45 wt% of the tablet core, such as from 37 to 45 wt% of the tablet core.
3. The unit dose pharmaceutical composition of embodiment 1 or 2, wherein colesevelam and/or pharmaceutically acceptable salts thereof is present in the non-granulate component in an amount of from 30 to 60 wt% of the tablet core, preferably 30 to 50 wt% of the tablet core, such as 35 to 50 wt% of the tablet core, more preferably 35 to 45 wt% of the tablet core, optionally, in an amount of from 36 to 45 wt% of the tablet core, such as from 37 to 45 wt% of the tablet core.
4. The unit dose pharmaceutical composition of any preceding embodiment, wherein colesevelam and/or pharmaceutically acceptable salts thereof is present in an amount of at least 76 wt% of the tablet core, preferably at least 80 wt% of the tablet core, more preferably at least 81 wt% of the tablet core, optionally at least 82 wt% of the tablet core.
5. The unit dose pharmaceutical composition of any preceding embodiment, wherein colesevelam and/or pharmaceutically acceptable salts thereof is present in an amount of from 400 mg to 1250 mg in the tablet core, preferably in an amount of from 400 mg to 1200 mg in the tablet core, such as from 700 to 1200 mg in the tablet core, more preferably in an amount of from 850 mg to 1100 mg in the tablet core.
6. The unit dose pharmaceutical composition of any preceding embodiment, wherein colesevelam and/or pharmaceutically acceptable salts thereof is present in an amount of about 900 mg in the tablet core.
7. The unit dose pharmaceutical composition of any preceding embodiment, wherein the unit dose is formulated for delayed release, optionally for delayed release in the ileum.
8. The unit dose pharmaceutical composition of any preceding embodiment, wherein the non-granulate component comprises one or more of a diluent, disintegrant, glidant and a lubricant.
9. The unit dose pharmaceutical composition of any preceding embodiment, wherein the diluent is selected from one or more of polyols such as mannitol, lactose, sugars such as sucrose, dextrose, dextrates, sorbitol, fructose, inorganic salts such as dibasic and tribasic calcium phosphate, sodium chloride, starch, modified starch and derivatives thereof, cellulose, and a cellulose derivative (i.e. cellulose derivatives such as, for example, methyl, ethyl, or hydroxyethyl cellulose), and kaolin.
10. The unit dose pharmaceutical composition of embodiment 9, wherein the diluent is selected from one or more of mannitol, sucrose, and cellulose, and a cellulose derivative.
11. The unit dose pharmaceutical composition of any preceding embodiment, wherein the diluent is present in an amount of from 5 to 20 wt% of the tablet core, such as from 6 to 18 wt% of the tablet core, preferably from 7 to 16 wt% of the tablet core, more preferably from 10 to 15 wt% of the tablet core.
12. The unit dose pharmaceutical composition of any preceding embodiment, wherein the binder is selected from one or more of polyvinyl pyrrolidone, polyvinyl alcohol, copovidone, cellulose, and a cellulose derivative (e.g. hydroxypropyl cellulose, hypromellose, ethyl cellulose), acacia gum, starch, and polymethylacrylates, preferably polyvinyl pyrrolidone and/or poly vinyl alcohol.
13. The unit dose pharmaceutical composition of any preceding embodiment, wherein the binder is present in an amount of from 1 wt% to 5 wt% of the tablet core, preferably from 2 wt % to 4 wt% of the tablet core.
14. The unit dose pharmaceutical composition of any preceding embodiment, wherein the disintegrant is selected from one or more of sodium carboxy methyl cellulose, such as cross-linked sodium carboxy methyl cellulose, crospovidone, bentonite, algins, gums and modified starch, preferably, wherein the disintegrant is cross-linked sodium carboxy methyl cellulose.
15. The unit dose pharmaceutical composition of any preceding embodiment, wherein the disintegrant is present in an amount of from 0.1 wt% to 7 wt% of the tablet core, preferably from 0.5 wt % to 5 wt%, such as from 1 to 2.5 wt% of the tablet core, more preferably from 1 to 2 wt% of the tablet core.
16. The unit dose pharmaceutical composition of any preceding embodiment, further comprising a glidant and/or lubricant.
17. The unit dose pharmaceutical composition of embodiment 16, wherein glidant is selected from the group comprising: talc, starch, magnesium oxide, silica (silicon dioxide) and silicates, such as magnesium or calcium silicate.
18. The unit dose pharmaceutical composition of embodiment 16 or 17, wherein the glidant is present in an amount of from 0.1 wt% to 2 wt% of the tablet core, optionally wherein the glidant is present in an amount of from 0.5 wt% to 1.5 wt% of the tablet core, optionally wherein the glidant is present in an amount of from 0.5 wt % to 1.1 wt%.
19. The unit dose pharmaceutical composition of any of embodiments 16 to 18, wherein the lubricant is selected from the group comprising: stearic acid and salts thereof (e.g. magnesium, calcium or zinc stearate), polyethylene glycol, fumaric acid and salts thereof, glyceryl behenate, glyceryl palmitostearate, wax, and sodium benzoate.
20. The unit dose pharmaceutical composition of any of embodiments 16 to 19, wherein the lubricant is present in an amount of from 0.1 wt% to 2 wt% of the tablet core, optionally in an amount of from 0.5 wt% to 1.5 wt% of the tablet core, optionally wherein the lubricant is present in an amount of from 0.5 wt % to 1.1 wt%.
21. The unit dose pharmaceutical composition of any preceding embodiment, further comprising a sub-coating between the tablet core and the gastro-resistant coating, wherein optionally, the sub-coating comprises hydroxypropyl cellulose.
22. The unit dose pharmaceutical composition of any preceding embodiment, wherein said gastro-resistant coating comprises methacrylic acid/methyl methacrylate (1:1) copolymer.
23. The unit dose pharmaceutical composition of any preceding embodiment, wherein said gastro-resistant coating comprises methacrylic acid/methyl methacrylate (1:2) copolymer.
24. The unit dose pharmaceutical composition of any preceding embodiment, wherein said gastro-resistant coating comprises a mixture of methacrylic acid/methyl methacrylate (1:1) copolymer and of methacrylic acid/methyl methacrylate (1:2) copolymer.
25. The unit dose pharmaceutical composition of embodiment 20, wherein the mixture comprises a weight ratio of 0.5:1 to 3:1 of methacrylic acid/methyl methacrylate (1:1) copolymer to methacrylic acid/methyl methacrylate (1:2), optionally, the mixture comprises a weight ratio of 1:1 to 3:1 of methacrylic acid/methyl methacrylate (1:1) copolymer to methacrylic acid/methyl methacrylate (1:2).
26. The unit dose pharmaceutical composition according to any preceding embodiment, wherein the gastro-resistant coating of the unit dose starts breaking or dissolving at or above a pH of 6.0, preferably at or above a pH of 6.5, more preferably at a pH of 6.8.
27. The unit dose pharmaceutical composition of any preceding embodiment, wherein the tablet core has a friability of < 1.0%, optionally, < 0.5%, preferably < 0.1% when measured in accordance with US Pharmacopeia monograph <1216> or with European Pharmacopoeia monograph 2.9.7.
28. The unit dose pharmaceutical composition of any preceding embodiment, wherein the tablet core has a hardness of > 70 N, optionally > 80 N, optionally, > 90 N, when measured in accordance with US Pharmacopeia monograph <1217> or with European Pharmacopoeia monograph 2.9.8.
29. The unit dose pharmaceutical composition of any preceding embodiment, wherein the colesevelam and/or the pharmaceutically acceptable salts thereof is present in the granulate component in an amount of from 30 to 50 wt% of the tablet core, more preferably 35 to 45 wt% of the tablet core, and wherein the colesevelam and/or the pharmaceutically acceptable salts thereof is present in the non-granulate component in an amount of from 30 to 50 wt% of the tablet core.
30. The unit dose pharmaceutical composition of any preceding embodiment, wherein the colesevelam and/or the pharmaceutically acceptable salts thereof is present in the granulate component in an amount of from 30 to 50 wt% of the tablet core, more preferably 35 to 45 wt% of the tablet core, and wherein the colesevelam is present in the non-granulate component in an amount of from 30 to 50 wt% of the tablet core, more preferably 35 to 45 wt% of the tablet core, and wherein colesevelam and/or pharmaceutically acceptable salts thereof is present in a total amount of at least 76 wt% of the tablet core, and wherein the colesevelam and/or pharmaceutically acceptable salts thereof is present in an amount of from 700 mg to 1200 mg in the tablet core, preferably from 850 mg to 1100 mg in the tablet core, such as about 900 mg in the tablet core.
31. The unit dose pharmaceutical composition of any preceding embodiment, wherein the diluent is selected from one or more of mannitol, sucrose and cellulose, and wherein the diluent is present in an amount of from 7 to 16 wt% of the tablet core, wherein the binder is polyvinyl pyrrolidone and is present in an amount of from 1 wt% to 5 wt% of the tablet core.
32. The unit dose pharmaceutical composition of any preceding embodiment, wherein the colesevelam and/or pharmaceutically acceptable salt thereof is colesevelam hydrochloride.
33. The unit dose pharmaceutical composition of any preceding embodiment, wherein the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
34. The unit dose pharmaceutical composition of any preceding embodiment, wherein the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
35. The unit dose pharmaceutical composition of any preceding embodiment, wherein the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
36. The unit dose pharmaceutical composition of any preceding embodiment, wherein the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
37. The unit dose pharmaceutical composition of any preceding embodiment, wherein the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
38. The unit dose pharmaceutical composition of any preceding embodiment, wherein the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
39. The unit dose pharmaceutical composition of any preceding embodiment, wherein the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
40. The unit dose pharmaceutical composition of any one of embodiments 1 to 32, wherein the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
41. The unit dose pharmaceutical composition of any one of embodiments 1 to 32, wherein the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
42. The unit dose pharmaceutical composition of any one of embodiments 1 to 32t, wherein the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
43. The unit dose pharmaceutical composition of any one of embodiments 1 to 32, wherein the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
44. The unit dose pharmaceutical composition of any one of embodiments 1 to 32, wherein the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
45. The unit dose pharmaceutical composition of any preceding embodiment, wherein the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
46. The unit dose pharmaceutical composition of any preceding embodiment, wherein the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
47. The unit dose pharmaceutical composition of any one of embodiments 1 to 32, wherein the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
48. The unit dose pharmaceutical composition of any one of embodiments 1 to 32, wherein the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
49. The unit dose pharmaceutical composition of any one of embodiments 1 to 32, wherein the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
50. The unit dose pharmaceutical composition of any one of embodiments 1 to 32, wherein the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
51. The unit dose pharmaceutical composition of any one of embodiments 1 to 32, wherein the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
52. The unit dose pharmaceutical composition of any one of embodiments 1 to 32, wherein the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
53. The unit dose pharmaceutical composition of any one of embodiments 1 to 32, wherein the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
54. A method of manufacturing a unit dose pharmaceutical composition for oral administration comprising the steps of:
   (a) providing a tablet core wherein the tablet core comprises:
      a granulate component and a non-granulate component,
      wherein the granulate component comprises colesevelam and/or a pharmaceutically acceptable salt thereof, a diluent and a binder,
      wherein the non-granulate component comprises colesevelam or a pharmaceutically acceptable salt thereof, and optionally, one or more of a diluent, a disintegrant, a glidant and lubricants, and
      wherein the colesevelam and/or the pharmaceutically acceptable salts thereof is present in an amount of at least 66 wt% of the tablet core, preferably at least 70 wt% of the tablet core; and
   (b) coating the tablet core with a gastro resistant coating and optionally a sub-coating.
55. The method of embodiment 54, wherein the method comprises dry or wet granulation, preferably wet granulation.
56. The method of embodiments 54 or 55, comprising the steps of:
   (i) combining colesevelam and/or pharmaceutically acceptable salts thereof, a diluent and a binder, to form a granulate;
   (ii) combining colesevelam and/or pharmaceutically acceptable salts thereof and, optionally, a diluent and a disintegrant, with the granulate of step (i), to form a tablet core having a granulate component and a non-granulate component:
      wherein the granulate component comprises colesevelam and/or a pharmaceutically acceptable salt thereof, a diluent and a binder;
      wherein the non-granulate component comprises colesevelam or a pharmaceutically acceptable salt thereof, and, optionally, one or more of a diluent, a disintegrant, a glidant and lubricants;
      wherein the colesevelam and/or the pharmaceutically acceptable salts thereof is present in an amount of at least 66 wt% of the tablet core, preferably at least 70 wt% of the tablet core; and
   (iii) coating the tablet core with a gastro resistant coating and optionally a sub-coating.
57. The method of embodiment 56, wherein step (i) involves wet granulation.
58. The method of embodiment 57, wherein the wet granulation is carried out using water or a non-aqueous solvent, preferably a non-aqueous solvent, such as an organic solvent.
59. The method of embodiment 58, wherein the organic solvent is an alcohol, a ketone or a chlorinated solvent.
60. The method of embodiment 58 or 59, wherein the organic solvent is an alcohol selected from methanol, ethanol, propanol, isopropanol, preferably wherein the alcohol is ethanol.
61. The method of any one of embodiments 54 to 60, for bulk manufacture of a plurality of unit doses comprising a pharmaceutical composition for oral administration, wherein the plurality of unit doses has a uniformity of mass of less than ±2%, preferably less than ±1%, more preferably less than ±0.8%.
62. A unit dose pharmaceutical composition for oral administration according to any one of embodiments 1 to 53 for use in a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering more than one of said unit dose oral pharmaceutical compositions to said patient.
63. A method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, comprising administering to said patient a unit does pharmaceutical composition for oral administration according to any one of embodiments 1 to 53, optionally, wherein the method comprises administering more than one of said unit dose oral pharmaceutical compositions to said patient.
64. Use of a unit dose pharmaceutical composition for oral administration according to any one of embodiments 1 to 53 for treating bile acid diarrhea, irritable bowel syndrome, optionally, irritable bowel syndrome subtype diarrhea (IBS-D), colitis, such as microscopic colitis, and/or bile acid malabsorption in a patient in need thereof, wherein optionally said use comprises administering more than one of said unit dose oral pharmaceutical compositions to said patient.
65. Use of a unit dose pharmaceutical composition for oral administration according to any one of embodiments 1 to 53 in the manufacture of a medicament for treating bile acid diarrhea, irritable bowel syndrome, optionally, irritable bowel syndrome subtype diarrhea (IBS-D), colitis, such as microscopic colitis, and/or bile acid malabsorption.
66. A unit dose pharmaceutical composition for oral administration comprising a tablet core and a gastro-resistant coating covering the core,
   wherein the tablet core comprises a granulate component and a non-granulate component:
   wherein the granulate component comprises colesevelam hydrochloride, a diluent and a binder;
   wherein the non-granulate component comprises colesevelam hydrochloride and optionally one or more of: a diluent, a disintegrant, a glidant and a lubricant;
   wherein the colesevelam hydrochloride is present in an amount of about 60 wt% to about 90 wt% of the tablet core.
67. The unit dose pharmaceutical composition of embodiment 66, wherein colesevelam hydrochloride is present in the granulate component in an amount of from about 37 wt% to about 45 wt% of the tablet core.
68. The unit dose pharmaceutical composition of embodiment 66 or 67, wherein colesevelam hydrochloride is present in the non-granulate component in an amount of from about 37 wt% to about 45 wt% of the tablet core.
69. The unit dose pharmaceutical composition of embodiments 66-68, wherein colesevelam hydrochloride is present in an amount of from about 700 to about 1200 mg in the tablet core.
70. The unit dose pharmaceutical composition of embodiments 66-69, wherein colesevelam hydrochloride is present in an amount of about 900 mg in the tablet core.

## Claims

1. A unit dose pharmaceutical composition for oral administration comprising a tablet core and a gastro-resistant coating covering the core,
wherein the tablet core comprises a granulate component and a non-granulate component:
wherein the granulate component comprises colesevelam and/or a pharmaceutically acceptable salt thereof, a diluent and a binder;
wherein the non-granulate component comprises colesevelam or a pharmaceutically acceptable salt thereof and optionally one or more of: a diluent, a disintegrant, a glidant and a lubricant;
wherein the colesevelam and/or the pharmaceutically acceptable salts thereof is present in an amount of at least 66 wt% of the tablet core, such as at least about 70 wt% of the tablet core.

2. The unit dose pharmaceutical composition of claim 1, wherein colesevelam and/or pharmaceutically acceptable salts thereof is present in the granulate component in an amount of from 30 to 60 wt% of the tablet core, preferably 30 to 50 wt% of the tablet core, such as 35 to 50 wt% of the tablet core, more preferably 35 to 45 wt% of the tablet core, optionally, in an amount of from 36 to 45 wt% of the tablet core, such as from 37 to 45 wt% of the tablet core.

3. The unit dose pharmaceutical composition of claim 1 or 2, wherein colesevelam and/or pharmaceutically acceptable salts thereof is present in the non-granulate component in an amount of from 30 to 60 wt% of the tablet core, preferably 30 to 50 wt% of the tablet core, such as 35 to 50 wt% of the tablet core, more preferably 35 to 45 wt% of the tablet core, optionally, in an amount of from 36 to 45 wt% of the tablet core, such as from 37 to 45 wt% of the tablet core.

4. The unit dose pharmaceutical composition of any preceding claim, wherein colesevelam and/or pharmaceutically acceptable salts thereof is present in an amount of at least 76 wt% of the tablet core, preferably at least 80 wt% of the tablet core, more preferably at least 81 wt% of the tablet core, optionally at least 82 wt% of the tablet core.

5. The unit dose pharmaceutical composition of any preceding claim, wherein colesevelam and/or pharmaceutically acceptable salts thereof is present in an amount of from 400 mg to 1250 mg in the tablet core, preferably in an amount of from 400 mg to 1200 mg in the tablet core, such as from 700 to 1200 mg in the tablet core, more preferably in an amount of from 850 mg to 1100 mg in the tablet core.

6. The unit dose pharmaceutical composition of any preceding claim, wherein the colesevelam and/or the pharmaceutically acceptable salts thereof is present in the granulate component in an amount of from 30 to 50 wt% of the tablet core, more preferably 35 to 45 wt% of the tablet core, and wherein the colesevelam is present in the non-granulate component in an amount of from 30 to 50 wt% of the tablet core, more preferably 35 to 45 wt% of the tablet core, and wherein colesevelam and/or pharmaceutically acceptable salts thereof is present in a total amount of at least 76 wt% of the tablet core, and wherein the colesevelam and/or pharmaceutically acceptable salts thereof is present in an amount of from 700 mg to 1200 mg in the tablet core, preferably from 850 mg to 1100 mg in the tablet core, such as about 900 mg in the tablet core.

7. The unit dose pharmaceutical composition of any preceding claim, wherein the tablet core has a friability of < 1.0%, optionally, < 0.5%, preferably < 0.1% when measured in accordance with US Pharmacopeia monograph <1216> or with European Pharmacopoeia monograph 2.9.7.

8. The unit dose pharmaceutical composition of any preceding claim, wherein the tablet core has a hardness of > 70 N, optionally > 80 N, optionally, > 90 N, when measured in accordance with US Pharmacopeia monograph <1217> or with European Pharmacopoeia monograph 2.9.8.

9. The unit dose pharmaceutical composition of any preceding claim, wherein the colesevelam and/or the pharmaceutically acceptable salts thereof is present in an amount of from 76 wt% to 90 wt%, suitably from 80 wt% to 90 wt%;
wherein colesevelam and/or pharmaceutically acceptable salts thereof is present in the granulate component in an amount of from 35 to 45 wt% of the tablet core; and
colesevelam and/or pharmaceutically acceptable salts thereof is present in the non-granulate component in an amount of from 35 to 45 wt% of the tablet core;
wherein the (total) colesevelam and/or pharmaceutically acceptable salts thereof (in the unit dose) is present in an amount of from 700 mg to 1200 mg in the tablet core, such as in an amount of from 850 mg to 1100 mg in the tablet core;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8; and
wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

10. The unit dose pharmaceutical composition of any preceding claim, wherein colesevelam and/or pharmaceutically acceptable salts thereof is present in an amount of about 900 mg in the tablet core.

11. The unit dose pharmaceutical composition of any preceding claim, wherein the non-granulate component comprises one or more of a diluent, disintegrant, glidant and a lubricant;
optionally, wherein the diluent is selected from one or more of polyols such as mannitol, lactose, sugars such as sucrose, dextrose, dextrates, sorbitol, fructose, inorganic salts such as dibasic and tribasic calcium phosphate, sodium chloride, starch, modified starch and derivatives thereof, cellulose, and a cellulose derivative (i.e. cellulose derivatives such as, for example, methyl, ethyl, or hydroxyethyl cellulose), and kaolin; further optionally, wherein the diluent is present in an amount of from 5 to 20 wt% of the tablet core, such as from 6 to 18 wt% of the tablet core, preferably from 7 to 16 wt% of the tablet core, more preferably from 10 to 15 wt% of the tablet core;
optionally, wherein the binder is selected from one or more of polyvinyl pyrrolidone, polyvinyl alcohol, copovidone, cellulose, and a cellulose derivative (e.g. hydroxypropyl cellulose, hypromellose, ethyl cellulose), acacia gum, starch, and a polymethylacrylate, preferably polyvinyl pyrrolidone and/or poly vinyl alcohol; further optionally, wherein the binder is present in an amount of from 1 wt% to 5 wt% of the tablet core, preferably from 2 wt % to 4 wt% of the tablet core;
optionally, wherein the disintegrant is selected from one or more of sodium carboxy methyl cellulose, such as cross-linked sodium carboxy methyl cellulose, crospovidone, bentonite, an algin, a gum and modified starch, preferably, wherein the disintegrant is cross-linked sodium carboxy methyl cellulose, further optionally, wherein the disintegrant is present in an amount of from 0.1 wt% to 7 wt% of the tablet core, preferably from 0.5 wt % to 5 wt%, such as from 1 to 2.5 wt% of the tablet core, more preferably from 1 to 2 wt% of the tablet core.

12. The unit dose pharmaceutical composition of any preceding claim, wherein the colesevelam and/or the pharmaceutically acceptable salts thereof is present in an amount of from 81 wt% to 90 wt% of the tablet core,
wherein colesevelam and/or pharmaceutically acceptable salts thereof is present in the granulate component in an amount of from 36 to 45 wt% of the tablet core; and
colesevelam and/or pharmaceutically acceptable salts thereof is present in the non-granulate component in an amount of from 36 to 45 wt% of the tablet core;
wherein the (total) colesevelam and/or pharmaceutically acceptable salts thereof (in the unit dose) is present in an amount of from 700 mg to 1200 mg in the tablet core, optionally from about 850 to about 1100 mg in the tablet core;
wherein said diluent is selected from one or more of mannitol, sucrose, cellulose and a cellulose derivative, wherein the diluent is present in an amount of from about 7 to about 16 wt% of the tablet core;
wherein said binder is selected from one or more of polyvinyl pyrrolidone, polyvinyl alcohol and related graft copolymers such as polyvinyl alcohol graft polyethylene glycol copolymer, copovidone, cellulose, a cellulose derivative, a polymethacrylate, acacia gum and starch, preferably polyvinyl pyrrolidone and/or polyvinyl alcohol; wherein the binder is present in an amount of from about 1 wt% to 4 wt % of the tablet core;
wherein the non-granulate component further comprises a lubricant and a glidant;
wherein the tablet core has a hardness of 70 N to 400 N, optionally from 80 N to 400 N, optionally, from 90 N to 400 N, when measured in accordance with US Pharmacopeia monograph <1217> or with European Pharmacopoeia monograph 2.9.8, and wherein the tablet core has a friability of 0.001% to 1%, optionally, from 0.01% to 0.5%, preferably from 0.01% to 0.1% when measured in accordance with US Pharmacopeia monograph <1216> or with European Pharmacopoeia monograph 2.9.7.

13. The unit dose pharmaceutical composition according to any preceding claim, wherein the gastro-resistant coating of the unit dose starts breaking or dissolving at or above a pH of 6.0, preferably at or above a pH of 6.5, more preferably at a pH of 6.8.

14. The unit dose pharmaceutical composition of any preceding claim, wherein the colesevelam and/or pharmaceutically acceptable salt thereof is colesevelam hydrochloride.

15. A method of manufacturing a unit dose pharmaceutical composition for oral administration comprising the steps of:
(a) providing a tablet core wherein the tablet core comprises:
a granulate component and a non-granulate component,
wherein the granulate component comprises colesevelam and/or a pharmaceutically acceptable salt thereof, a diluent and a binder,
wherein the non-granulate component comprises colesevelam or a pharmaceutically acceptable salt thereof, and optionally, one or more of a diluent, a disintegrant, a glidant and a lubricant, and
wherein the colesevelam and/or the pharmaceutically acceptable salts thereof is present in an amount of at least 66 wt% of the tablet core, preferably at least 70 wt% of the tablet core; and
(b) coating the tablet core with a gastro resistant coating and optionally a sub-coating.

16. The method of claim 15, comprising the steps of:
(i) combining colesevelam and/or pharmaceutically acceptable salts thereof, a diluent and a binder, to form a granulate;
(ii) combining colesevelam and/or pharmaceutically acceptable salts thereof and, optionally, a diluent and a disintegrant, with the granulate of step (i), to form a tablet core having a granulate component and a non-granulate component:
wherein the granulate component comprises colesevelam and/or a pharmaceutically acceptable salt thereof, a diluent and a binder;
wherein the non-granulate component comprises colesevelam or a pharmaceutically acceptable salt thereof, and, optionally, one or more of a diluent, a disintegrant, a glidant and a lubricant;
wherein the colesevelam and/or the pharmaceutically acceptable salts thereof is present in an amount of at least 66 wt% of the tablet core, preferably at least 70 wt% of the tablet core; and
(iii) coating the tablet core with a gastro resistant coating and optionally a sub-coating;
wherein optionally, step (i) involves wet granulation, further optionally,
the wet granulation is carried out using water or a non-aqueous solvent, preferably a non-aqueous solvent, such as an organic solvent, such as an alcohol, preferably ethanol, a ketone or a chlorinated solvent.

17. The method of any one of claims 15 or 16, for bulk manufacture of a plurality of unit doses comprising a pharmaceutical composition for oral administration, wherein the plurality of unit doses has a uniformity of mass of less than ±2%, preferably less than ±1%, more preferably less than ±0.8%.

18. A unit dose pharmaceutical composition for oral administration according to any one of claims 1 to 14 for use in a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering more than one of said unit dose oral pharmaceutical compositions to said patient.

19. A unit dose pharmaceutical composition for oral administration comprising a tablet core and a gastro-resistant coating covering the core,
wherein the tablet core comprises a granulate component and a non-granulate component:
wherein the granulate component comprises colesevelam hydrochloride, a diluent and a binder;
wherein the non-granulate component comprises colesevelam hydrochloride and optionally one or more of: a diluent, a disintegrant, a glidant and a lubricant;
wherein the colesevelam hydrochloride is present in an amount of about 66 wt% to about 90 wt% of the tablet core.

20. The unit dose pharmaceutical composition of claim 19, wherein colesevelam hydrochloride is present in the granulate component in an amount of from about 37 wt% to about 45 wt% of the tablet core; and/or wherein colesevelam hydrochloride is present in the non-granulate component in an amount of from about 37 wt% to about 45 wt% of the tablet core.

21. The unit dose pharmaceutical composition of claim 19 or 20, wherein colesevelam hydrochloride is present in an amount of from about 700 to about 1200 mg in the tablet core; suitably, wherein colesevelam hydrochloride is present in an amount of about 900 mg in the tablet core.
